(19) Europäisches Patentamt
European Patent Office
Office européen des brevets

(11) **EP 1 431 369 B1**

(12) **EUROPÄISCHE PATENTSCHRIFT**


(45) Veröffentlichungstag und Bekanntmachung des Hinweises auf die Patenterteilung:
**31.01.2007 Patentblatt 2007/05**

(51) Int Cl.:
***C09K 19/34*** *(2006.01)* ***C09K 19/46*** *(2006.01)*
***C09K 19/02*** *(2006.01)*

(21) Anmeldenummer: **03028928.4**

(22) Anmeldetag: **17.12.2003**


(54) **Flüssigkristallines Medium**

Liquid crystalline medium

Milieu liquide cristallin


(84) Benannte Vertragsstaaten:
**AT BE BG CH CY CZ DE DK EE ES FI FR GB GR HU IE IT LI LU MC NL PT RO SE SI SK TR**

(30) Priorität: **21.12.2002 DE 10260517**

(43) Veröffentlichungstag der Anmeldung:
**23.06.2004 Patentblatt 2004/26**



(73) Patentinhaber: **Merck Patent GmbH**
**64293 Darmstadt (DE)**

(72) Erfinder:
- **Hirschmann, Harald, Dr.**
**64291 Darmstadt (DE)**
- **Poetsch, Eike, Dr.**
**64367 Mühltal (DE)**
- **Kirsch, Peer, Dr.**
**64342 Seeheim-Jugenheim (DE)**
- **Schoen, Sabine, Dr.**
**45701 Herten (DE)**


(56) Entgegenhaltungen:

| | |
|---|---|
| **EP-A- 0 117 476** | **EP-A- 0 967 261** |
| **EP-A- 0 976 703** | **EP-A- 1 026 143** |
| **EP-A- 1 191 083** | **WO-A-02/12415** |
| **DE-A- 10 008 712** | **DE-A- 10 050 071** |
| **DE-A- 10 053 896** | **DE-A- 19 528 106** |
| **GB-A- 2 248 847** | **US-A- 5 811 028** |
| **US-A1- 2002 030 654** | |

EP 1 431 369 B1


Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft flüssigkristalline Verbindungen mit einem Pyranring und ein flüssigkristallines Medium sowie verdrillte und hochverdrillte nematische Flüssigkristallanzeigen (englisch: Twisted Nematic, kurz: TN; bzw. Supertwisted Nematic, kurz: STN) mit sehr kurzen Schaltzeiten und guten Steilheiten und Winkelabhängigkeiten sowie die darin verwendeten neuen nematischen Flüssigkristallmischungen.

TN-Anzeigen sind bekannt, z.B. aus M. Schadt und W. Helfrich, Appl. Phys. Lett., 18, 127 (1971). STN-Anzeigen sind bekannt, z.B. aus EP 0 131 216 B1; DE 34 23 993 A1; EP 0 098 070 A2; M. Schadt und F. Leenhouts, 17. Freiburger Arbeitstagung Flüssigkristalle (8.-10.04.87); K. Kawasaki et al., SID 87 Digest 391 (20.6); M. Schadt und F. Leenhouts, SID 87 Digest 372 (20.1); K. Katoh et al., Japanese Journal of Applied Physics, Vol. 26, No. 11, L 1784-L 1786 (1987); F. Leenhouts et al., Appl. Phys. Lett. 50 (21), 1468 (1987); H.A. van Sprang und H.G. Koopman, J. Appl. Phys. 62 (5), 1734 (1987); T.J. Scheffer und J. Nehring, Appl. Phys. Lett. 45 (10), 1021 (1984), M. Schadt und F. Leenhouts, Appl. Phys. Lett. 50 (5), 236 (1987) und E.P. Raynes, Mol. Cryst. Liq. Cryst. Letters Vol. 4 (1), pp. 1-8 (1986). Der Begriff STN umfasst hier jedes höher verdrillte Anzeigeelement mit einem Verdrillungswinkel dem Betrage nach zwischen 160° und 360°, wie beispielsweise die Anzeigeelemente nach Waters et al. (C.M. Waters et al., Proc. Soc. Inf. Disp. (New York) (1985) (3rd Intern. Display Conference, Kobe, Japan), die STN-LCD's (DE OS 35 03 259), SBE-LCD's (T.J. Scheffer und J. Nehring, Appl. Phys. Lett. 45 (1984) 1021), OMI-LCD's (M. Schadt und F. Leenhouts, Appl. Phys. Lett. 50 (1987), 236, DST-LCD's (EP OS 0 246 842) oder BW-STN-LCD's (K. Kawasaki et al., SID 87 Digest 391 (20.6)).

Insbesondere STN-Anzeigen zeichnen sich im Vergleich zu Standard-TN-Anzeigen durch wesentlich bessere Steilheiten der elektrooptischen Kennlinie und damit verbundenen besseren Kontrastwerten sowie durch eine wesentlich geringere Winkelabhängigkeit des Kontrastes aus.

Von besonderem Interesse sind TN- und STN-Anzeigen mit sehr kurzen Schaltzeiten insbesondere auch bei tieferen Temperaturen. Zur Erzielung von kurzen Schaltzeiten wurden bisher die Rotationsviskositäten der Flüssigkristallmischungen optimiert unter Verwendung von meist monotropen Zusätzen mit relativ hohem Dampfdruck. Die erzielten Schaltzeiten waren jedoch nicht für jede Anwendung ausreichend.

Zur Erzielung einer steilen elektrooptischen Kennlinie in den erfindungsgemäßen Anzeigen sollen die Flüssigkristallmischungen relativ große Werte für das Verhältnis der elastischen Konstanten $K_{33}/K_{11}$, sowie relativ kleine Werte für $\Delta\varepsilon/\varepsilon_\perp$ aufweisen, wobei $\Delta\varepsilon$ die dielektrische Anisotropie und die $\varepsilon_\perp$ dielektrische Konstante senkrecht zur Moleküllängsachse ist.

Über die Optimierung des Kontrastes und der Schaltzeiten hinaus werden an derartige Mischungen weitere wichtige Anforderungen gestellt:

1. Breites d/p-Fenster

2. Hohe chemische Dauerstabilität

3. Hoher elektrischer Widerstand

4. Geringe Frequenz- und Temperaturabhängigkeit der Schwellenspannung.

Die erzielten Parameterkombinationen sind bei weitem noch nicht ausreichend, insbesondere für Hochmultiplex-STN-Anzeigen (mit einer Multiplexrate im Bereich von ca. 1/400), aber auch für Mittel- und Niedermultiplex-STN- (mit Multiplexraten im Bereich von ca. 1/64 bzw. 1/16), und TN-Anzeigen. Zum Teil ist dies darauf zurückzuführen, dass die verschiedenen Anforderungen durch Materialparameter gegenläufig beeinflusst werden.

Es besteht somit immer noch ein großer Bedarf nach TN- und STN-Anzeigen, insbesondere für Mittel- und Niedermultiplex-STN-Anzeigen, mit sehr kurzen Schaltzeiten bei gleichzeitig großem Arbeitstemperaturbereich, hoher Kennliniensteilheit, guter Winkelabhängigkeit des Kontrastes und niedriger Schwellenspannung, die den oben angegebenen Anforderungen gerecht werden.

Der Erfindung liegt die Aufgabe zugrunde flüssigkristalline Medien insbesondere für TN- und STN-Anzeigen, bereitzustellen, die die oben angegebenen Nachteile nicht oder nur in geringerem Maße und gleichzeitig kurze Schaltzeiten, insbesondere bei tiefen Temperaturen, und sehr gute Steilheiten aufweisen. Weiterhin sind die erfindungsgemäßen Medien für IPS-Anwendungen (In Plane Switching) geeignet.

Es wurde nun gefunden, dass diese Aufgabe gelöst werden kann, wenn man Flüssigkristallmischungen verwendet, die eine oder mehrere Verbindungen der Formel A

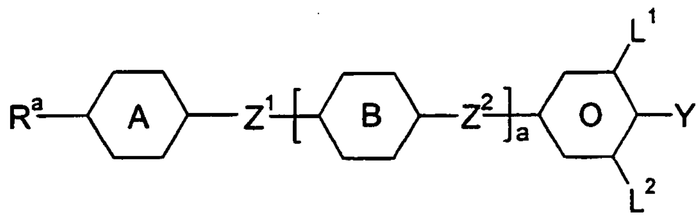

A

und mindestens eine Verbindung der Formel B

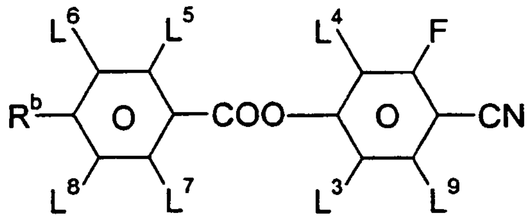

B

enthalten,
worin

$R^a$ und $R^b$ jeweils unabhängig voneinander H, einen unsubstituierten, einen einfach durch CN oder $CF_3$ oder einen mindestens einfach durch Halogen substituierten Alkylrest mit 1 bis 12 C-Atomen, wobei in diesen Resten auch eine oder mehrere $CH_2$-Gruppen jeweils unabhängig voneinander durch -O-, -S-,

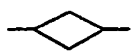

, -CH=CH-, -C≡C-, -CO-, -CO-O-, -O-CO- oder -O-CO-O- so ersetzt sein können, dass O-Atome nicht direkt miteinander verknüpft sind,

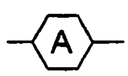 und 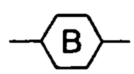

jeweils unabhängig voneinander

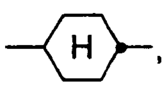, 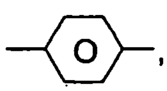, 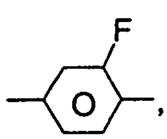, 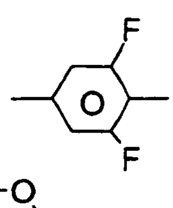

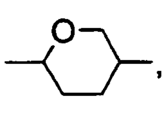, 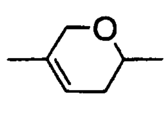 oder 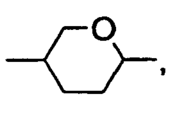,

mindestens ein Ring bedeutet

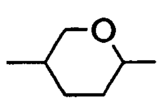,

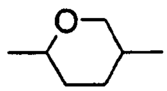 oder 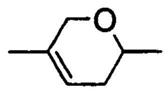 ,

$Z^1$ und $Z^2$ jeweils unabhängig voneinander $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ oder eine Einfachbindung,

a 0 oder 1,

$L^1$ bis $L^9$ jeweils unabhängig voneinander H oder F, und

Y F, Cl, $SF_5$, NCS, SCN, CN, OCN oder ein einfach oder mehrfach halogenierter Alkyl-, Alkoxy-, Alkenyl- oder Alkenyloxyrest mit jeweils bis zu 5 C-Atomen,

bedeuten.

Durch den Einsatz von Verbindungen der Formel A mit einem Pyranring in Kombination mit Verbindungen der Formel B werden insbesondere TN- und STN-Mischungen realisiert, die sich gegenüber dem Stand der Technik durch relativ hohe Klärpunkte, gute Werte für die optische Anisotropie (Δn) und sehr hohe Werte für die dielektrische Anisotropie und durch eine deutlich verbesserte Rotationsviskosität auszeichnen.

In den Offeniegungsschriften EP 0 117 476 A1, DE 19528 106 A1 und U.S. 2002/0030654 A1 werden Tetrahydropyrane von den generischen Formeln umfasst, aber die erfindungsgemäßen Tetrahydropyrane werden dort weder genannt noch wird die Verwendung dieser Verbindungen in Kombination mit einem Ester der Formel B offenbart.

Die Verwendung der Verbindungen der Formeln A und B in den Mischungen für erfindungsgemäße TN- und STN-Anzeigen bewirkt

- hohe Steilheit der elektrooptischen Kennlinie
- geringe Temperaturabhängigkeit der Schwellenspannung und
- sehr schnelle Schaltzeiten, insbesondere bei tiefen Temperaturen.

Die Verbindungen der Formel A und B verkürzen insbesondere deutlich die Schaltzeiten von TN- und STN-Mischungen bei gleichzeitiger Erhöhung der Steilheit und geringer Temperaturabhängigkeit der Schwellenspannung.

Weiterhin zeichnen sich die erfindungsgemäßen Mischungen durch folgende Vorzüge aus:

- sie besitzen eine niedrige Viskosität,
- sie besitzen eine niedrige Schwellenspannung und Operationsspannung,
- sie bewirken lange Lagerzeiten im Display bei tiefen Temperaturen.
  Gegenstand der Erfindung ist weiterhin ein Flüssigkristall-Display mit
- zwei Trägerplatten, die mit einer Umrandung eine Zelle bilden,
- einer in der Zelle befindlichen nematischen Flüssigkristallmischung mit positiver dielektrischer Anisotropie,
- Elektrodenschichten mit Orientierungsschichten auf den Innenseiten der Trägerplatten,
- einem Anstellwinkel zwischen der Längsachse der Moleküle an der Oberfläche der Trägerplatten und den Trägerplatten von 0 Grad bis 30 Grad, und
- einem Verdrillungswinkel der Flüssigkristallmischung in der Zelle von Orientierungsschicht zu Orientierungsschicht dem Betrag nach zwischen 22,5° und 600°,
- einer nematischen Flüssigkristallmischung bestehend aus

  a) 15 - 75 Gew.% einer flüssigkristallinen Komponente A, bestehend aus einer oder mehreren Verbindungen mit einer dielektrischen Anisotropie von über +1,5;

b) 25 - 85 Gew.% einer flüssigkristallinen Komponente B, bestehend aus einer oder mehreren Verbindungen mit einer dielektrischen Anisotropie zwischen -1,5 und +1,5;

c) 0 - 20 Gew.% einer flüssigkristallinen Komponente D, bestehend aus einer oder mehreren Verbindungen mit einer dielektrischen Anisotropie von unter -1,5 und

d) gegebenenfalls einer optisch aktiven Komponente C in einer Menge, dass das Verhältnis zwischen Schichtdicke (Abstand der Trägerplatten) und natürlicher Ganghöhe der chiralen nematischen Flüssigkristallmischung etwa 0,2 bis 1,3 beträgt,

dadurch gekennzeichnet, dass die Komponente A mindestens eine Verbindung der Formel A

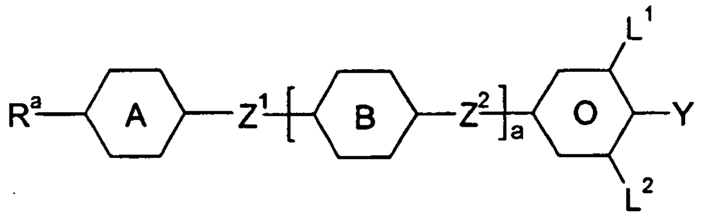

A

und mindestens eine Verbindung der Formel B

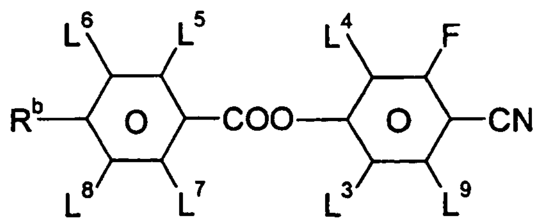

B

enthält,
worin $R^a$, $R^b$, Ring A, Ring B, a, $Z^1$, $Z^2$, $L^1$, $L^2$, $L^3$, $L^4$, $L^5$, $L^6$, $L^7$, $L^8$, $L^9$ und Y die angegebenen Bedeutungen haben.

Gegenstand der Erfindung sind auch TN- und STN-Anzeigen, insbesondere in mittel- und niedrigmultiplexierten STN-Anzeigen, die die erfindungsgemäße Flüssigkristallmischung enthalten.

Die Verwendung der Verbindungen der Formeln A und B in IPS-Mischungen führt zu verbesserten Schaltzeiten aufgrund der reduzierten Rotationsviskosität und zu einer Reduzierung der Ansteuerspannung für Monitor- und TV-Anwendungen.

Formel A umfasst insbesondere Verbindungen der Unterformeln A-1 bis A-56,

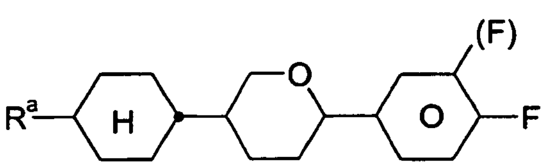

A-1

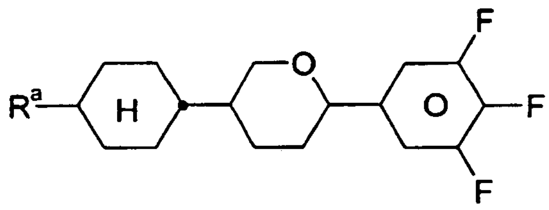
A-2

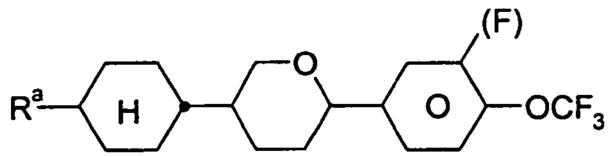
A-3

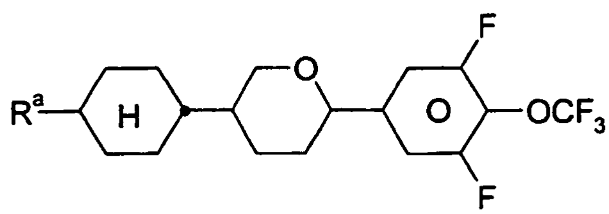
A-4

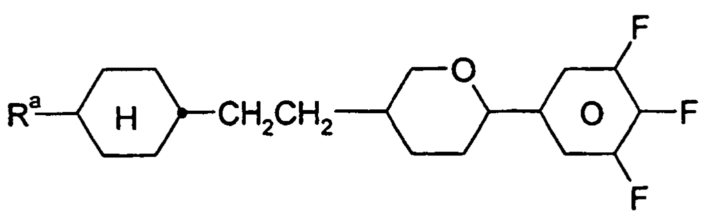
A-5

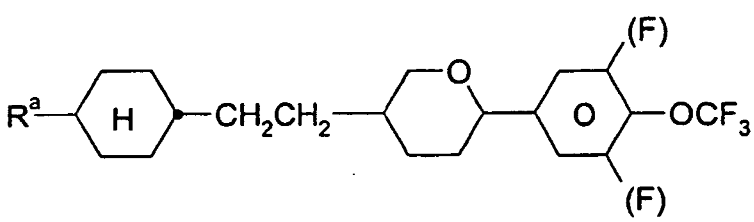
A-6

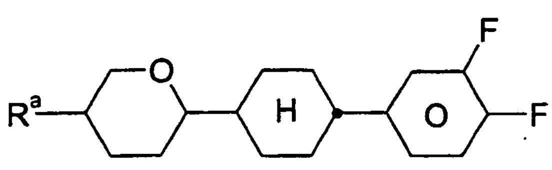
A-7

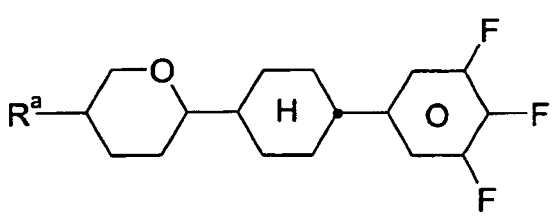
A-8

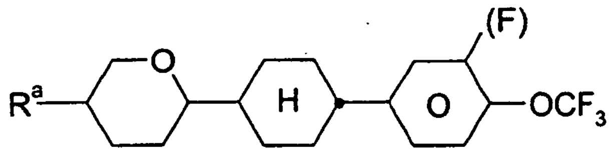

A-9

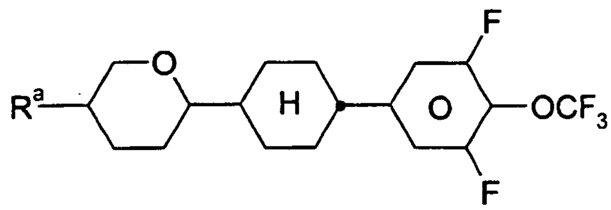

A-10

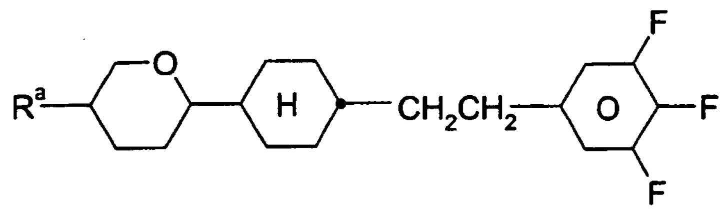

A-11

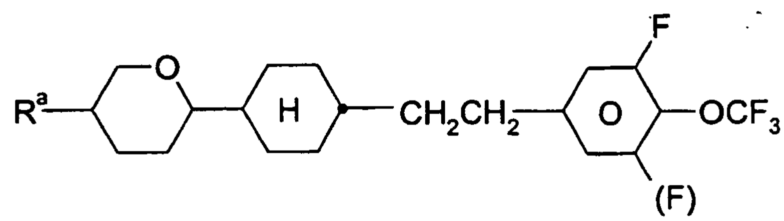

A-12

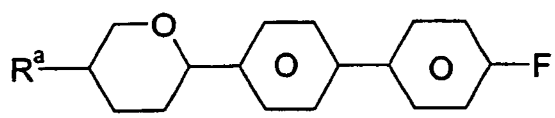

A-13

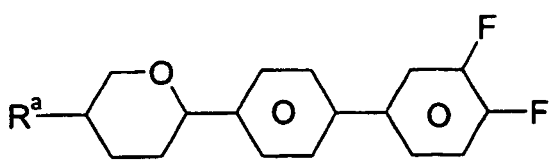

A-14

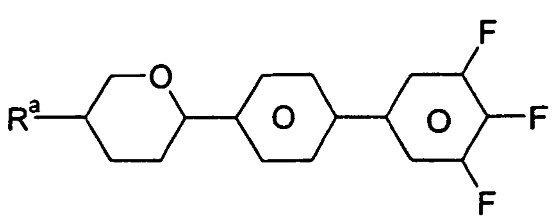

A-15

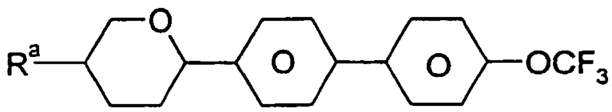

A-16

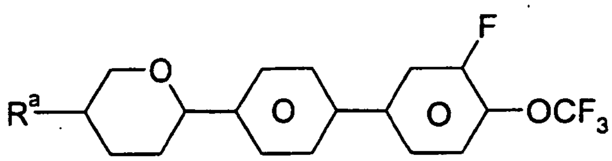

A-17

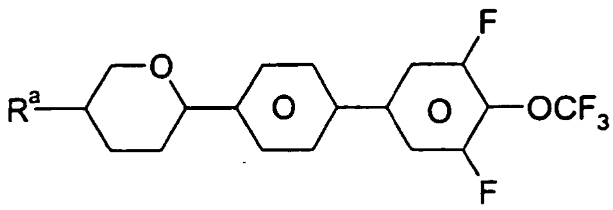

A-18

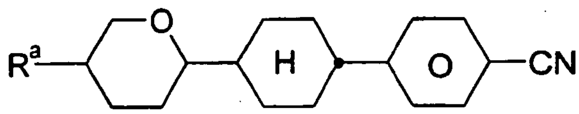

A-19

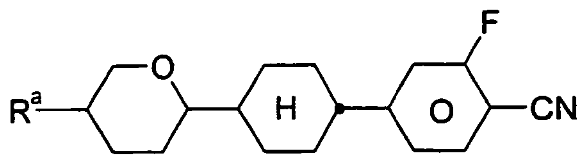

A-20

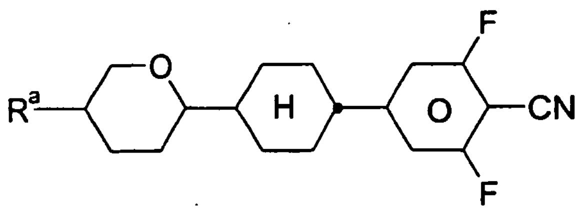

A-21

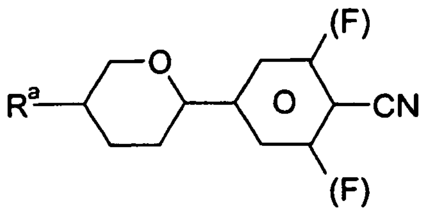

A-22

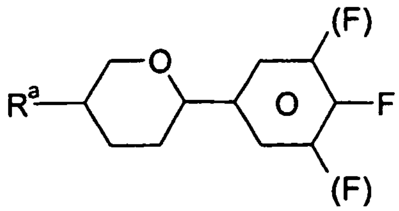

A-23

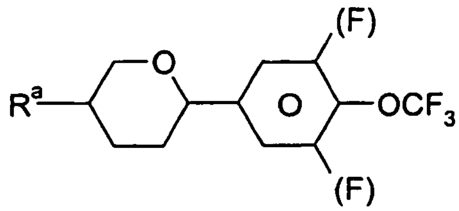

A-24

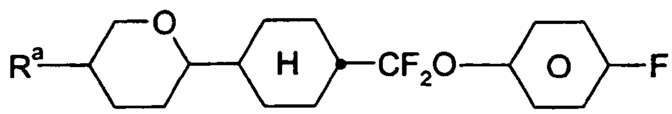

A-25

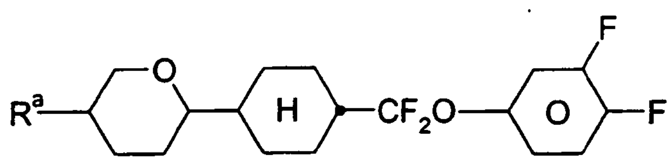

A-26

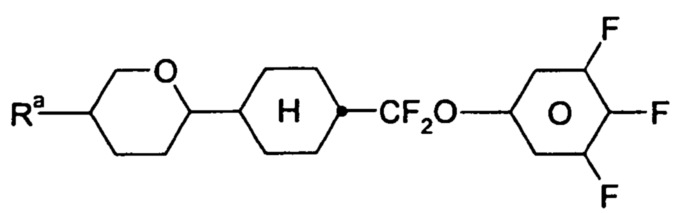

A-27

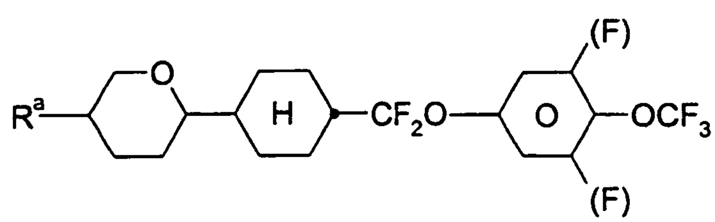

A-28

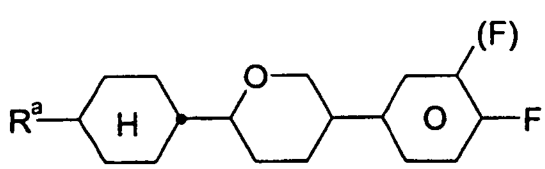

A-29

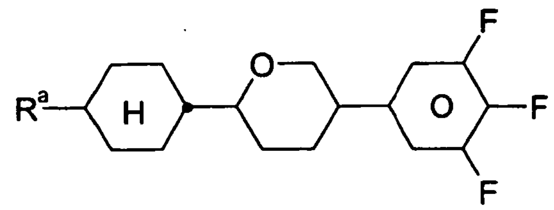

A-30

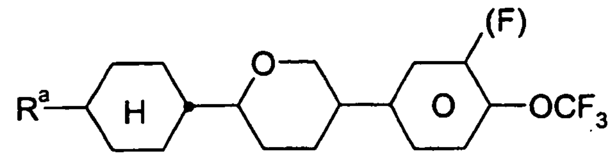

A-31

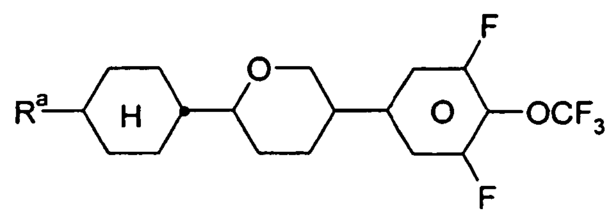

A-32

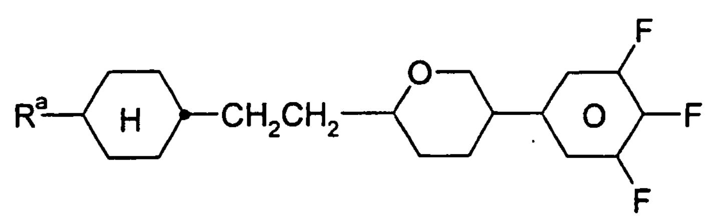

A-33

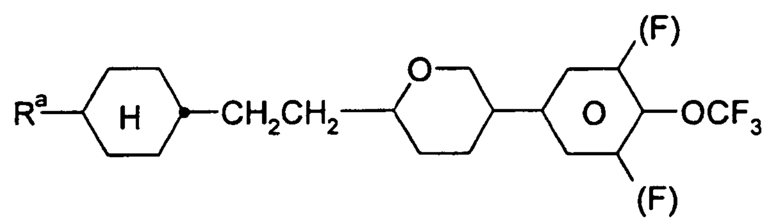

A-34

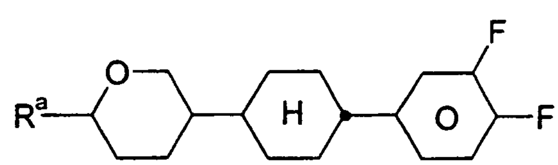

A-35

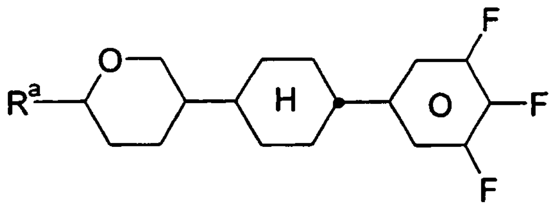

A-36

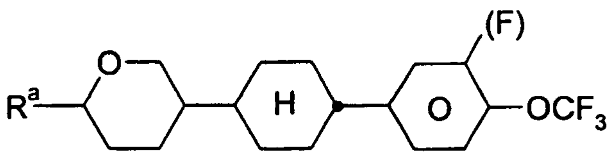

A-37

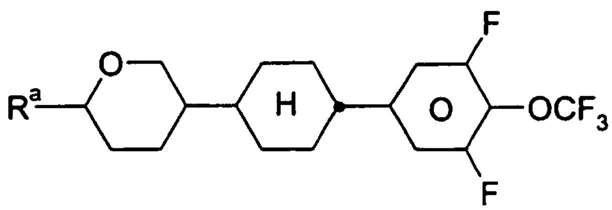

A-38

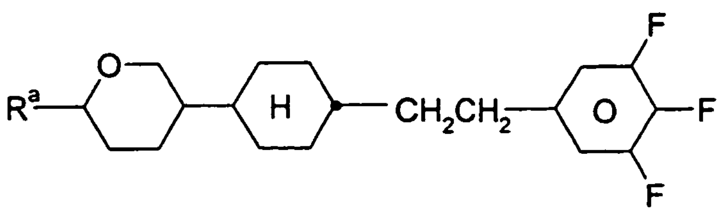

A-39

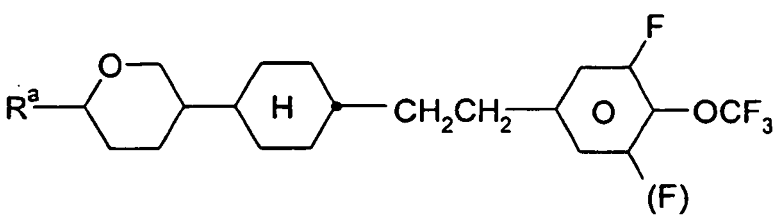

A-40

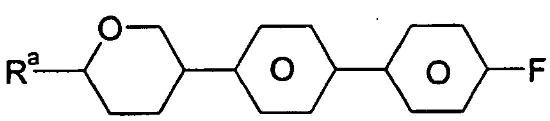

A-41

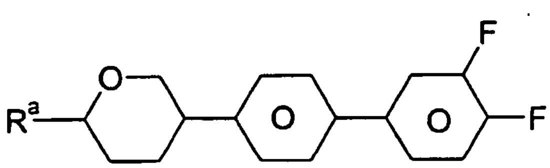

A-42

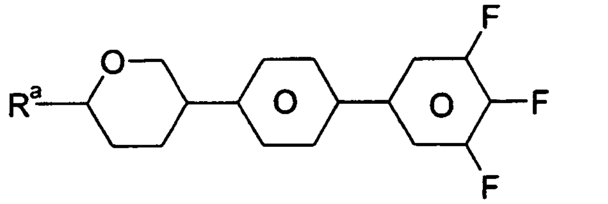

A-43

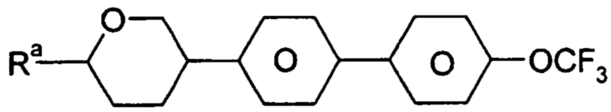

A-44

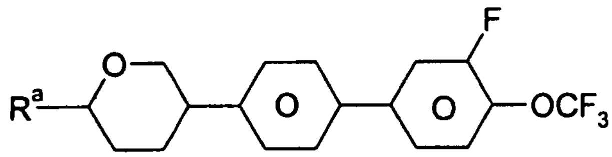

A-45

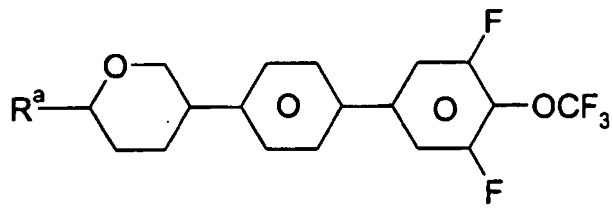

A-46

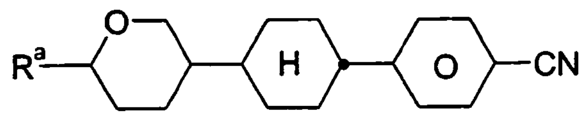

A-47

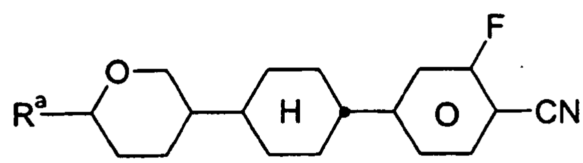

A-48

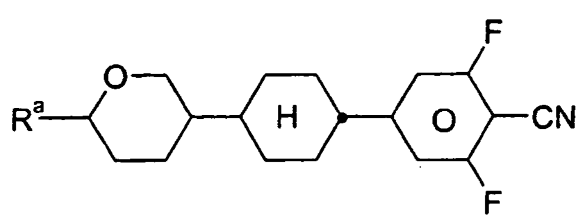

A-49

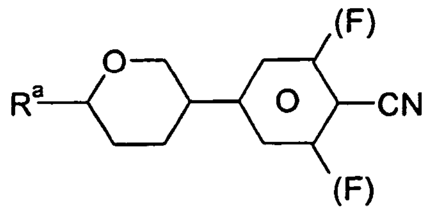

A-50

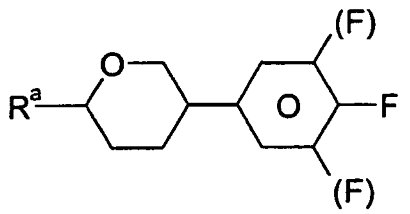

A-51

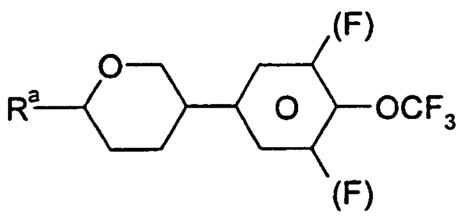

A-52

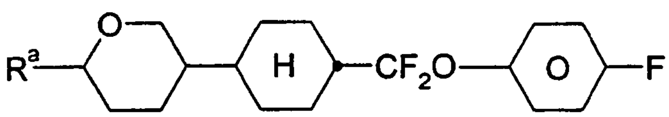

A-53

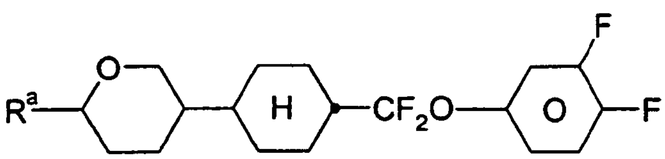

A-54

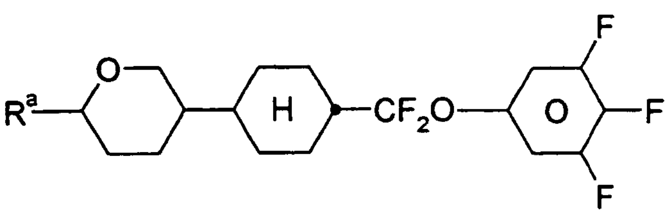

A-55

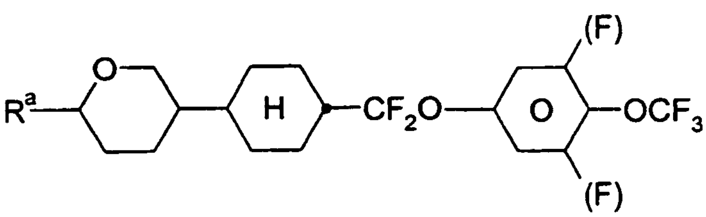

A-56

worin $R^a$ die in Anspruch 1 angegebenen Bedeutungen besitzt.

Gegenstand der Erfindung sind auch Verbindungen der Formel A,

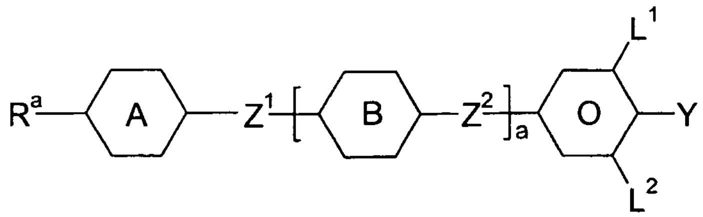

A

worin

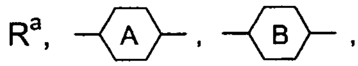

a, $L^2$ und Y die in Anspruch 1 angegebenen Bedeutungen haben,

$L^1$ F, und

$Z^1$ und $Z^2$ jeweils unabhängig voneinander $-CH_2CH_2-$, $-(CH_2)_4-$, $-CH_2O-$, $-OCH_2-$, $-OCF_2-$, -C≡C-, -CF=CF-, -CH=CH-, -COO-, $-CH_2-$,$(CH_2)_3-$ oder eine Einfachbindung,

bedeutet,

insbesondere **Verbindungen** der **folgenden** Formeln:

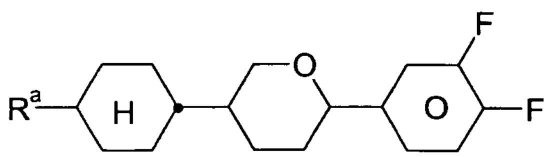

A-1*

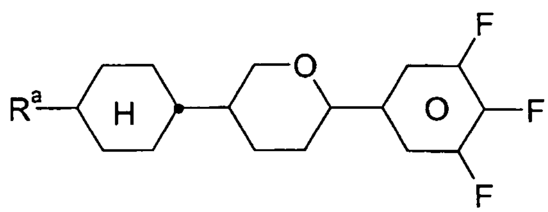

A-2

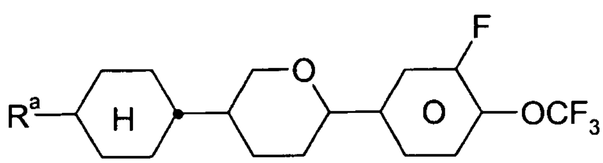

A-3*

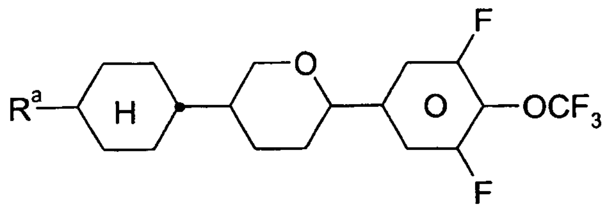

A-4

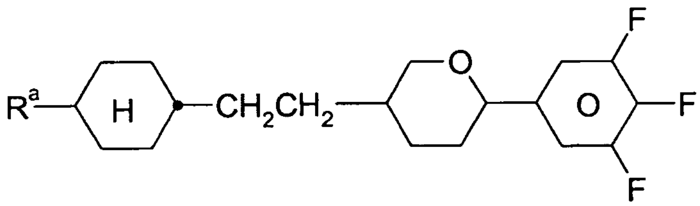

A-5

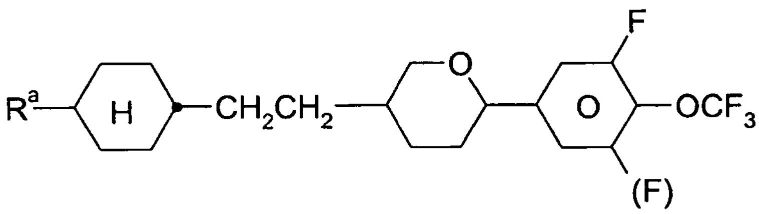

A-6*

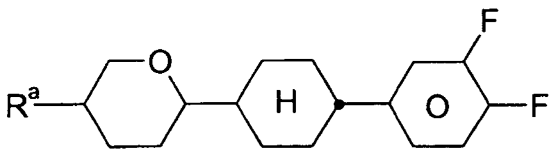

A-7

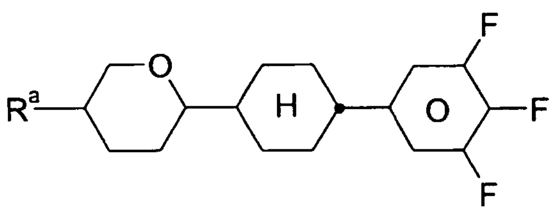

A-8

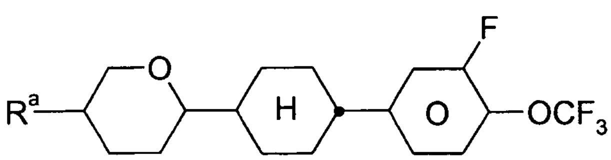

A-9*

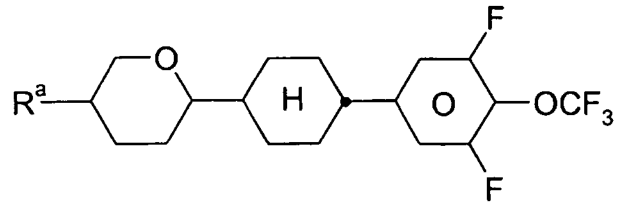

A-10

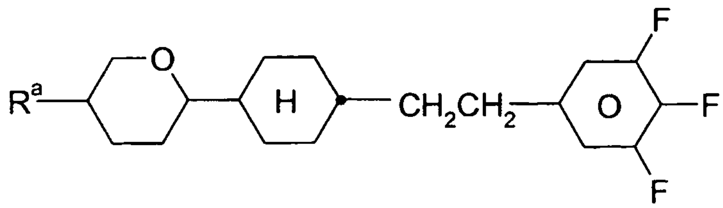

A-11

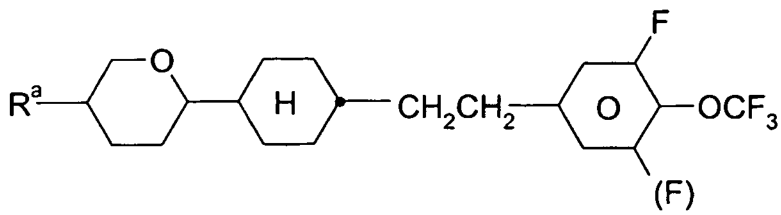

A-12

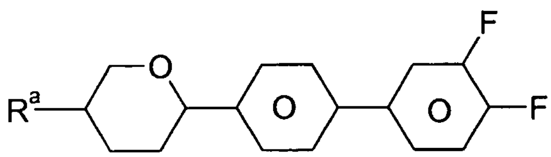

A-14

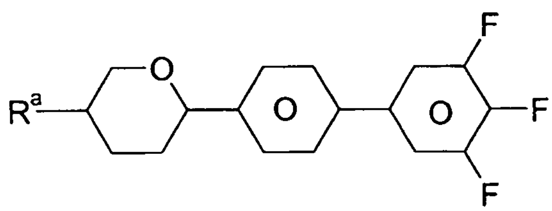

A-15

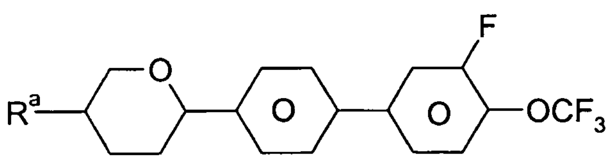

A-17

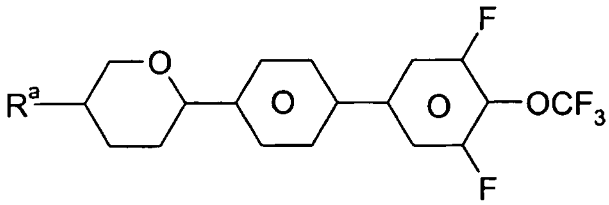

A-18

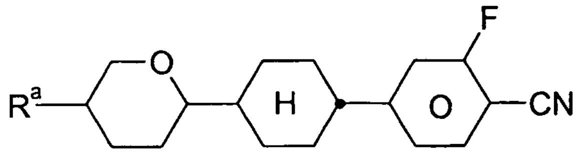

A-20

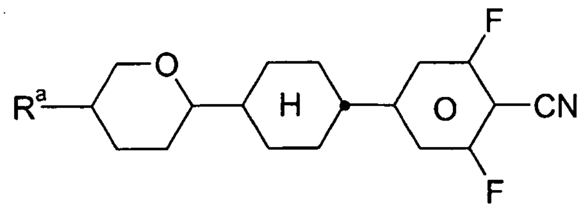

A-21

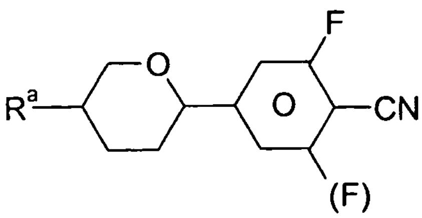

A-22*

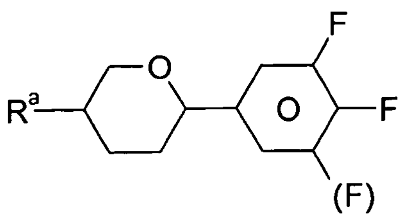

A-23*

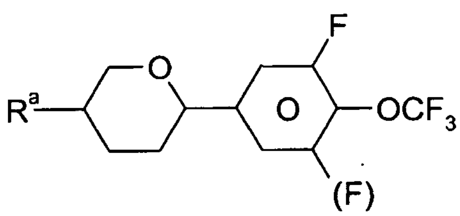

A-24*

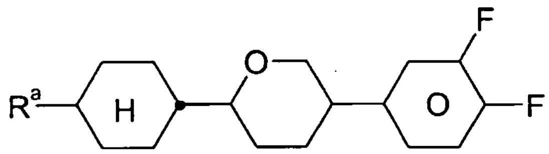

A-29*

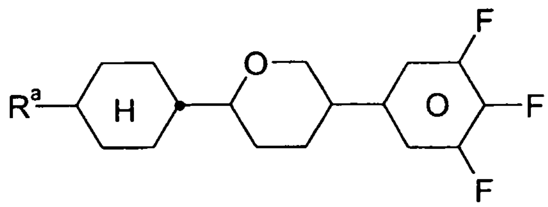

A-30

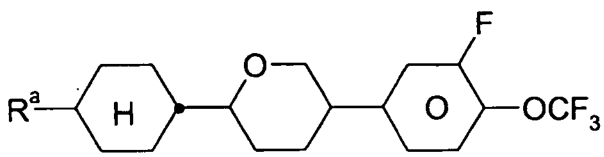

A-31*

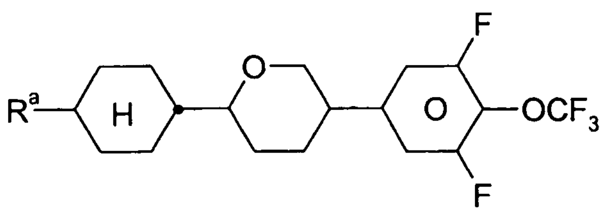

A-32

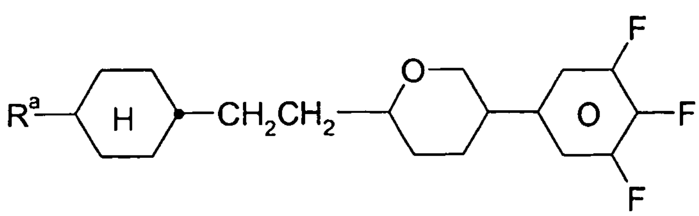

A-33

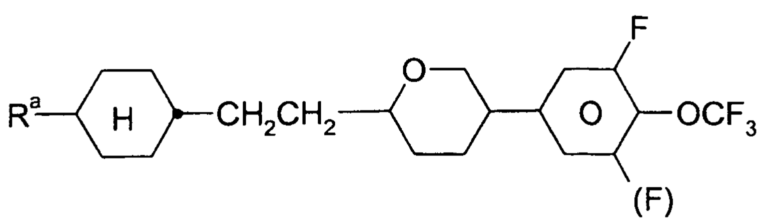

A-34*

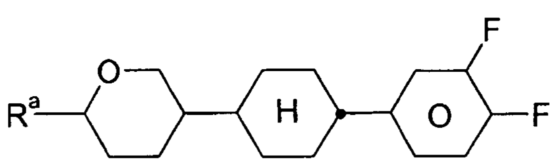

A-35

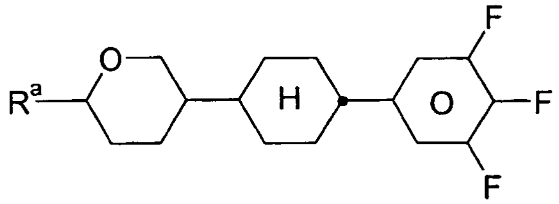 A-36

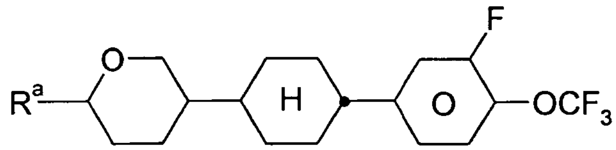 A-37*

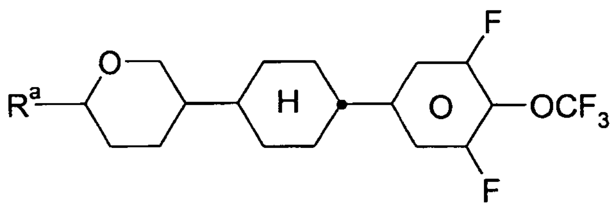 A-38

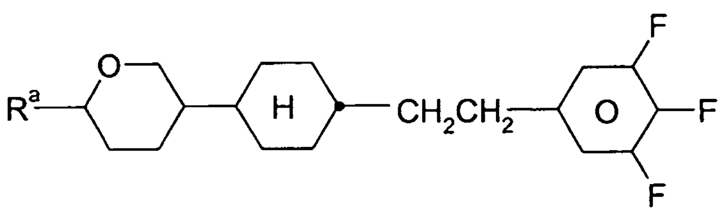 A-39

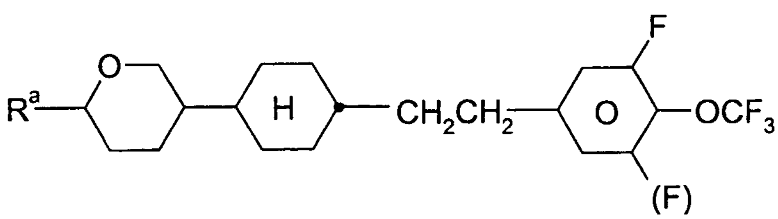 A-40

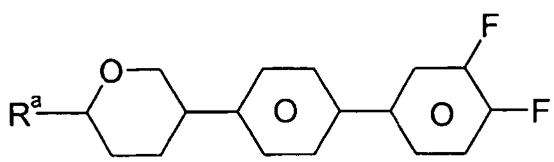 A-42

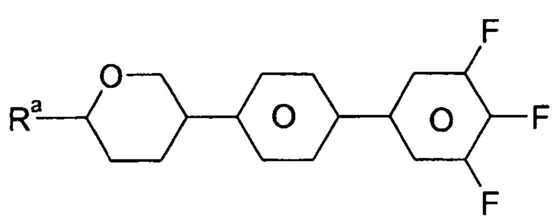 A-43

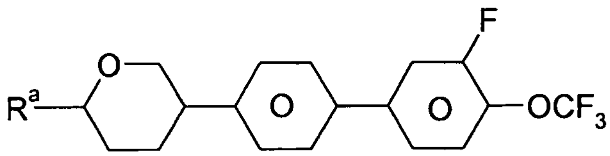

A-45

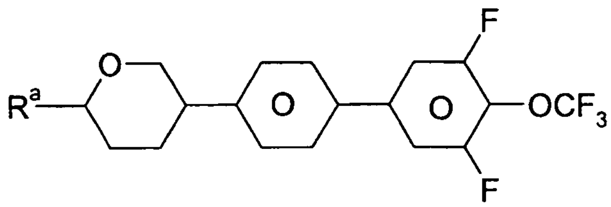

A-46

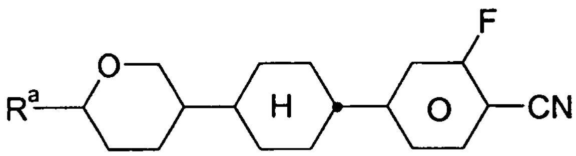

A-48

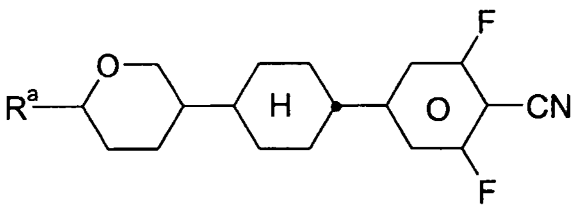

A-49

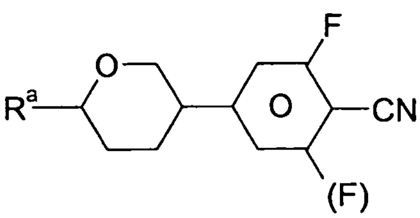

A-50*

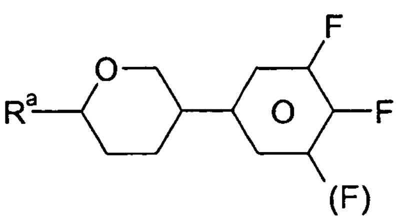

A-51*

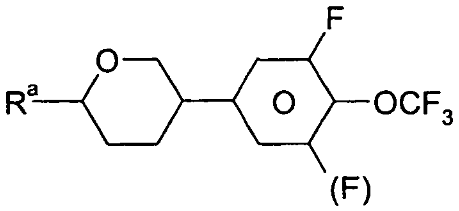

A-52*

Von den Verbindungen der Formeln A-1 bis A-56 sind in den erfindungsgemäßen Mischungen besonders bevorzugt die Verbindungen der Formeln A-1, A-2, A-3, A-4, A-7, A-8, A-9, A-10, A-13, A-14, A-15, A-16, A-17, A-18, A-22, A-25, A-26, A-27, A-28, ferner A-29, A-30, A-31, A-32, A-35, A-36, A-37, A-38, A-41, A-42, A-43, A-44, A-45, A-46, A-50, A-53, A-54, A-55 und A-56.

Insbesondere bevorzugt sind die Verbindungen der Formeln A-2, A-8, A-15, A-17, A-18 und A-27.

Besonders bevorzugt sind erfindungsgemäße Mischungen, die wenigstens eine Verbindung der Formel A-2 und/oder A-8, enthalten.

In den Formeln A und A-1 bis A-12 bedeutet $R^a$ besonders bevorzugt geradkettiges Alkyl oder Alkoxy, 1 E-Alkenyl oder 3E-Alkenyl mit 2 bis 7 C Atomen.

Y bedeutet vorzugsweise F, Cl, CN, $CF_3$, $C_2F_5$, $C_3F_7$, $CF_2H$, $OCF_3$, $OCF_2H$, $OCFHCF_3$, $OCFHCFH_2$, $OCFHCF_2H$, $OCF_2CH_3$, $OCF_2CFH_2$, $OCF_2CF_2H$, $OCF_2CF_2CF_2H$, $OCF_2CF_2CFH_2$, $OCFHCF_2CF_3$, $OCFHCF_2CF_2H$, $OCFHCFHCF_3$, $OCH_2CF_2CF_3$, $OCF_2CF_2CF_3$, $OCF_2CFHCFH_2$, $OCF_2CH_2CF_2H$, $OCFHCF_2CFH_2$, $OCFHCFHCF_2H$, $OCFHCH_2CF_3$, $OCH_2CFHCF_3$, $OCH_2CF_2CF_2H$, $OCF_2CFHCH_3$, $OCF_2CH_2CFH_2$, $OCFHCF_2CH_3$, $OCFHCFHCFH_2$, $OCFHCH_2CF_3$, $OCH_2CF_2CFH_2$, $OCH_2CFHCF_2H$, $OCF_2CH_2CH_3$, $OCFHCFHCH_3$, $OCFHCH_2CFH_2$, $OCH_2CF_2CH_3$, $OCH_2CFHCFH_2$, $OCH_2CH_2CF_2H$, $OCHCH_2CH_3$, $OCH_2CFHCH_3$, $OCH_2CH_2CF_2H$, $OCClFCF_3$, $OCClFCClF_2$, $OCClFCFH_2$, $OCFHCCl_2F$, $OCClFCF_2H$, $OCClFCClF_2$, $OCF_2CClH_2$, $OCF_2CCl_2H$, $OCF_2CCl_2F$, $OCF_2CClFH$, $OCF_2CClF_2$, $OCF_2CF_2CClF_2$, $OCF_2CF_2CCl_2F$, $OCClFCF_2CF_3$, $OCClFCF_2CF_2H$, $OCClFCF_2CClF_2$, $OCClFCFHCF_3$, $OCClFCClFCF_3$, $OCCl_2CF_2CF_3$, $OCClHCF_2CF_3$, $OCClFCF_2CF_3$, $OCClFCClFCF_3$, $OCF_2CClFCFH_2$, $OCF_2CF_2CCl_2F$, $OCF_2CCl_2CF_2H$, $OCF_2CH_2CClF_2$, $OCClFCF_2CFH_2$, $OCFHCF_2CCl_2F$, $OCClFCFHCF_2H$, $OCClFCClFCF_2H$, $OCFHCFHCClF_2$, $OCClFCH_2CF_3$, $OCFHCCl_2CF_3$, $OCCl_2CFHCF_3$, $OCH_2CClFCF_3$, $OCCl_2CF_2CF_2H$, $OCH_2CF_2CClF_2$, $OCF_2CClFCH_3$, $OCF_2CFHCCl_2H$, $OCF_2CCl_2CFH_2$, $OCF_2CH_2CCl_2F$, $OCClFCF_2CH_3$, $OCFHCF_2CCl_2H$, $OCClFCClFCFH_2$, $OCFHCFHCCl_2F$, $OCClFCH_2CF_3$, $OCFHCCl_2CF_3$, $OCCl_2CF_2CFH_2$, $OCH_2CF_2CCl_2F$, $OCCl_2CFHCF_2H$, $OCClHCClFCF_2H$, $OCF_2CClHCClH_2$, $OCF_2CH_2CCl_2H$, $OCClFCFHCH_3$, $OCF_2CClFCCl_2H$, $OCClFCH_2CFH_2$, $OCFHCCl_2CFH_2$, $OCCl_2CF_2CH_3$, $OCH_2CF_2CClH_2$, $OCCl_2CFHCFH_2$, $OCH_2CClFCFCl_2$, $OCH_2CH_2CF_2H$, $OCClHCClHCF_2H$, $OCH_2CCl_2CF_2H$, $OCClFCH_2CH_3$, $OCFHCH_2CCl_2H$, $OCClHCFHCClH_2$, $OCH_2CFHCCl_2H$, $OCCl_2CH_2CF_2H$, $OCH_2CCl_2CF_2H$, $CH=CF_2$, $CF=CF_2$, $OCH=CF_2$, $OCF=CF_2$, CH=CHF, OCH=CHF, CF=CHF, OCF=CHF, insbesondere F, Cl, CN, $CF_3$, $CF_2H$, $C_2F_5$, $C_3F_7$, $OCF_3$, $OCF_2H$, $OCFHCF_3$, $OCFHCFH_2$, $OCFHCF_2H$, $OCF_2CH_3$, $OCF_2CFH_2$, $OCF_2CF_2H$, $OCF_2CF_2CF_2H$, $OCF_2CF_2CFH_2$, $OCFHCF_2CF_3$, $OCFHCF_2CF_2H$, $OCF_2CF_2CF_3$, $OCF_2CHFCF_3$, $OCClFCF_2CF_3$.

Formel B umfasst insbesondere Verbindungen der Unterformeln B-1 bis B-6,

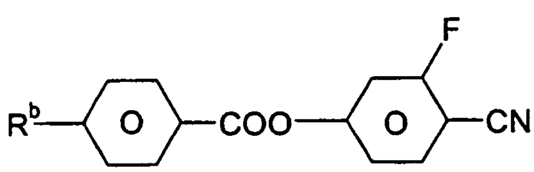

B-1

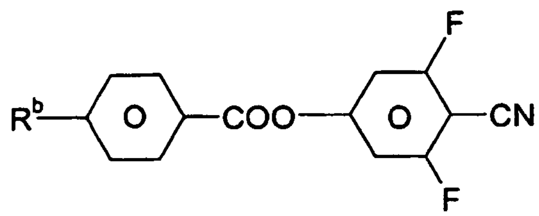

B-2

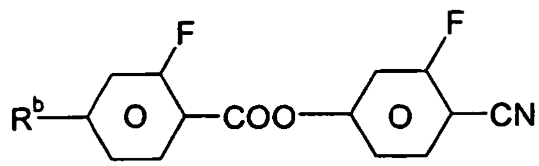

B-3

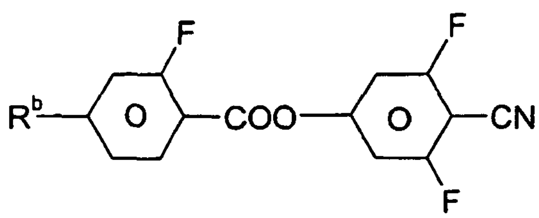

B-4

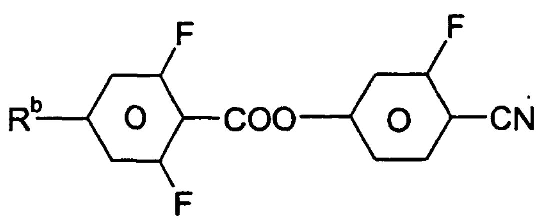

B-5

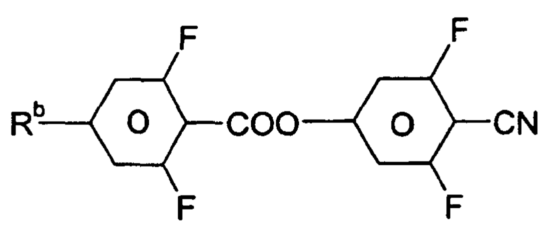

B-6

worin $R^b$ die in Anspruch 1 angegebenen Bedeutungen besitzt.

Besonders bevorzugt sind Verbindungen, worin $R^b$ ein geradkettiger Alkylrest mit 1-7 C-Atomen oder ein Alkenylrest mit 2-7 C-Atomen ist. Insbesondere bevorzugt sind Verbindungen der Formeln B-1, B-2 und B-4.

Die Verwendung von Verbindungen der Formeln A und B führt in den erfindungsgemäßen Flüssigkristallmischungen zu besonders niedrigen Werten der Rotationsviskosität und zu TN- und STN-Anzeigen mit einer hohen Steilheit und schnellen Schaltzeiten insbesondere bei niedrigen Temperaturen.

Die Komponente A bzw. die erfindungsgemäße flüssigkristalline Mischung enthält neben den Verbindungen der Formel A, vorzugsweise zusätzlich eine oder mehr 3,4,5-Trifluorphenylverbindungen ausgewählt aus der Gruppe

der Verbindungen der Formeln IIa bis IIk,

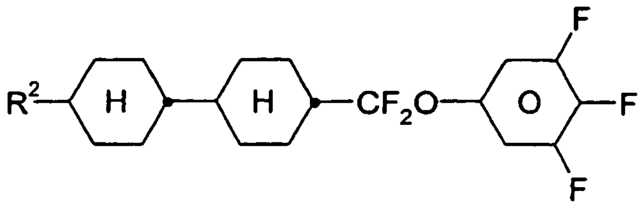

IIa

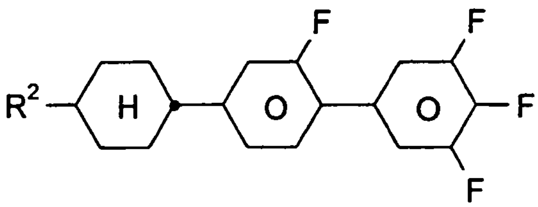

IIb

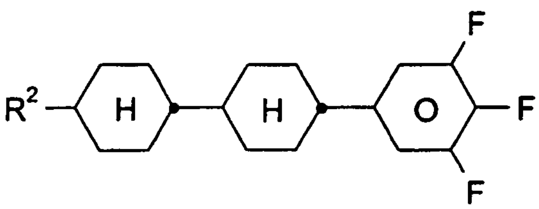

IIc

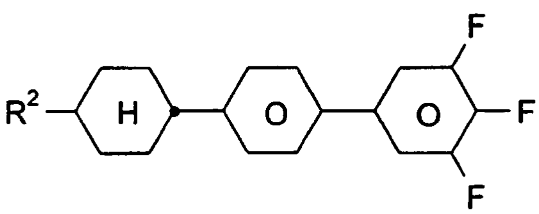

IId

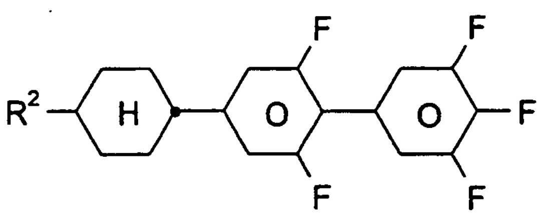

IIe

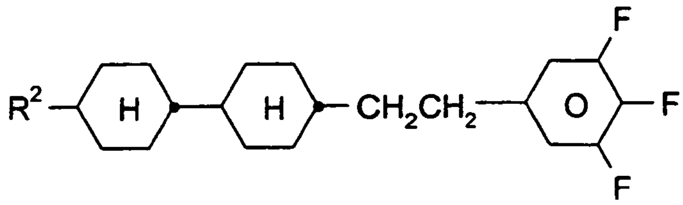

IIf

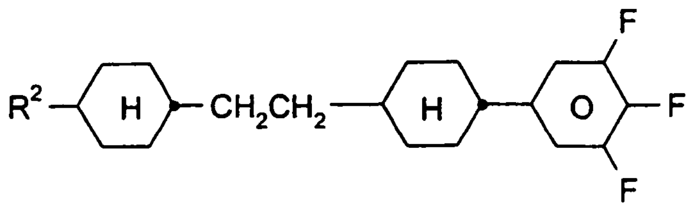

IIg

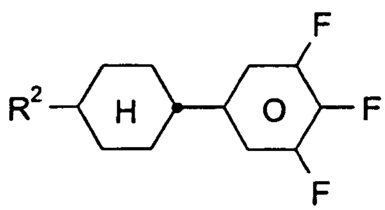

IIh

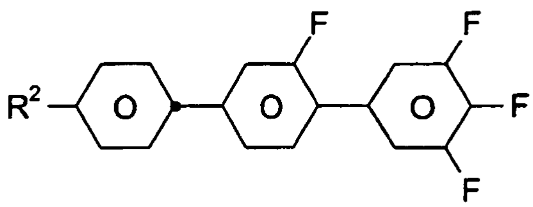

IIi

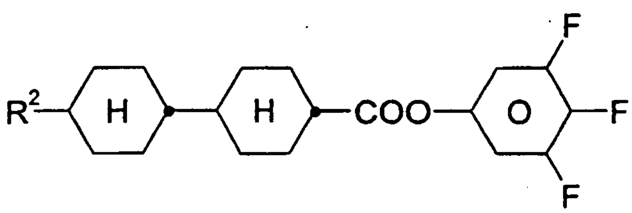

IIj

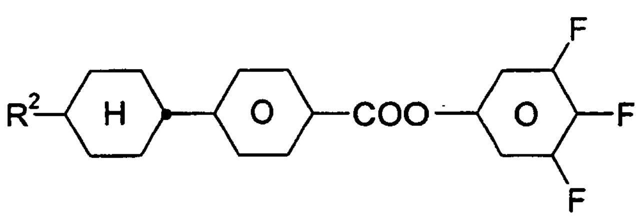

IIk

worin

$R^2$ H, einen unsubstituierten, einen einfach durch CN oder $CF_3$ oder einen mindestens einfach durch Halogen substituierten Alkylrest mit 1 bis 12 C-Atomen, wobei in diesen Resten auch eine oder mehrere $CH_2$-Gruppen jeweils unabhängig voneinander durch -O-, -S-,

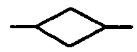 ,

-CH=CH-, -C≡C-, -CO-, -CO-O-, -O-CO- oder -O-CO-O- so ersetzt sein können, dass O-Atome nicht direkt miteinander verknüpft sind,

bedeutet.

Das erfindungsgemäße Medium kann neben den Verbindungen der Formeln A und B zusätzlich eine oder mehrere Verbindungen mit polarer Endgruppe der Formeln II*a bis II*s enthalten,

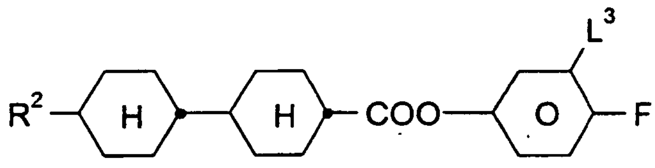

II*a

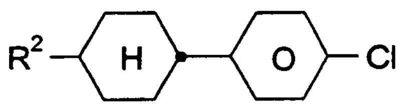

II*b

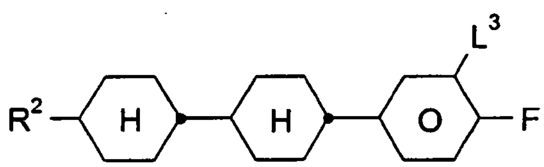

II*c

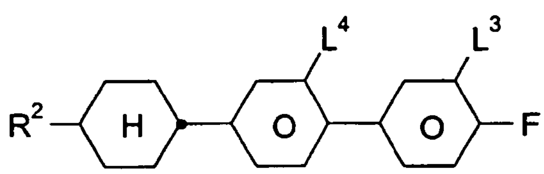

II*d

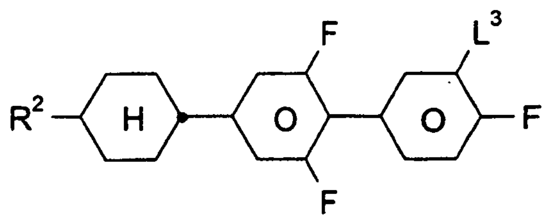

II*e

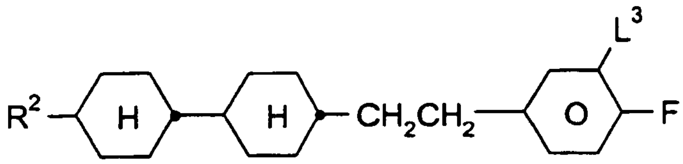

II*f

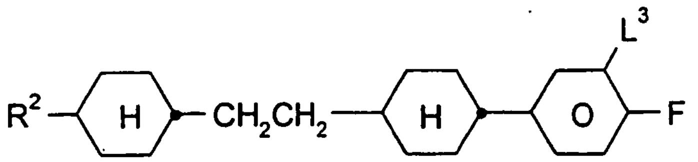

II*g

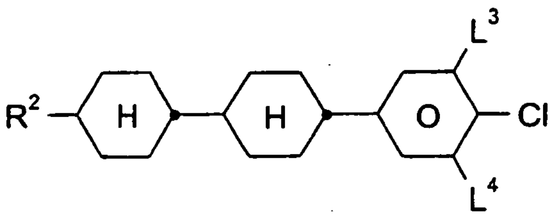

II*h

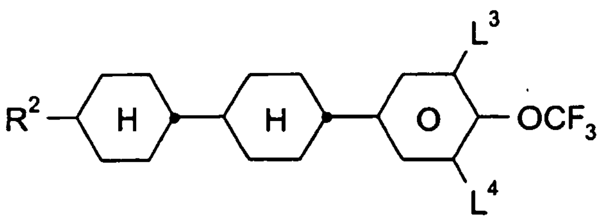

II*i

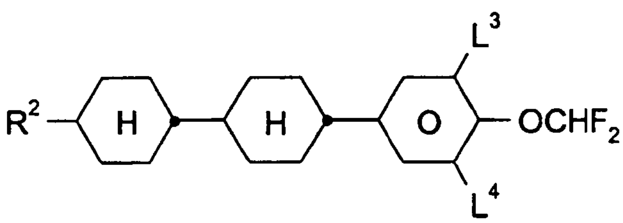

II*k

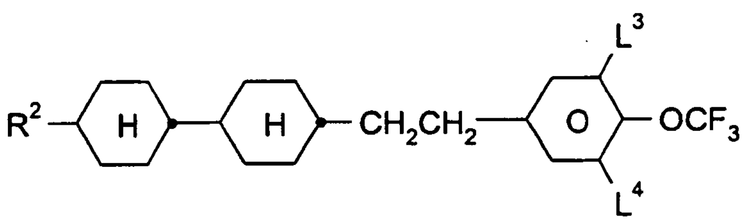

II*m

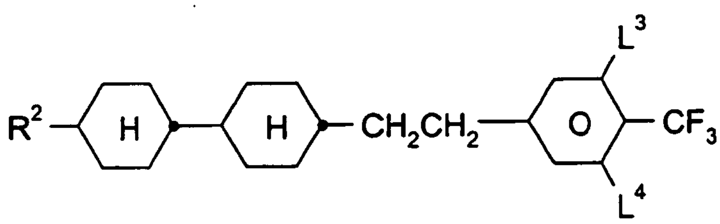

II*n

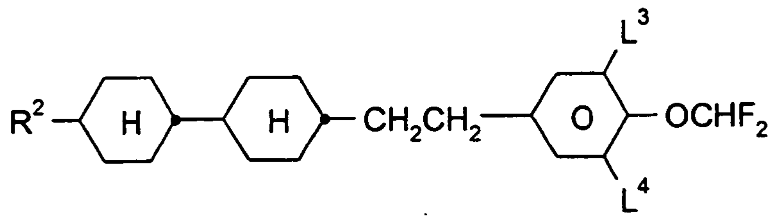

II*o

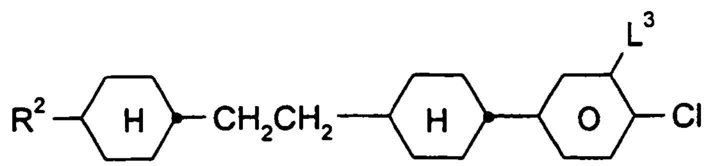

II*p

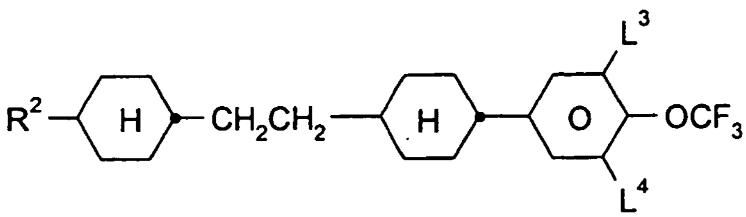

II*q

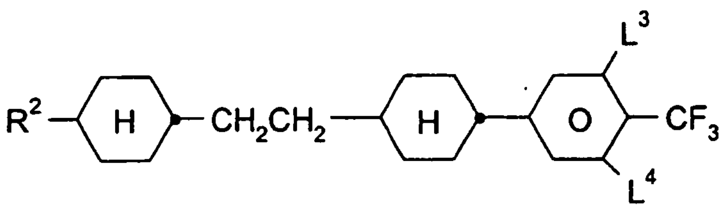

II*r

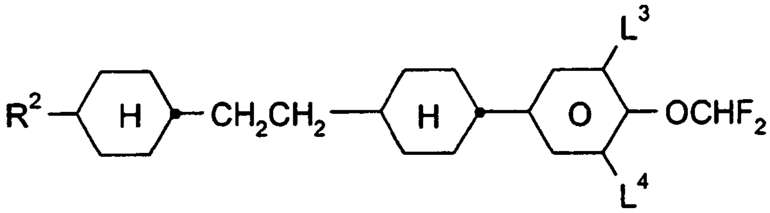

II*s

worin $R^2$ eine der für $R^a$ angegebenen Bedeutungen besitzt und $L^3$ und $L^4$ jeweils unabhängig voneinander H oder F bedeuten. $R^2$ bedeutet in diesen Verbindungen besonders bevorzugt Alkyl, Alkenyl oder Alkoxy mit bis zu 7 C-Atomen.

Besonders bevorzugt enthält das erfindungsgemäße Medium bzw. Komponente A Verbindungen der Formeln IIa, IIb, IIc, IId, IIe, IIf, IIg, IIj, II*b, II*c, II*d, II*f und/oder II*i, insbesondere Verbindungen der Formeln IIa, IIb, IId, IIi, II*a und II*i.

Die erfindungsgemäße Mischung enthält vorzugsweise neben einer oder mehreren Verbindungen der Formel B eine oder mehrere Cyanoverbindungen der Formeln IIIa bis IIIj:

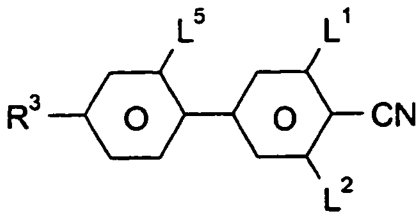

IIIa

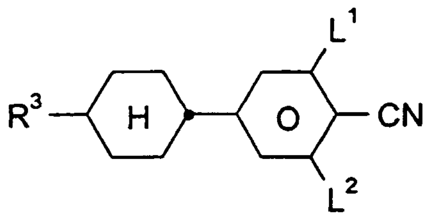

IIIb

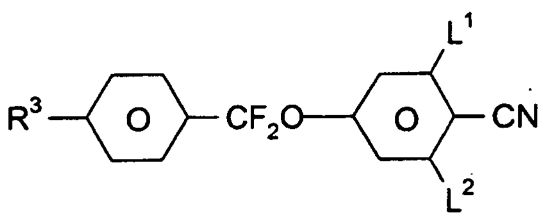

IIIc

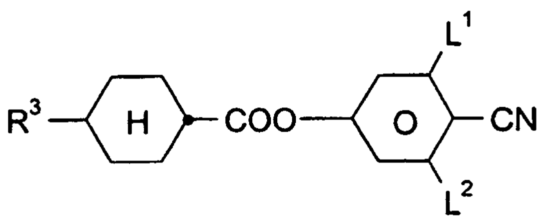

IIId

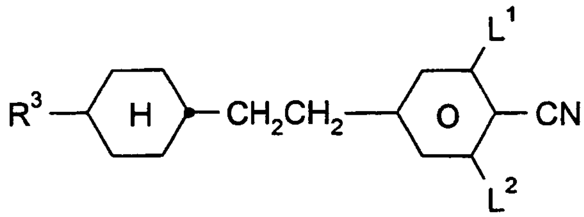

IIIe

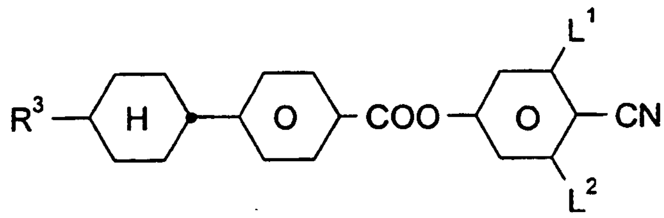

IIIf

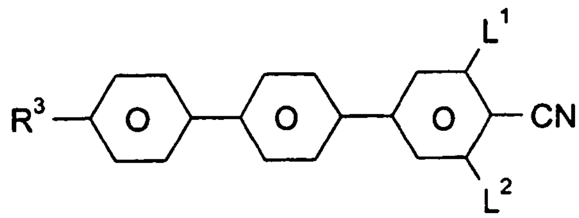

IIIg

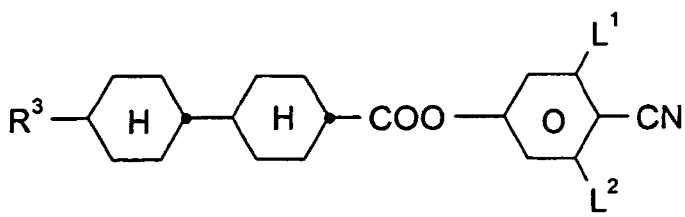

IIIh

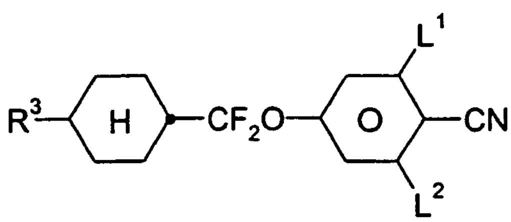

IIIi

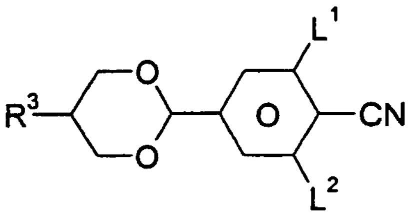

IIIj

worin $R^3$ eine der für $R^a$ angegebenen Bedeutungen besitzt und $L^1$, $L^2$ und $L^5$ jeweils unabhängig voneinander H oder F bedeuten. $R^3$ bedeutet in diesen Verbindungen besonders bevorzugt Alkyl, Alkenyl oder Alkoxy mit bis zu 7 C-Atomen.

Besonders bevorzugt sind Mischungen, die eine oder mehrere Verbindungen der Formeln IIIb, IIIc, IIIf und IIIj, insbesondere solche, worin $L^1$ und/oder $L^2$ F bedeuten, enthalten.

Weiterhin bevorzugt sind Mischungen, die eine oder mehrere Verbindungen der Formel IIIf und/oder IIIg enthalten, worin $L^2$ H und $L^1$ H oder F, insbesondere F, bedeutet.

Die einzelnen Verbindungen der Formeln A, B sowie der Formeln IIa-IIk, II*a-II*s und IIIa bis IIIj bzw. deren Unterformeln oder auch andere Verbindungen, die in den erfindungsgemäßen Mischungen bzw. TN- und STN-Anzeigen verwendet werden können, sind entweder bekannt, oder sie können analog zu den bekannten Verbindungen hergestellt werden.

Die Verbindungen der Formel A besitzen niedrige Viskositäten, insbesondere niedrige Rotationsviskositäten, sowie niedrige Werte für das Verhältnis der elastischen Konstanten $K_{33}/K_{11}$, und führen daher zu in den erfindungsgemäßen Anzeigen zu kurzen Schaltzeiten, während die Anwesenheit von Verbindungen der Formel B mit hoher dielektrischer Anisotropie, insbesondere in erhöhten Konzentrationen, eine Verringerung der Viskosität bewirkt.

Bevorzugte Flüssigkristallmischungen enthalten eine oder mehrere Verbindungen der Komponente A vorzugsweise in einem Anteil von 15 % bis 75 %, besonders bevorzugt von 20 % bis 65 %. Diese Verbindungen besitzen eine dielektrische Anisotropie von $\Delta\varepsilon \geq +3$, insbesondere von $\Delta\varepsilon \geq +8$, besonders bevorzugt von $\Delta\varepsilon \geq +12$.

Weitere bevorzugte Mischungen enthalten

- eine oder mehrere, insbesondere zwei bis vier, Verbindungen der Formel A,
- jeweils eine, zwei oder drei Verbindungen der Formel A,
- eine oder mehrere, insbesondere eine oder zwei, Verbindungen der Formel B,
- eine oder mehrere, insbesondere zwei bis fünf, Verbindungen der Formel IIIa, IIIb, IIIc und/oder IIIf.

Bevorzugte Flüssigkristallmischungen enthalten eine oder mehrere Verbindungen der Komponente B, vorzugsweise 2 bis 85 %, insbesondere 5 bis 80 %, ganz besonders bevorzugt 5 bis 75 %. Die Verbindungen der Gruppe B zeichnen sich insbesondere durch ihre niedrigen Werte für die Rotationsviskosität $\gamma_1$ aus.

Die Komponente B enthält vorzugsweise eine oder mehrere Verbindungen der Formel IV,

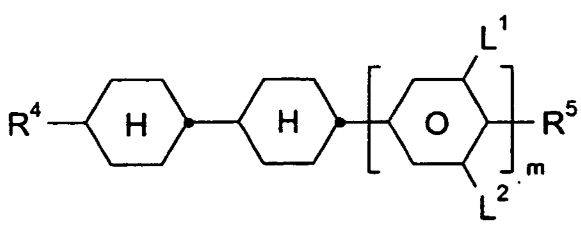

IV

worin

m 0 oder 1,

$R^4$ eine Alkenylgruppe mit 2 bis 7 C-Atomen,

| | |
|---|---|
| $R^5$ | eine der für $R^a$ angegebenen Bedeutungen, oder falls m = 1 ist, auch F, Cl, $CF_3$, $OCF_3$ bedeutet, |
| $L^1$ und $L^2$ | jeweils unabhängig voneinander H oder F |

bedeuten.

Besonders bevorzugte Verbindungen der Formel IV sind solche, worin $R^4$ Alkenyl mit 2 bis 7 C-Atomen bedeuten, insbesondere solche der folgenden Formeln

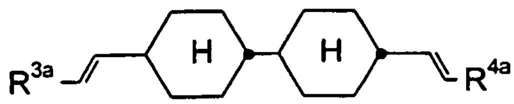

IV-1

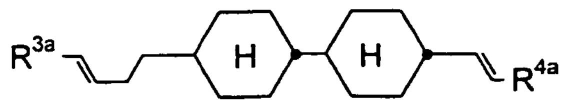

IV-2

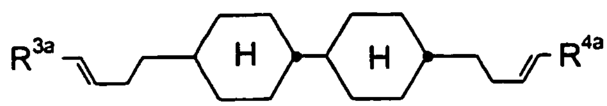

IV-3

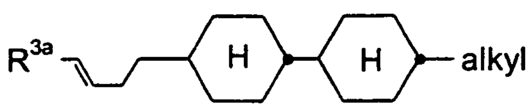

IV-4

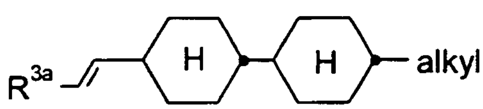

IV-5

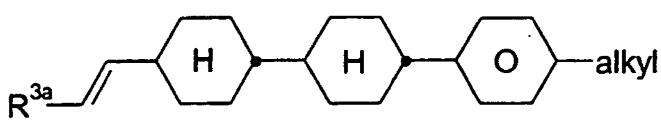

IV-6

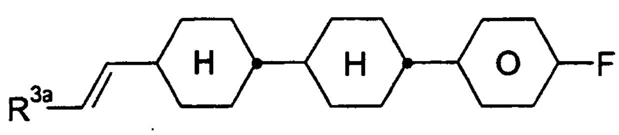

IV-7

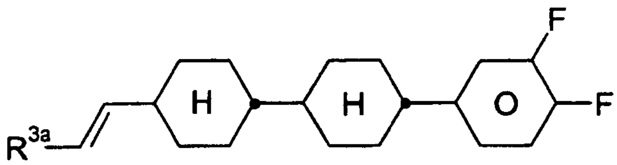

IV-8

worin $R^{3a}$ und $R^{4a}$ jeweils unabhängig voneinander H, $CH_3$, $C_2H_5$ oder n-$C_3H_7$ und alkyl eine Alkylgruppe mit 1 bis 7 C-Atomen bedeuten.

Besonders bevorzugt sind erfindungsgemäße TN- und STN-Anzeigen, worin die Flüssigkristallmischung mindestens eine Verbindung der Formel IV-1 und/oder IV-3 enthält, in denen $R^{3a}$ und $R^{4a}$ jeweils dieselbe Bedeutung aufweisen, sowie Anzeigen, worin die Flüssigkristallmischung mindestens eine Verbindung der Formel IV-5 enthält.

In einer weiteren bevorzugten Ausführungsform enthalten die erfindungsgemäßen Mischungen eine oder mehrere Verbindungen der Formel IV-6.

Die Komponente B enthält weiterhin vorzugsweise Verbindungen ausgewählt aus der Gruppe bestehend aus den Zweiringverbindungen der Formeln V-1 bis V-9,

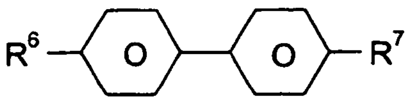

V-1

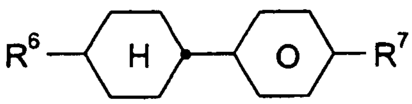

V-2

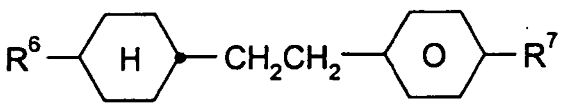

V-3

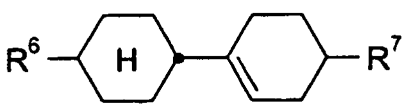

V-4

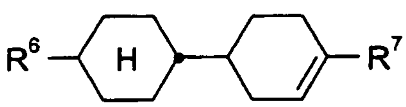

V-5

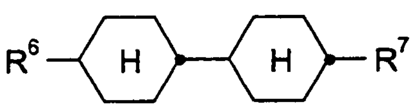

V-6

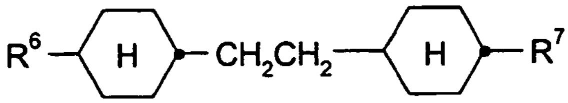

V-7

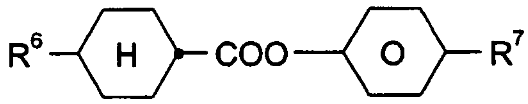

V-8

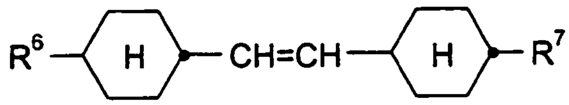

V-9

und/oder eine oder mehrere Verbindungen ausgewählt aus der Gruppe bestehend aus den Dreiringverbindungen der Formeln V-10 bis V-27,

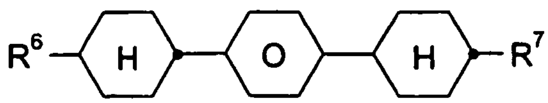

V-10

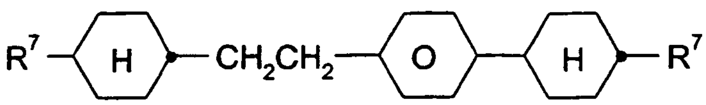

V-11

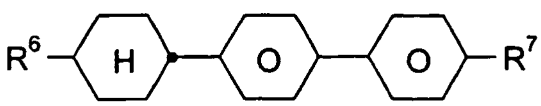

V-12

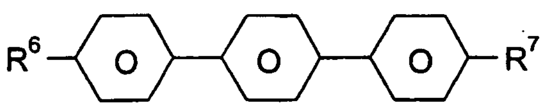

V-13

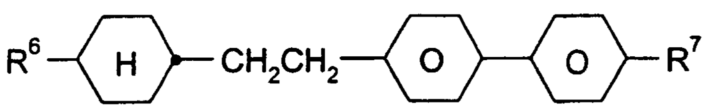

V-14

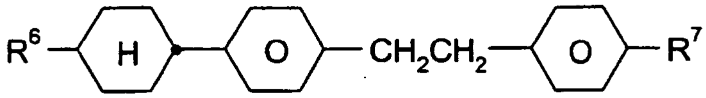

V-15

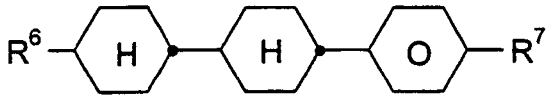

V-16

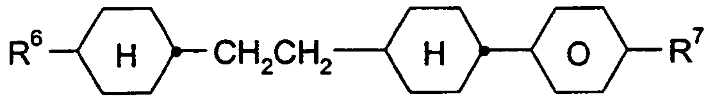

V-17

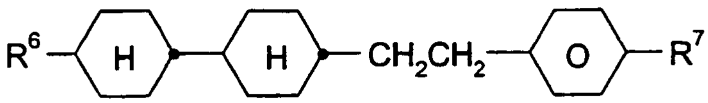

V-18

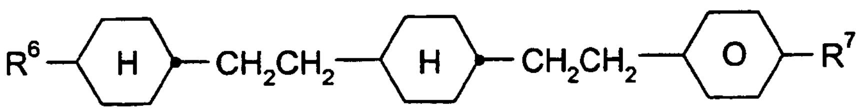

V-19

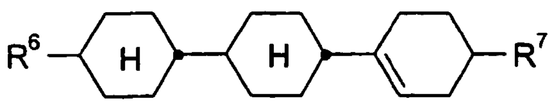

V-20

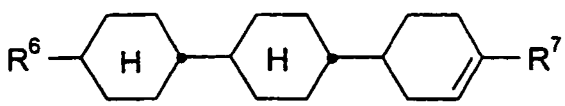

V-21

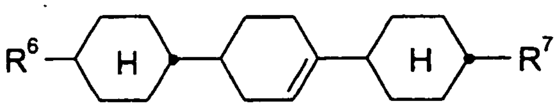

V-22

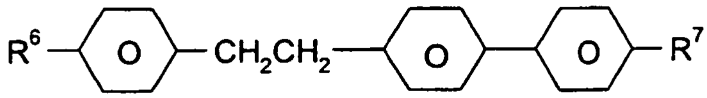

V-23

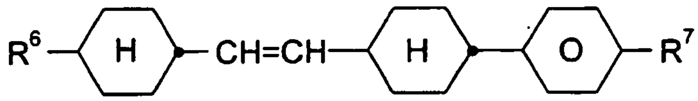

V-24

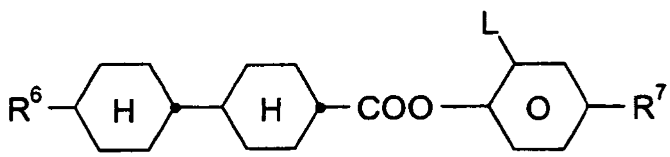

V-25

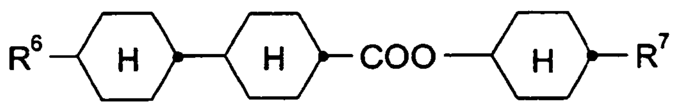

V-26

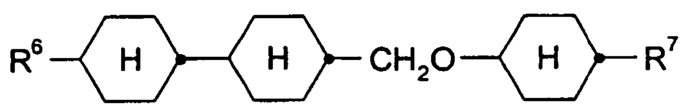

V-27

und/oder eine oder mehrere Verbindungen ausgewählt aus der Gruppe bestehend aus den Vierringverbindungen der Formeln V-28 bis V-34,

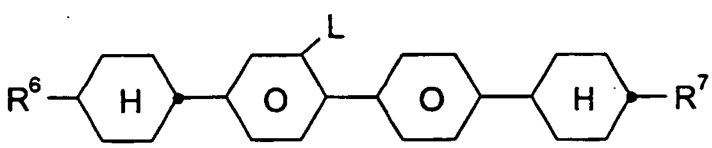

V-28

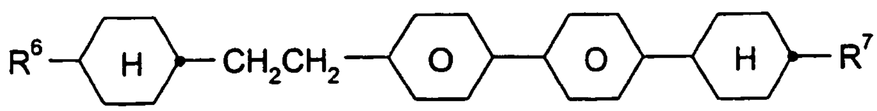

V-29

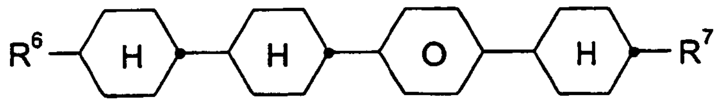

V-30

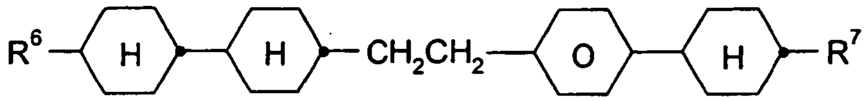

V-31

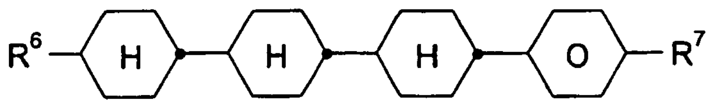

V-32

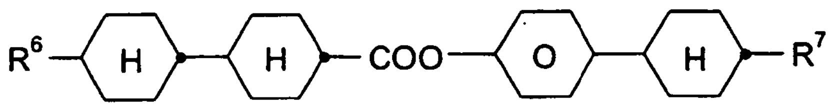

V-33

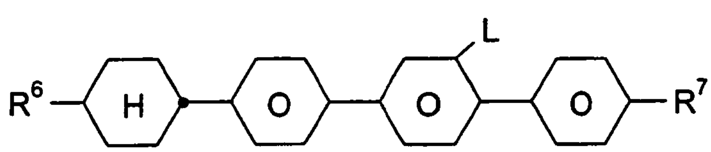

V-34

worin $R^6$ und $R^7$ die für $R^a$ in Anspruch 1 angegebenen Bedeutungen haben, L H oder F bedeutet.

Besonders bevorzugt sind Verbindungen der Formeln V-25 bis V-34, worin $R^6$ Alkyl und $R^7$ Alkyl oder Alkoxy, insbesondere Alkoxy, jeweils mit 1 bis 7 C-Atomen, bedeutet. Ferner bevorzugt sind Verbindungen der Formel V-25, V-28 und V-34, worin L F bedeutet.

$R^6$ und $R^7$ in den Verbindungen der Formeln V-1 bis V-34 bedeuten besonders bevorzugt geradkettiges Alkyl oder Alkoxy mit 1 bis 12 C-Atomen.

Besonders bevorzugt sind erfindungsgemäße Mischungen, die eine oder mehrere Verbindungen der Formel B-3a und/oder B-5a

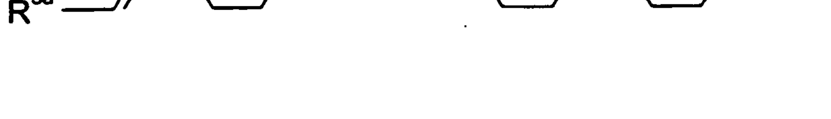

B-3a

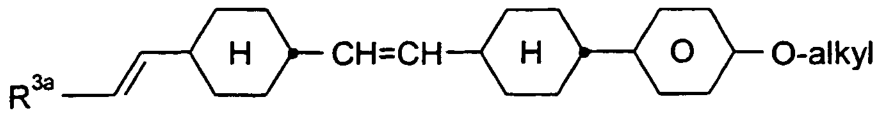 B-5a

enthalten. Vorzugsweise enthalten die Mischungen 2-25 Gew.%, insbesondere 2-15 Gew.% an Verbindungen der Formel B-5a.

Die flüssigkristallinen Mischungen enthalten gegebenenfalls eine optisch aktive Komponente C in einer Menge, dass das Verhältnis zwischen Schichtdicke (Abstand der Trägerplatten) und natürlicher Ganghöhe der chiralen nematischen Flüssigkristallmischung größer 0,2 ist. Für die Komponente stehen dem Fachmann eine Vielzahl, zum Teil kommerziell erhältlicher, chiraler Dotierstoffe zur Verfügung z.B. wie Cholesterylnonanoat, S-811, S-1011, S-2011, S-4011, S-5011 der Merck KGaA, Darmstadt und CB15 (BDH, Poole, UK). Die Wahl der Dotierstoffe ist an sich nicht kritisch.

Der Anteil der Verbindungen der Komponente C beträgt vorzugsweise 0 bis 10 %, insbesondere 0 bis 5 %, besonders bevorzugt 0 bis 3 %.

Die erfindungsgemäßen Mischungen können auch gegebenenfalls bis zu 20 % einer oder mehrerer Verbindungen mit einer dielektrischen Anisotropie von weniger als -2 (Komponente D) enthalten.

Falls die Mischungen Verbindungen der Komponente D enthalten, so sind dies vorzugsweise eine oder mehrere Verbindungen mit dem Strukturelement 2,3-Difluor-1,4-phenylen, z.B. Verbindungen gemäß DE-OS 38 07 801, 38 07 861, 38 07 863, 38 07 864 oder 38 07 908. Besonders bevorzugt sind Tolane mit diesem Strukturelement gemäß der Internationalen Patentanmeldung PCT/DE 88/00133.

Weitere bekannte Verbindungen der Komponente D sind z.B. Derivate der 2.3-Dicvanhvdrochinone oder Cyclohexanderivate mit dem Strukturelement

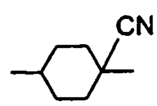 oder 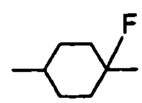

gemäß DE-OS 32 31 707 bzw. DE-OS 34 07 013.

Vorzugsweise enthalten die erfindungsgemäßen Flüssigkristallanzeigen keine Verbindungen der Komponente D.

Der Ausdruck "Alkenyl" in der Bedeutung von $R^a$, $R^b$, $R^2$, $R^3$, $R^4$, $R^5$, $R^6$ und $R^7$ umfasst geradkettige und verzweigte Alkenylgruppen, insbesondere die geradkettigen Gruppen. Besonders bevorzugte Alkenylgruppen sind $C_2$-$C_7$-1E-Alkenyl, $C_4$-$C_7$-3E-Alkenyl, $C_5$-$C_7$-4-Alkenyl, $C_6$-$C_7$-5-Alkenyl, und $C_7$-6-Alkenyl, insbesondere $C_2$-$C_7$-1E-Alkenyl, $C_4$-$C_7$-3E-Alkenyl und $C_5$-$C_7$-4-Alkenyl.

Beispiele bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Weitere bevorzugte Ausführungsformen beziehen sich auf erfindungsgemäßen Flüssigkristallmischungen, welche

- zusätzlich eine oder mehrere, besonders bevorzugt eine, zwei oder drei, heterocyclische Verbindungen der Formel Va und/oder Vb enthalten,

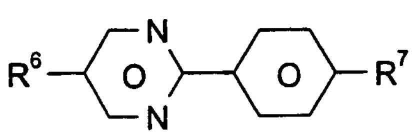 · Va

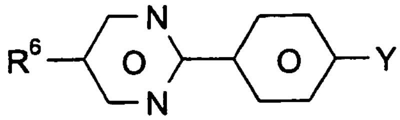

Vb

worin

| | |
|---|---|
| $R^6$ und $R^7$ | die oben angegebenen Bedeutungen besitzen und |
| Y | F oder Cl |

bedeuten,

- der Anteil der Verbindungen aus der Gruppe enthaltend Va und Vb ist vorzugsweise 2 bis 35 %, insbesondere 5 bis 20 %,

zusätzlich eine oder mehrere, besonders bevorzugt eine, zwei oder drei, Tolan-Verbindungen der Formeln T2a, T2b und/oder T2c enthalten,

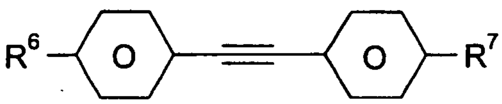

T2a

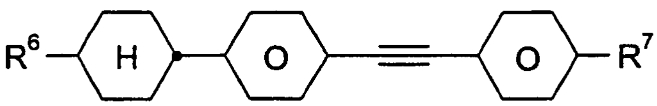

T2b

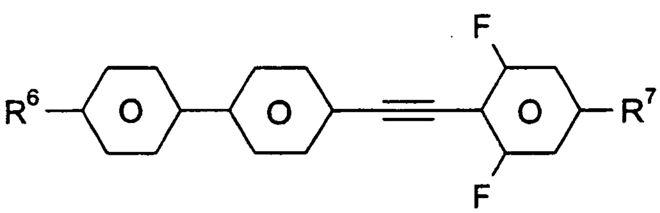

T2c

worin $R^6$ und $R^7$ die oben angegebene Bedeutung besitzen.

Der Anteil der Verbindungen aus der Gruppe enthaltend T2a, T2b und/oder T2c ist vorzugsweise 2 bis 20 %, insbesondere 4 bis 12 %. Vorzugsweise enthält die erfindungsgemäße Mischung zwei oder drei Verbindungen der Formeln T2a und/oder T2b.

In besonders bevorzugten Ausführungsformen enthalten die Mischungen

- mindestens eine Verbindung der Formel A-2

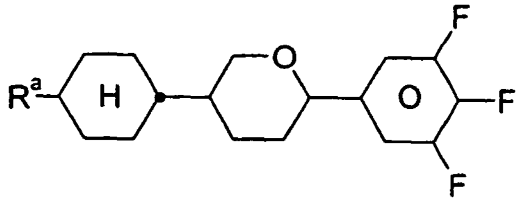

A-2

und
mindestens eine Verbindung der Formel B-1

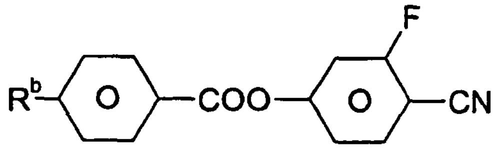

B-1

- mindestens eine Verbindung der Formel IIj;
- mindestens eine Tolan-Verbindung der Formel T2c;
- mindestens zwei, insbesondere drei Verbindungen der Formel A;
- mindestens zwei, insbesondere drei Verbindungen der Formel B;
- mindestens eine Verbindung der Formel T2a und mindestens eine Verbindung der Formel T2b;
- mindestens eine Verbindung der Formel VI,

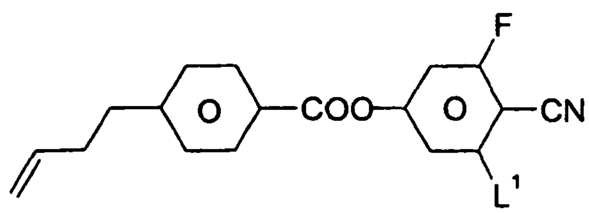

VI

worin $L^1$ H oder F bedeutet;

- mindestens eine Verbindung der Formel VII

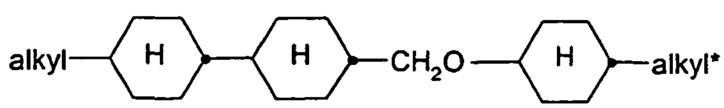

VII

worin alkyl bzw. alkyl* jeweils unabhängig voneinander eine Alkylgruppe mit 1 bis 7 C-Atomen bedeutet.

- mindestens 2 bis 30 Gew. % vorzugsweise 2 - 25 Gew. %, insbesondere 3 bis 15 Gew. % an Verbindungen der Formel VII
- mindestens 2,5 Gew.% der Verbindungen der Formel IV5;
- 5-30 Gew.%, der vorzugsweise 10-25 Gew.%, der Verbindungen der Formel A;

- 3-30 Gew.%, vorzugsweise 3-20 Gew.%, der Verbindungen der Formel B;

- mindestens drei Homologe der Verbindungen der Formel A, vorzugsweise bedeutet $R^a$ dann $C_2H_5$, n-$C_3H_7$ und n-$C_5H_{11}$.

Weitere besonders bevorzugte Ausführungsformen beziehen sich auf Flüssigkristallmischungen, die

- insgesamt drei bis sechs Verbindungen der Formeln A und B enthalten, wobei der Anteil dieser Verbindungen an der gesamten Mischung 25 bis 65 %, insbesondere 30 bis 55 %, beträgt,

- mehr als 20 % an Verbindungen mit positiver dielektrischer Anisotropie, insbesondere mit$\Delta\varepsilon \geq$ +12, enthalten,

Die erfindungsgemäßen Mischungen zeichnen sich insbesondere beim Einsatz in TN- und STN-Anzeigen mit hohen Schichtdicken durch sehr niedrige Summenschaltzeiten aus ($t_{ges} = t_{on} + t_{off}$).
Die in den erfindungsgemäßen TN- und STN-Zellen verwendeten Flüssigkristallmischungen sind dielektrisch positiv mit $\Delta\varepsilon \geq 1$. Besonders bevorzugt sind Flüssigkristallmischungen mit $\Delta\varepsilon \geq 3$, insbesondere mit $\Delta\varepsilon \geq 5$.
Die erfindungsgemäßen Flüssigkristallmischungen weisen günstige Werte für die Schwellenspannung $V_{10/0/20}$ und für die Rotationsviskosität $\gamma_1$ auf. Ist der Wert für den optischen Wegunterschied $d \cdot \Delta n$ vorgegeben, wird der Wert für die Schichtdicke d durch die optische Anisotropie $\Delta n$ bestimmt. Insbesondere bei relativ hohen Werten für $d \cdot \Delta n$ ist i.a. die Verwendung erfindungsgemäßer Flüssigkristallmischungen mit einem relativ hohen Wert für die optische Anisotropie bevorzugt, da dann der Wert für d relativ klein gewählt werden kann, was zu günstigeren Werten für die Schaltzeiten führt. Aber auch solche erfindungsgemäßen Flüssigkristallanzeigen, die erfindungsgemäße Flüssigkristallmischungen mit kleineren Werten für $\Delta n$ enthalten, sind durch vorteilhafte Werte für die Schaltzeiten gekennzeichnet.
Die erfindungsgemäßen Flüssigkristallmischungen sind weiter durch vorteilhafte Werte für die Steilheit der elektrooptischen Kennlinie gekennzeichnet, und können insbesondere bei Temperaturen über 20 °C mit hohen Multiplexraten betrieben werden. Darüber hinaus weisen die erfindungsgemäßen Flüssigkristallmischungen eine hohe Stabilität und günstige Werte für den elektrischen Widerstand und die Frequenzabhängigkeit der Schwellenspannung auf. Die erfindungsgemäßen Flüssigkristallanzeigen weisen einen großen Arbeitstemperaturbereich und eine gute Winkelabhängigkeit des Kontrastes auf.
Der Aufbau der erfindungsgemäßen Flüssigkristall-Anzeigeelemente aus Polarisatoren, Elektrodengrundplatten und Elektroden mit einer solchen Oberflächenbehandlung, dass die Vorzugsorientierung (Direktor) der jeweils daran angrenzenden Flüssigkristall-Moleküle von der einen zur anderen Elektrode gewöhnlich um betragsmäßig 160° bis 720° gegeneinander verdreht ist, entspricht der für derartige Anzeigeelemente üblichen Bauweise. Dabei ist der Begriff der üblichen Bauweise hier weit gefasst und umfasst auch alle Abwandlungen und Modifikationen der TN- und STN-Zelle, insbesondere auch Matrix-Anzeigeelemente sowie die zusätzliche Magnete enthaltenden Anzeigeelemente.
Der Oberflächentiltwinkel an den beiden Trägerplatten kann gleich oder verschieden sein. Gleiche Tiltwinkel sind bevorzugt. Bevorzugte TN-Anzeigen weisen Anstellwinkel zwischen der Längsachse der Moleküle an der Oberfläche der Trägerplatten und den Trägerplatten von 0° bis 7°, vorzugsweise 0,01° bis 5°, insbesondere 0,1 bis 2° auf. In den STN-Anzeigen ist der Anstellwinkel bei 1° bis 30°, vorzugsweise bei 1 ° bis 12° und insbesondere bei 3° bis 10°.
Der Verdrillungswinkel der TN-Mischung in der Zelle liegt dem Betrag nach zwischen 22,5° und 170°, vorzugsweise zwischen 45° und 130° und insbesondere zwischen 80° und 115°. Der Verdrillungswinkel der STN-Mischung in der Zelle von Orientierungsschicht zu Orientierungschicht liegt dem Betrag nach zwischen 100° und 600°, vorzugsweise zwischen 170° und 300° und insbesondere zwischen 180° und 270°.
Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.
Die Dielektrika können auch weitere, dem Fachmann bekannte und in der Literatur beschriebene Zusätze enthalten. Beispielsweise können 0-15 % pleochroitische Farbstoffe, Stabilisatoren, wie z. B. Tinuvin® der Fa. Ciba, Antioxidantien, UV-Absorber, etc., zugesetzt werden.
In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen. Die Alkenylreste weisen die trans-Konfiguration auf. Die Codierung gemäß Tabelle B versteht sich von selbst. In Tabelle A ist nur das Acronym für den Grundkörper angegeben.
Im Einzelfall folgt getrennt vom Acronym für den Grundkörper mit einem Strich der in der untenstehenden Tabelle angegebene Code für die Substituenten $R^{1*}$, $R^{2*}$, $L^{1*}$, $L^{2*}$ und $L^{3*}$.

Die Displays, insbesondere TN-, STN- und IPS-Displays, enthalten vorzugsweise flüssigkristalline Mischungen, die sich aus ein oder mehreren Verbindungen aus den Tabellen A und B zusammensetzen.

| Code für $R^{1*}$, $R^{2*}$, $L^{1*}$,$L^{2*}$,$L^{3*}$ | $R^{1*}$ | $R^{2*}$ | $L^{1*}$ | $L^{2*}$ | $L^{3*}$ |
|---|---|---|---|---|---|
| nm | $C_nH_{2n+1}$ | $C_mH_{2m+1}$ | H | H | H |
| nOm | $OC_nH_{2n+1}$ | $C_mH_{2m+1}$ | H | H | H |
| nO.m | $C_nH_{2n+1}$ | $OC_mH_{2m+1}$ | H | H | H |
| n | $C_nH_{2n+1}$ | CN | H | H | H |
| nN.F | $C_nH_{2n+1}$ | CN | H | H | F |
| nN.F.F | $C_nH_{2n+1}$ | CN | H | F | F |
| nF | $C_nH_{2n+1}$ | F | H | H | H |
| nOF | $OC_nH_{2n+1}$ | F | H | H | H |
| nF.F | $C_nH_{2n+1}$ | F | H | H | F |
| nmF | $C_nH_{2n+1}$ | $C_mH_{2m+1}$ | F | H | H |
| $nOCF_3$ | $C_nH_{2n+1}$ | $OCF_3$ | H | H | H |
| n-Vm | $C_nH_{2n+1}$ | $-CH=CH-C_mH_{2m+1}$ | H | H | H |
| nV-Vm | $C_nH_{2n+1}-CH=CH-$ | $-CH=CH-C_mH_{2m+1}$ | H | H | H |
| n.F.F.F | $C_nH_{2n+1}$ | F | H | F | F |

**Tabelle A:** ($L^{1*}$, $L^{2*}$, $L^{3*}$ = H oder F)

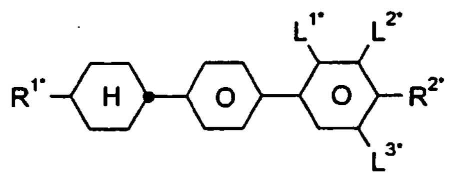

**BCH**

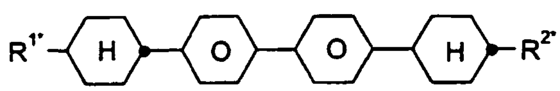

**CBC**

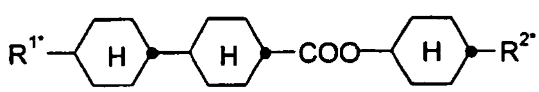

**CH**

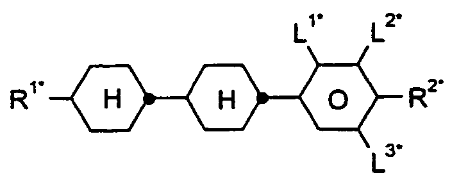

**CCP**

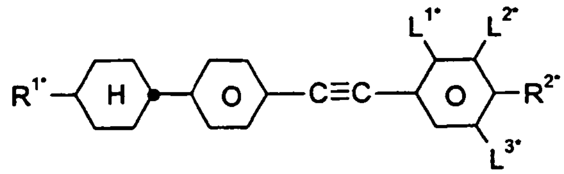

CPTP

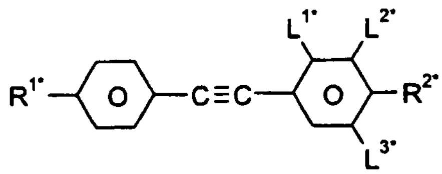

PTP

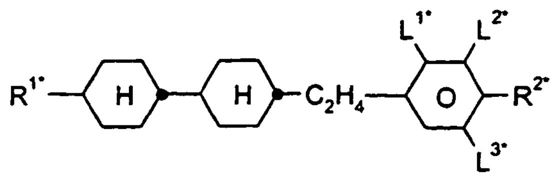

ECCP

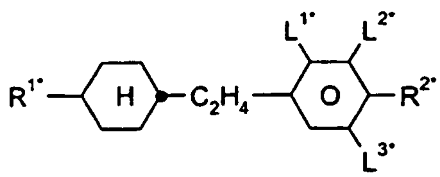

EPCH

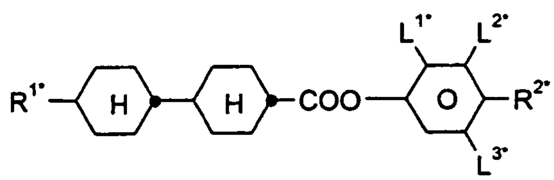

CP

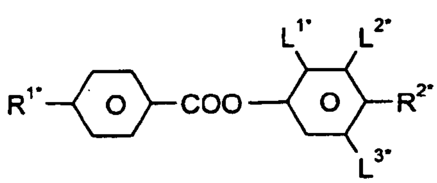

ME

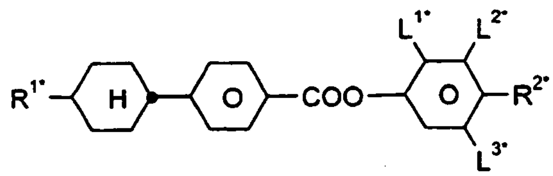

**HP**

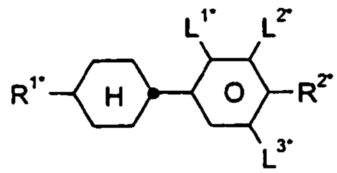

**PCH**

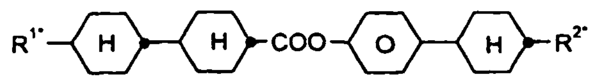

**CCPC**

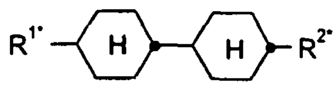

**CCH**

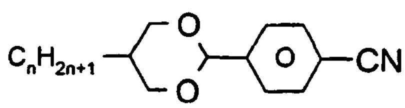

**PDX-n**

Tabelle B:

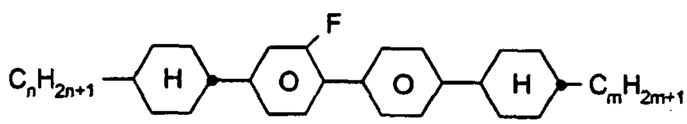

**CBC-nmF**

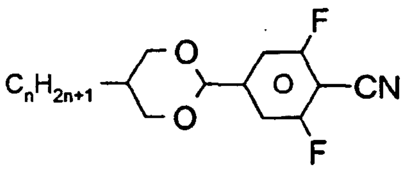

DU-n-N

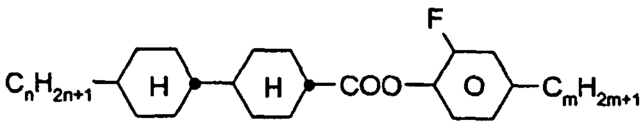

CP-nmF

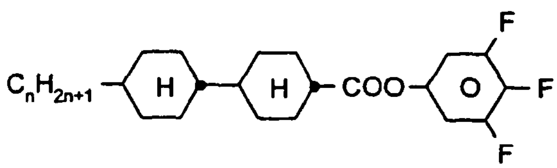

CCZU-n-F

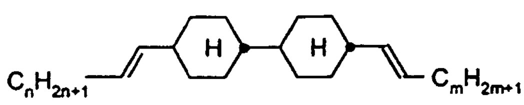

CC-nV-Vm

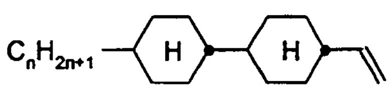

CC-n-V

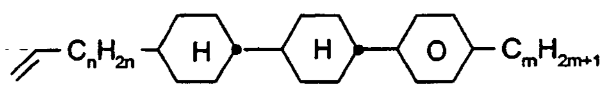

CCP-Vn-m

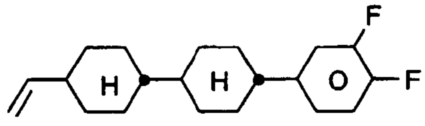

CCG-V-F

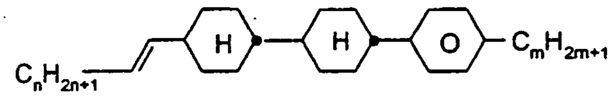

**CCP-nV-m**

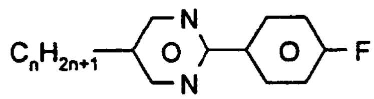

**PYP-nF**

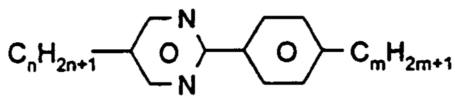

**PYP-nm**

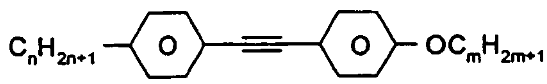

**PTP-nOm**

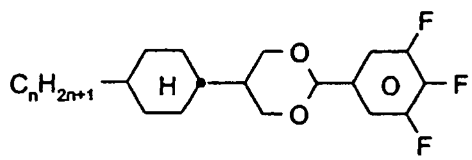

**CDU-n-F**

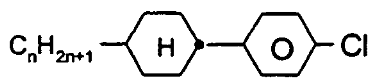

**PCH-nCl**

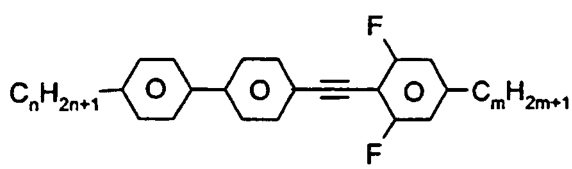

**PPTUI-n-m**

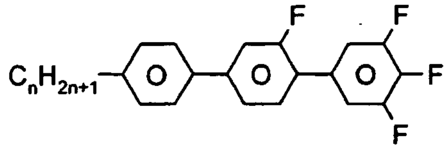

**PGU-n-F**

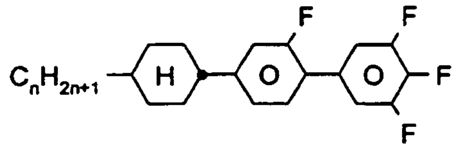

CGU-n-F

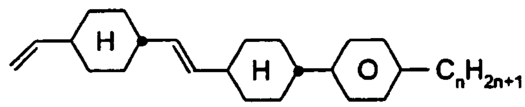

CVCP-V-n

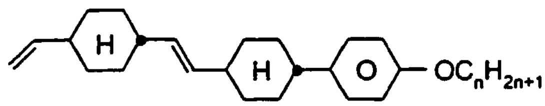

CVCP-V-On

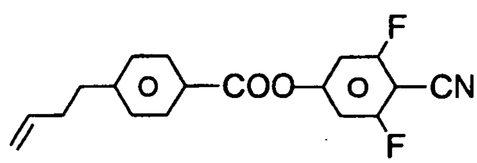

PZU-V2-N

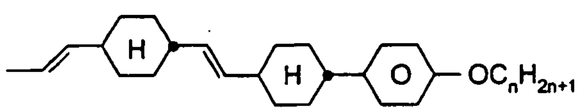

CVCP-1V-On

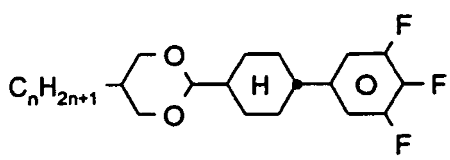

DCU-n-F

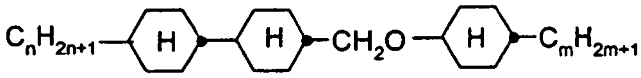

CCOC-n-m

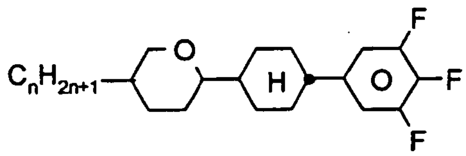

ACU-n-F

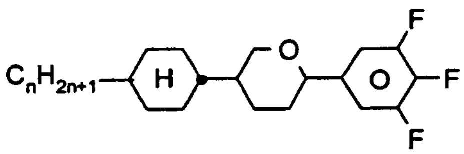

CAU-n-F

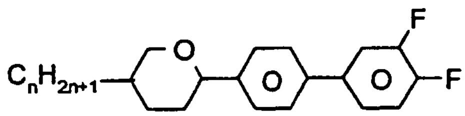

APG-n-F

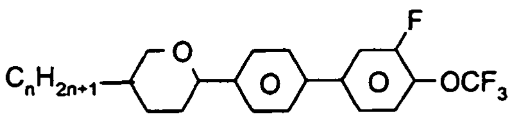

APG-n-OT

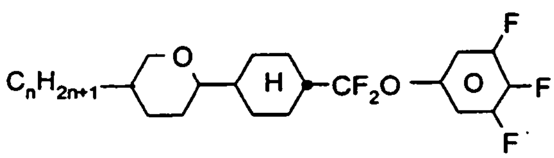

ACQU-n-F

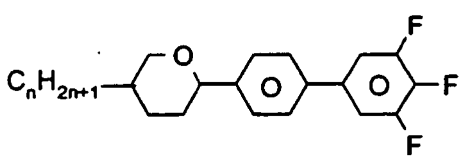

APU-n-F

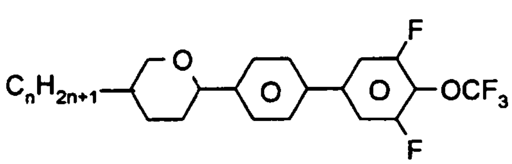

APU-n-OT

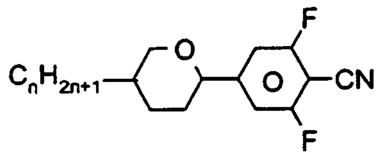

**AU-n-N**

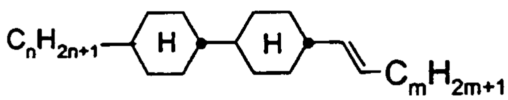

**CC-n-Vm**

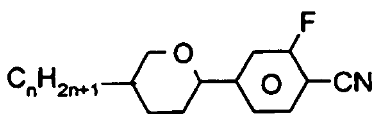

**AG-n-N**

**Tabelle C**

In der Tabelle C werden Dotierstoffe genannt, die den erfindungsgemäßen Mischungen zugesetzt werden können. Vorzugsweise enthalten die Mischungen 0,01 bis 10 Gew.% an Dotierstoff.

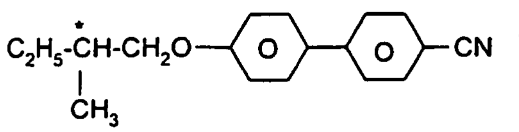

**C 15**

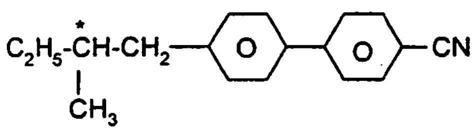

**CB 15**

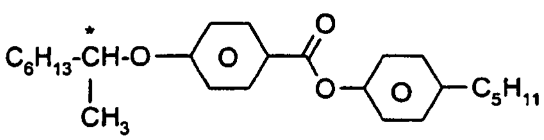

**CM 21**

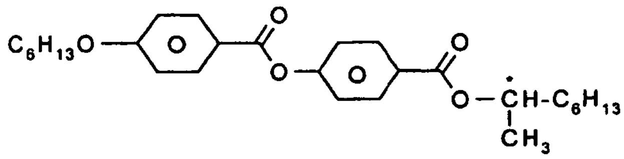

**R/S-811**

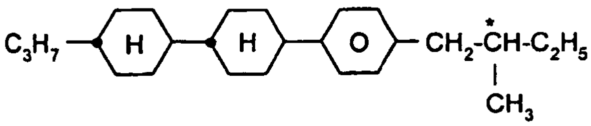

**CM 44**

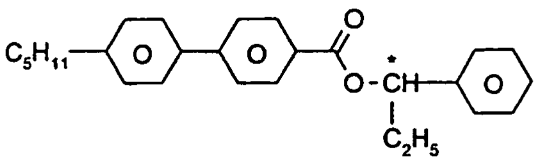

**CM 45**

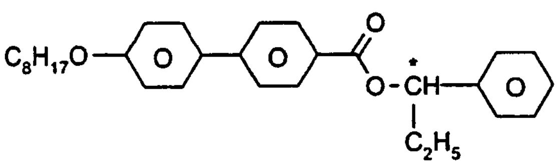

**CM 47**

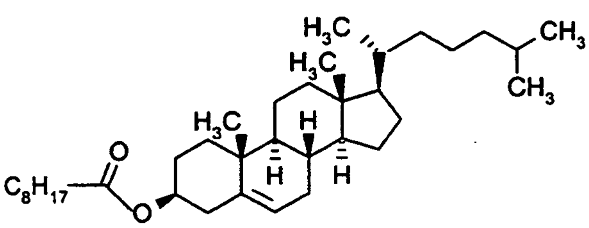

**CN**

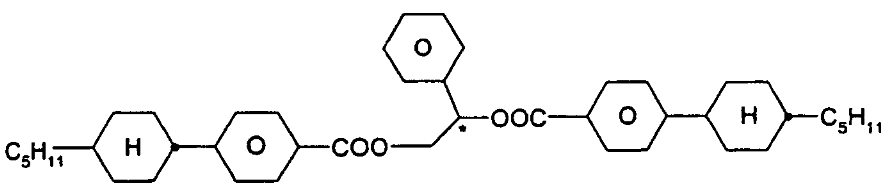

**R/S-1011**

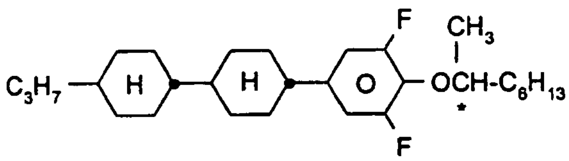

**R/S-2011**

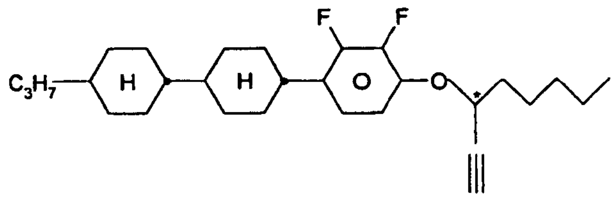

**R/S-3011**

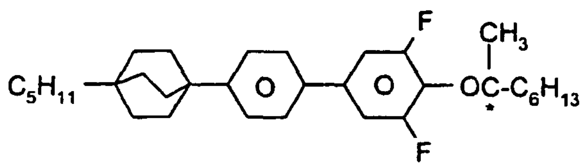

**R/S-4011**

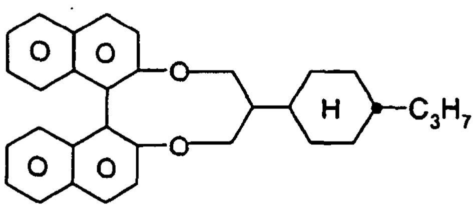

**R/S-5011**

**Tabelle D**

Stabilisatoren, die beispielsweise den erfindungsgemäßen Mischungen zugesetzt werden können, werden nachfolgend genannt.

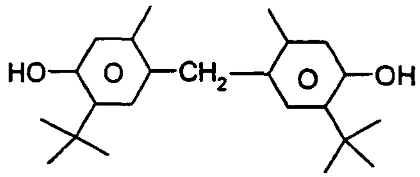

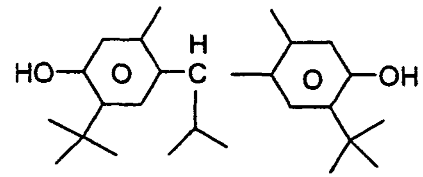

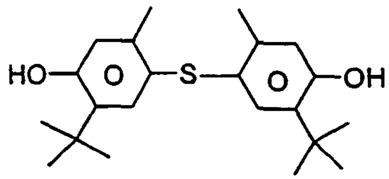

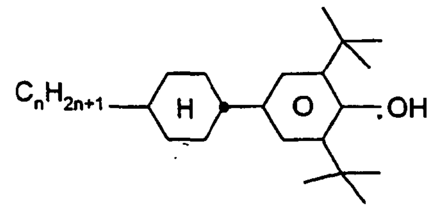

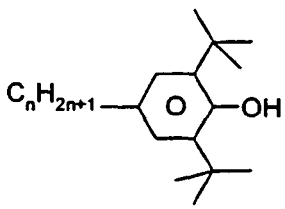

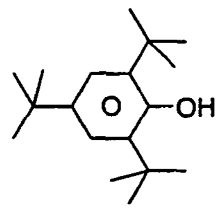

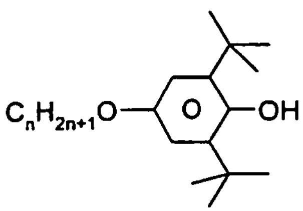

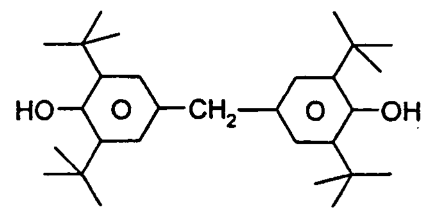

(fortgesetzt)

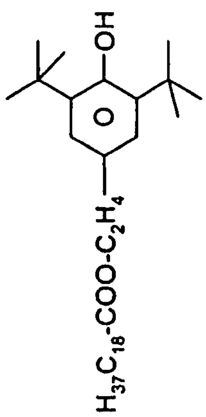

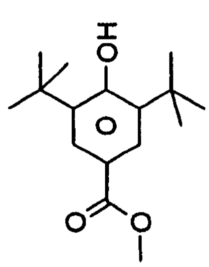

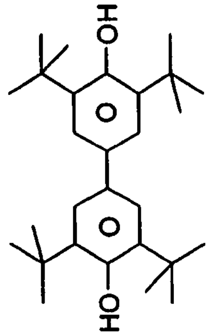

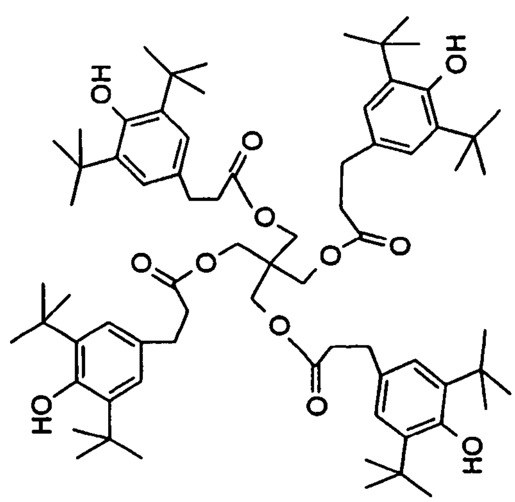

(fortgesetzt)

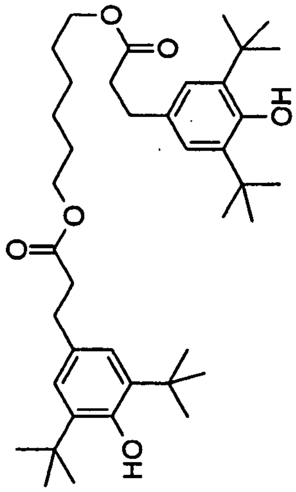

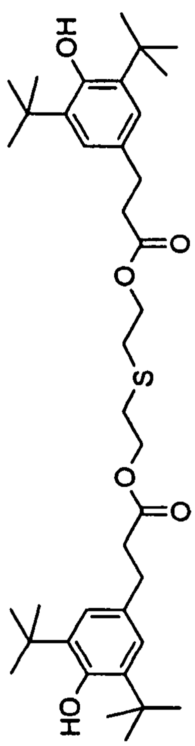

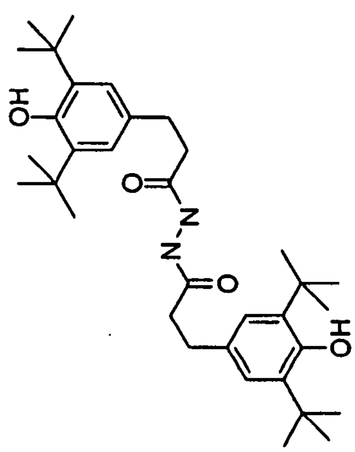

(fortgesetzt)

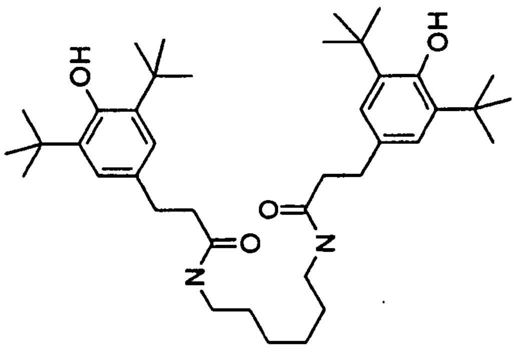

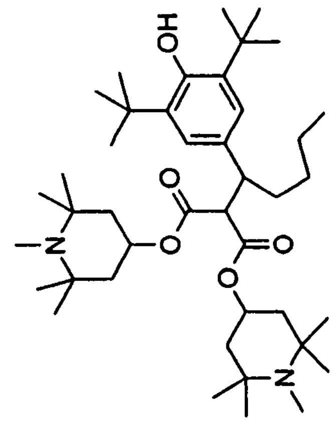

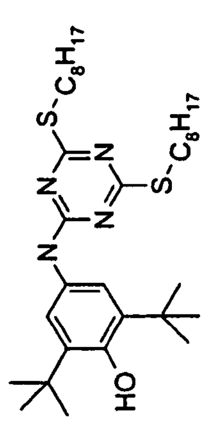

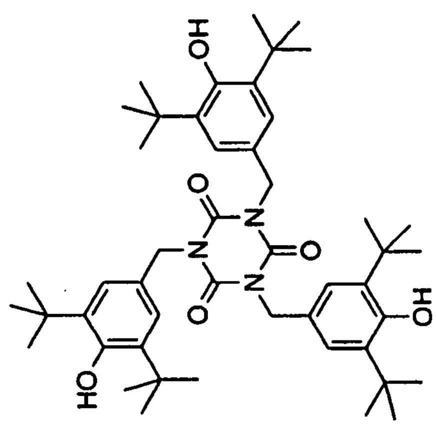

(fortgesetzt)

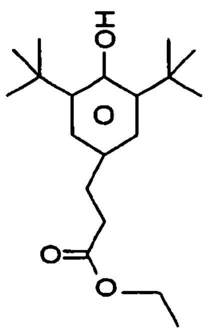

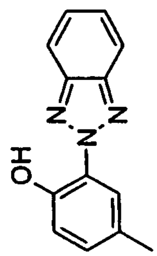

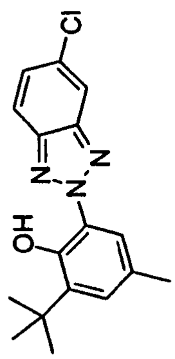

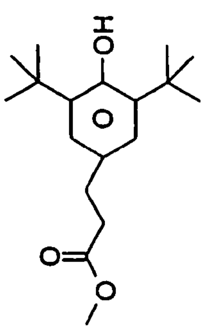

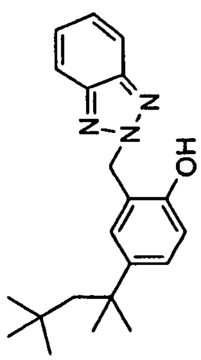

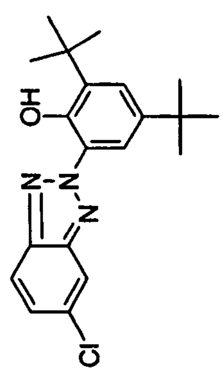

(fortgesetzt)

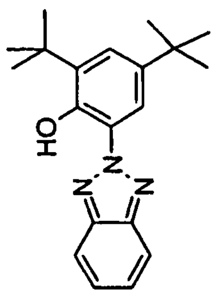

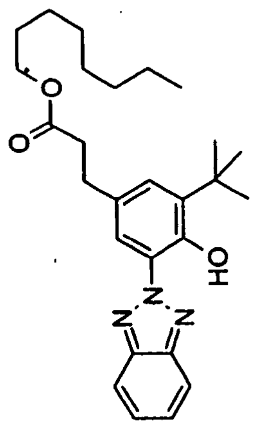

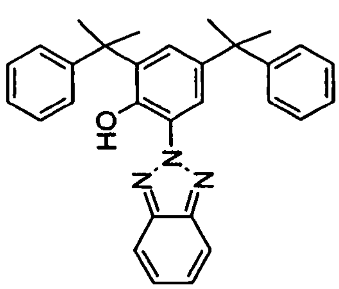

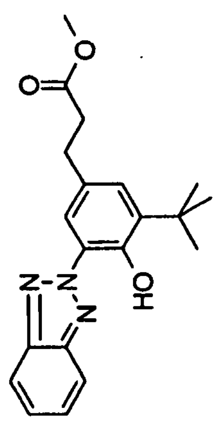

(fortgesetzt)

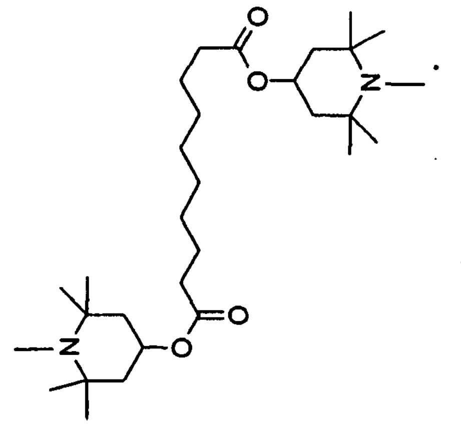

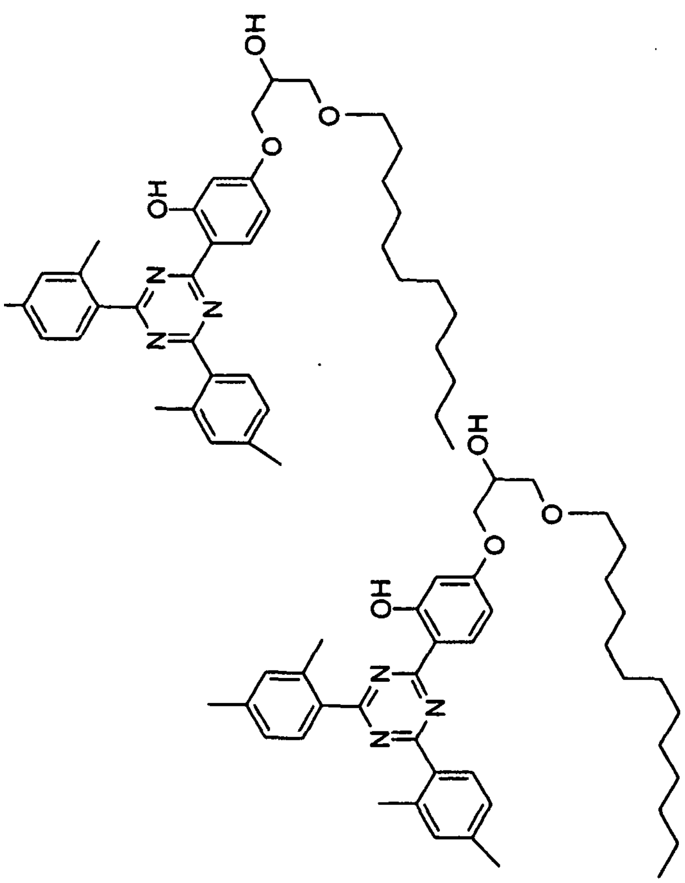

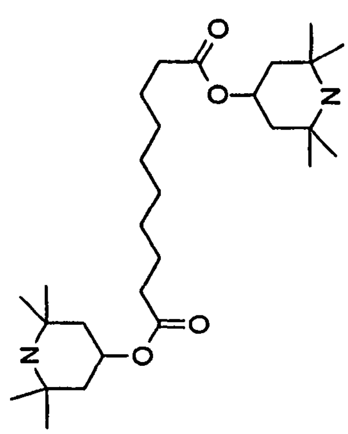

Die folgenden Beispiele sollen die Erfindung erläutern, ohne sie zu begrenzen. Es bedeuten

| | |
|---|---|
| Klp. | Klärpunkt (Phasenübergangstemperatur nematisch-isotrop) |
| S-N | Phasenübergangstemperatur smektisch-nematisch |
| $\nu_{20}$ | Fließviskosität ($mm^2/s$, soweit nicht anders angegeben, bei 20 ˚C) |
| $\Delta n$ | optische Anisotropie (589 nm, 20 ˚C) |
| $\Delta\varepsilon$ | dielektrische Anisotropie (1 kHz, 20 ˚C) |
| $\gamma_1$ | Rotationsviskosität (mPa · s bei 20 ˚C) |
| steep | Kennliniensteilheit = $(V_{90}/V_{10} - 1) \cdot 100$ [%] |
| $V_{10}$ | Schwellenspannung = charakteristische Spannung bei einem relativen Kontrast von 10 % |
| $V_{90}$ | …he Spannung bei einem relativen Kontrast von 90 % |
| $t_{ave}$ | $\frac{t_{on} + t_{off}}{2}$ (mittlere Schaltzeit) |
| $t_{on}$ | Zeit vom Einschalten bis zur Erreichung von 90 % des maximalen Kontrastes |
| $t_{off}$ | Zeit vom Ausschalten bis zur Erreichung von 10 % des maximalen Kontrastes |
| Mux | Multiplexrate |
| $t_{store}$ | Tieftemperatur-Lagerstabilität in Stunden (- 20 ˚C, - 30 ˚C, - 40 ˚C) |

Vor- und nachstehend sind alle Temperaturen in ˚C angegeben. Die Prozentzahlen sind Gewichtsprozente. Alle Werte beziehen sich auf 20 ˚C, soweit nicht anders angegeben. Die Ansteuerung der Anzeigen erfolgt, soweit nicht anders angegeben, nicht multiplexiert.

Beispiel 1

| | | | |
|---|---|---|---|
| ME2N.F | 6,00 % | Klärpunkt [˚C]: | 95,0 |
| ME3N.F | 7,00 % | $\Delta n$ [589 nm, 20 ˚C]: | 0,1419 |
| ME4N.F | 11,00 % | $\Delta\varepsilon$ [1kHz, 20 ˚C]: | 28,3 |
| ME5N.F | 10,00 % | $\gamma_1$ [20 ˚C; mPa.s]: | 334 |
| CGU-2-F | 8,00 % | Verdrillung [˚]: | 240 |
| CGU-3-F | 8,00 % | | |
| CCZU-2-F | 4,00 % | | |
| CCZU-3-F | 10,50 % | | |
| CCZU-5-F | 4,00 % | | |
| CCPC-33 | 2,00 % | | |
| CCPC-34 | 2,00 % | | |
| CBC-33F | 5,00 % | | |
| CBC-53F | 5,00 % | | |
| CBC-55F | 5,00 % | | |
| CPTP-301 | 3,50 % | | |
| APU-3-F | 9,00 % | | |

Beispiel 2

| | | | |
|---|---|---|---|
| PCH-3N.F.F | 12,00 % | Klärpunkt [˚C]: | 106,0 |
| ME2N.F | 2,00 % | $\Delta n$ [589 nm, 20 ˚C]: | 0,1388 |
| ME3N.F | 3,00 % | $\Delta\varepsilon$ [1 kHz, 20 ˚C]: | 11,9 |
| CGU-2-F | 5,00 % | $\gamma_1$ [20 ˚C; mPa.s]: | 195 |
| CGU-3-F | 5,00 % | Verdrillung [˚]: | 240 |
| APU-3-F | 7,00 % | | |
| CCP-2F.F.F | 4,00 % | | |

(fortgesetzt)

| | |
|---|---|
| CAU-3-F | 4,00 % |
| CC-5-V | 2,00 % |
| CCP-V-1 | 9,00 % |
| CCP-V2-1 | 5,00 % |
| CCG-V-F | 8,00 % |
| CVCP-1V-01 | 4,00 % |
| PTP-102 | 4,00 % |
| PTP-201 | 4,00 % |
| PTP-301 | 4,00 % |
| PTP-302 | 3,00 % |
| CCPC-33 | 5,00 % |
| CCPC-34 | 5,00 % |
| CCPC-35 | 5,00 % |

Beispiel 3

| | | | |
|---|---|---|---|
| PCH-3N.F.F | 8,00 % | Klärpunkt [˚C]: | 79,0 |
| ME2N.F | 10,00 % | Δn [589 nm, 20 ˚C]: | 0,1314 |
| ME3N.F | 10,00 % | Δε [1 kHz, 20 ˚C]: | 45,0 |
| ME4N.F | 11,00 % | $\gamma_1$ [20 ˚C; mPa.s]: | 397 |
| ME5N.F | 10,00 % | Verdrillung [˚]: | 240 |
| HP-3N.F | 5,00 % | | |
| HP-4N.F | 5,00 % | | |
| HP-5N.F | 4,00 % | | |
| CCP-2F.F.F | 5,00 % | | |
| CCP-3F.F.F | 5,00 % | | |
| CCP-5F.F.F | 5,00 % | | |
| CCPC-33 | 4,00 % | | |
| CCPC-34 | 4,00 % | | |
| CCPC-35 | 4,00 % | | |
| ACU-3-F | 10,00 % | | |

Beispiel 4

| | | | |
|---|---|---|---|
| PCH-3N.F.F | 8,00 % | Klärpunkt [˚C]: | 79,0 |
| ME2N.F | 10,00 % | Δn [589 nm, 20 ˚C]: | 0,1312 |
| ME3N.F | 10,00 % | Δε [1kHz, 20 ˚C]: | 45,0 |
| ME4N.F | 11,00 % | $\gamma_1$ [20 ˚C; mPa.s]: | 385 |
| ME5N.F | 10,00 % | | |
| HP-3N.F | 5,00 % | | |
| HP-4N.F | 5,00 % | | |
| HP-5N.F | 4,00 % | | |
| CCP-2F.F.F | 5,00 % | | |
| CCP-3F.F.F | 6,00 % | | |
| CCP-5F.F.F | 5,00 % | | |
| CCPC-33 | 4,00 % | | |
| CCPC-34 | 3,00 % | | |

(fortgesetzt)

| | |
|---|---|
| CCPC-35 | 4,00 % |
| ACQU-3-F | 10,00 % |

Beispiel 5

| | | | |
|---|---|---|---|
| PCH-3N.F.F | 8,00 % | Klärpunkt [˚C]: | 79,5 |
| ME2N.F | 10,00 % | Δn [589 nm, 20 ˚C]: | 0,1326 |
| ME3N.F | 10,00 % | Δε [1kHz, 20 ˚C]: | 44,7 |
| ME4N.F | 11,00 % | $\gamma_1$[20 ˚C; mPa.s]: | 399 |
| ME5N.F | 10,00 % | | |
| HP-3N.F | 5,00 % | | . |
| HP-4N.F | 5,00 % | | |
| HP-5N.F | 4,00 % | | |
| CCP-2F.F.F | 5,00 % | | |
| CCP-3F.F.F | 6,00 % | | |
| CCP-5F.F.F | 5,00 % | | |
| CCPC-33 | 3,00 % | | |
| CCPC-34 | 4,00 % | | |
| CCPC-35 | 4,00 % | | |
| CAU-3-F | 10,00 % | | |

Beispiel 6

| | | | |
|---|---|---|---|
| PCH-3N.F.F | 8,00 % | Klärpunkt [˚C]: | 80,0 |
| ME2N.F | 10,00 % | Δn [589 nm, 20 ˚C]: | 0,1389 |
| ME3N.F | 10,00 % | Δε [1 kHz, 20 ˚C]: | 45,2 |
| ME4N.F | 11,00 % | $\gamma_1$ [20 ˚C; mPa.s]: | 403 |
| ME5N.F | 10,00 % | | |
| HP-3N.F | 5,00 % | | |
| HP-4N.F | 5,00 % | | |
| HP-5N-F | 4,00 % | | |
| CCP-2F.F.F | 5,00 % | | |
| CCP-3F.F.F | 5,00 % | | |
| CCP-5F.F.F | 5,00 % | | |
| CCPC-33 | 4,00 % | | |
| CCPC-34 | 4,00 % | | |
| CCPC-35 | 4,00 % | | |
| APU-3-F | 10,00 % | | |

Beispiel 7

| | | | |
|---|---|---|---|
| PCH-3N.F.F | 8,00 % | Klärpunkt [˚C]: | 79,0 |
| ME2N.F | 10,00 % | Δn [589 nm, 20 ˚C]: | 0,1382 |
| ME3N.F | 10,00 % | Δε [1 kHz, 20 ˚C]: | 46,0 |
| ME4N.F | 11,00 % | $\gamma_1$ [20 ˚C; mPa.s]: | 406 |

(fortgesetzt)

| | |
|---|---|
| ME5N.F | 10,00 % |
| HP-3N.F | 5,00 % |
| HP-4N.F | 5,00 % |
| HP-5N.F | 4,00 % |
| CCP-2F.F.F | 5,00 % |
| CCP-3F.F.F | 6,00 % |
| CCP-5F.F.F | 5,00 % |
| CCPC-33 | 3,00 % |
| CCPC-34 | 4,00 % |
| CCPC-35 | 4,00 % |
| APU-3-OT | 10,00 % |

Beispiel 8

| | | | |
|---|---|---|---|
| PCH-3N.F.F | 8,00 % | Klärpunkt [°C]: | 79,0 |
| ME2N.F | 10,00 % | Δn [589 nm, 20 °C]: | 0,1377 |
| ME3N.F | 10,00 % | Δε [1kHz, 20 °C]: | 45,1 |
| ME4N.F | 11,00 % | $\gamma_1$ [20 °C; mPa.s]: | 391 |
| ME5N.F | 10,00 % | | |
| HP-3N.F | 5,00 % | | |
| HP-4N.F | 5,00 % | | |
| HP-5N.F | 4,00 % | | |
| CCP-2F.F.F | 5,00 % | | |
| CCP-3F.F.F | 7,50 % | | |
| CCP-5F.F.F | 5,00 % | | |
| CCPC-33 | 3,00 % | | |
| CCPC-34 | 3,50 % | | |
| CCPC-35 | 3,00 % | | |
| APG-3-OT | 10,00 % | | |

Beispiel 9

| | | | |
|---|---|---|---|
| ME2N.F | 3,00 % | Klärpunkt [°C]: | 78,0 |
| PZU-V2-N | 5,00 % | Δn [589 nm, 20 °C]: | 0,1078 |
| AU-3-N | 7,00 % | Δε [1 kHz, 20 °C]: | 10,9 |
| PGU-2-F | 9,00 % | $\gamma_1$ [20 °C; mPa.s]: | 85 |
| PGU-3-F | 6,00 % | | |
| CCP-30$CF_3$ | 8,00 % | | |
| CCP-40$CF_3$ | 2,00 % | | |
| CC-3-V1 | 12,00 % | | |
| CC-5-V | 14,00 % | | |
| PCH-302 | 8,00 % | | |
| CCP-V-1 | 15,00 % | | |
| CCP-V2-1 | 11,00 % | | |

Beispiel 10

| | | | |
|---|---|---|---|
| ME2N.F | 3,00 % | Klärpunkt [˚C]: | 78,0 |
| PZU-V2-N | 5,00 % | Δn [589 nm, 20 ˚C]: | 0,1075 |
| AG-3-N | 9,00 % | Δε [1kHz, 20 ˚C]: | 10,8 |
| PGU-2-F | 9,50 % | $\gamma_1$ [20 ˚C; mPa.s]: | 87 |
| PGU-3-F | 5,00 % | | |
| CCP-30CF$_3$ | 8,00 % | | |
| CCP-40CF$_3$ | 4,00 % | | |
| CC-3-V1 | 12,00 % | | |
| CC-5-V | 15,00 % | | |
| PCH-302 | 5,50 % | | |
| CCP-V-1 | 15,00 % | | |
| CCP-V2-1 | 9,00 % | | |

Beispiel 11

| | | | |
|---|---|---|---|
| CCP-20CF$_3$ | 7,00 % | Klärpunkt [˚C]: | 75,0 |
| CCP-30CF$_3$ | 7,00 % | Δn [589 nm, 20 ˚C]: | 0,0750 |
| PDX-3 | 6,00 % | Δε [1 kHz, 20 ˚C]: | 9,2 |
| PDX-4 | 4,00 % | $\gamma_1$ [20 ˚C; mPa.s]: | 95 |
| ME2N.F | 2,50 % | | |
| CCZU-2-F | 7,00 % | | |
| CCZU-3-F | 15,00 % | | |
| CH-33 | 1,50 % | | |
| CH-35 | 2,00 % | | . |
| CH-43 | 2,00 % | | |
| CC-5-V | 18,00 % | | |
| CC-3-V1 | 11,00 % | | |
| PCH-302 | 2,50 % | | |
| ACU-2-F | 7,00 % | | |
| ACU-3-F | 7,50 % | | |

Beispiel 12

| | | | |
|---|---|---|---|
| ACU-2-F | 10,00 % | Klärpunkt [˚C]: | 73,5 |
| ACQU-3-F | 11,00 % | Δn [589 nm, 20 ˚C]: | 0,0782 |
| CCP-20CF$_3$ | 7,00 % | Δε [1 kHz, 20 ˚C]: | 18,4 |
| CCP-30CF$_3$ | 8,00 % | $\gamma_1$ [20 ˚C; mPa.s]: | 137 |
| CP-30CF$_3$ | 7,00 % | | |
| CP-50CF$_3$ | 7,00 % | | |
| DU-3-N | 12,50 % | | |
| ME2N.F | 3,00 % | | |
| ME3N.F | 2,00 % | | |
| CCZU-2-F | 4,00 % | | |
| CCZU-3-F | 15,00 % | | |
| CCZU-4-F | 6,00 % | | |

(fortgesetzt)

| | |
|---|---|
| CC-3-V1 | 2,50 % |
| CCH-35 | 5,00 % |

Beispiel 13

| | | | |
|---|---|---|---|
| ACU-2-F | 9,00 % | Klärpunkt [˚C]: | 70,0 |
| ACU-3-F | 9,00 % | $\Delta n$ [589 nm, 20 ˚C]: | 0,0754 |
| ACQU-3-F | 9,00 % | $\Delta\varepsilon$ [1kHz, 20 ˚C]: | 10,1 |
| CCP-20CF$_3$ | 3,00 % | $\gamma_1$ [20 ˚C; mPa.s]: | 83 |
| CCP-30CF$_3$ | 8,00 % | | |
| CP-30CF$_3$ | 7,00 % | | |
| CP-50CF$_3$ | 6,00 % | | |
| PCH-3N.F.F | 10,00 % | | |
| ME2N.F | 4,00 % | | |
| ME3N.F | 2,00 % | | |
| CCZU-2-F | 4,00 % | | |
| CCZU-3-F | 15,00 % | | |
| CCZU-5-F | 3,00 % | | |
| CC-5-V | 11,00 % | | |

**Patentansprüche**

**1.** Flüssigkristallines Medium, **dadurch gekennzeichnet, dass** es eine oder mehrere Verbindungen der Formel A

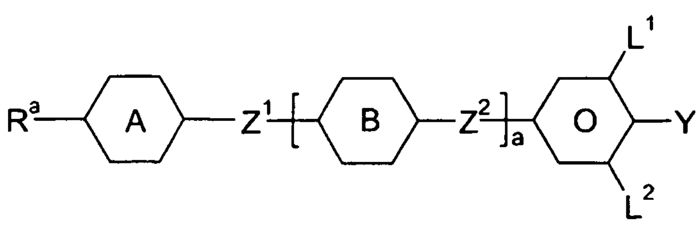

A

und mindestens eine Verbindung der Formel B

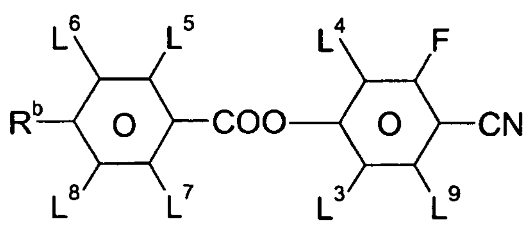

B

enthält,
worin

$R^a$ und $R^b$ jeweils unabhängig voneinander H, einen unsubstituierten, einen einfach durch CN oder $CF_3$ oder einen mindestens einfach durch Halogen substituierten Alkylrest mit 1 bis 12 C-Atomen, wobei in diesen Resten

auch eine oder mehrere $CH_2$-Gruppen jeweils unabhängig voneinander durch -O-, -S-,

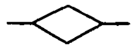 ,

-CH=CH-, -C≡C-, -CO-, -CO-O-, -O-CO- oder -O-CO-O- so ersetzt sein können, dass O-Atome nicht direkt miteinander verknüpft sind,

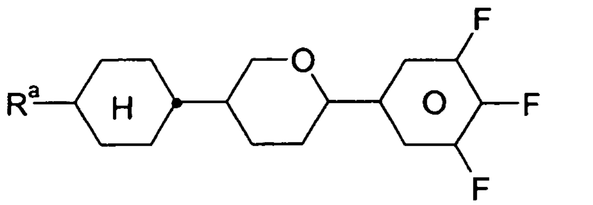

A-2

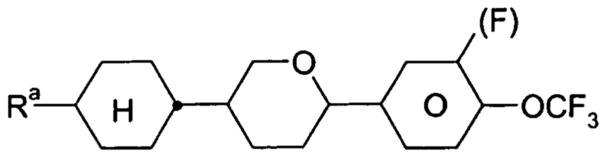

A-3

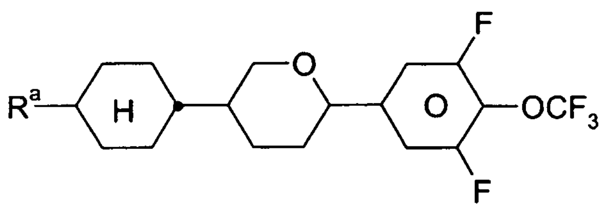

A-4

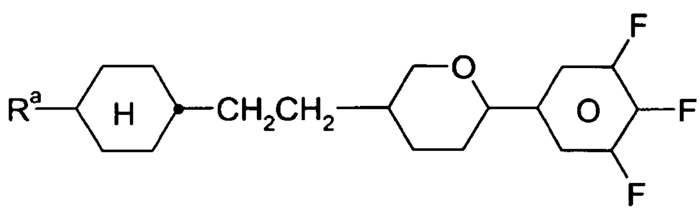

A-5

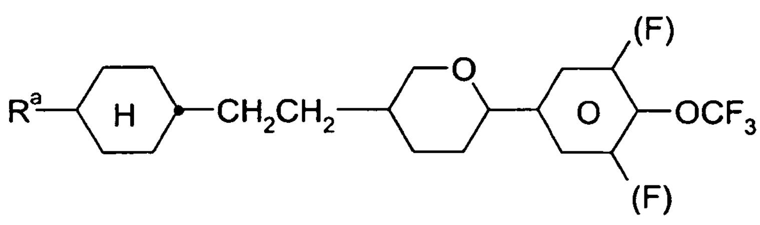

A-6

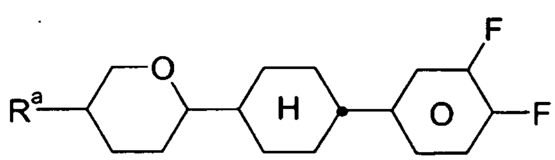

A-7

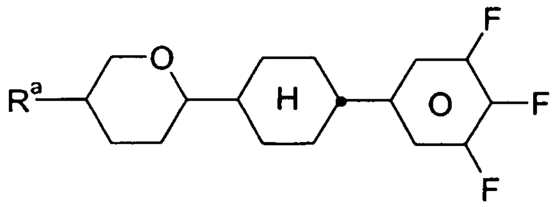

A-8

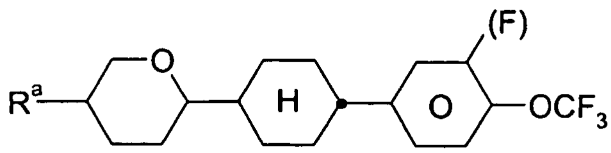

A-9

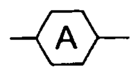 und 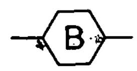

jeweils unabhängig voneinander

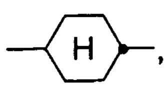, 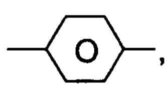, 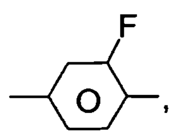, 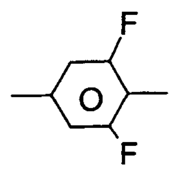

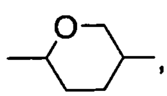, 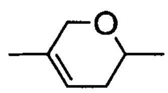 oder 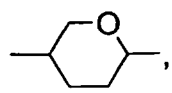,

mindestens ein Ring bedeutet

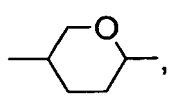,

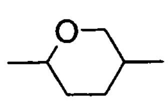 oder 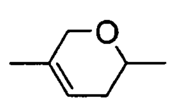,

$Z^1$ und $Z^2$ jeweils unabhängig voneinander $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $(CH_2)_3-$ oder eine Einfachbindung,
a 0 oder 1,
$L^1$ bis $L^9$ jeweils unabhängig voneinander H oder F, und
Y F, Cl, $SF_5$, CN, NCS, OCN, SCN oder ein einfach oder mehrfach halogenierter Alkyl-, Alkoxy-, Alkenyl- oder Alkenyloxyrest mit jeweils 1 bis zu 5 C-Atomen,

bedeuten.

**2.** Flüssigkristallines Medium nach Anspruch 1, **dadurch gekennzeichnet, dass** es mindestens eine Verbindung der Formeln A-1 bis A-56

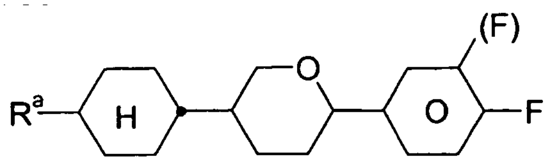
A-1

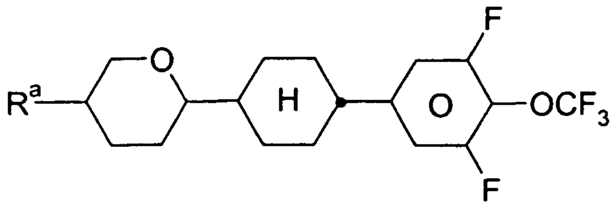
A-10

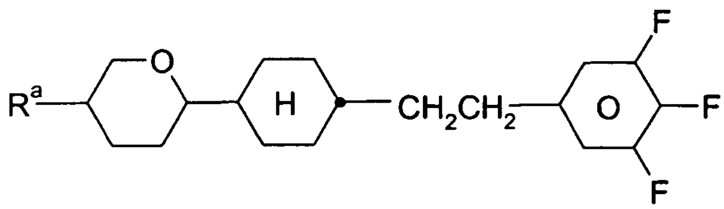
A-11

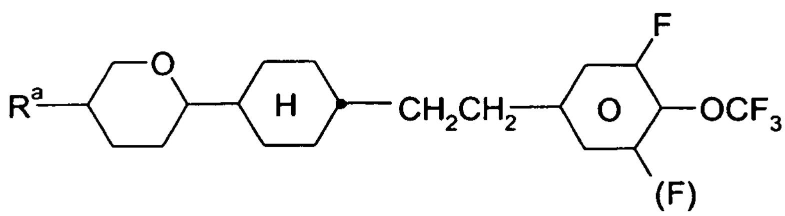
A-12

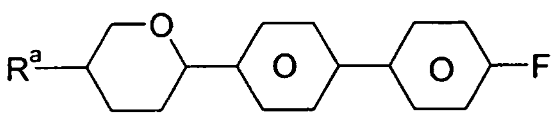
A-13

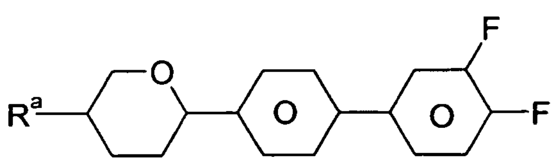
A-14

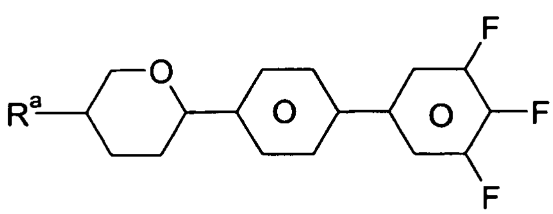
A-15

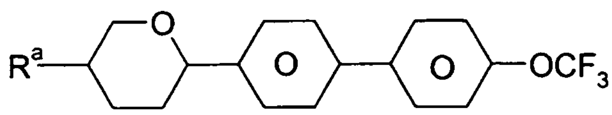 A-16

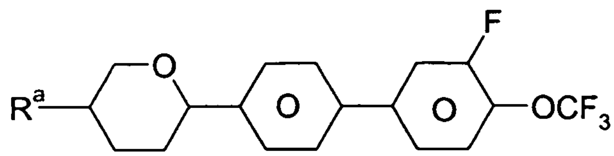 A-17

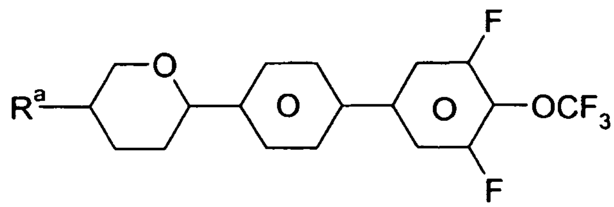 A-18

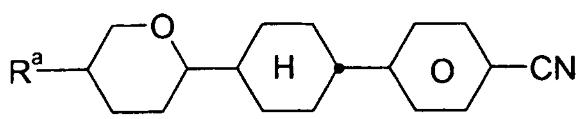 A-19

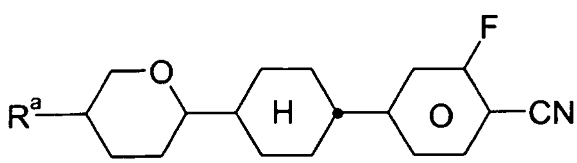 A-20

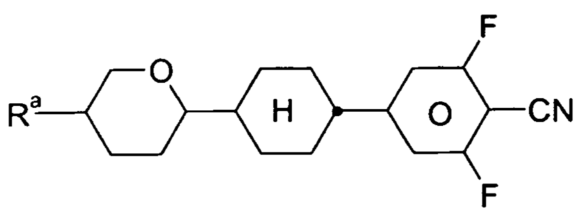 A-21

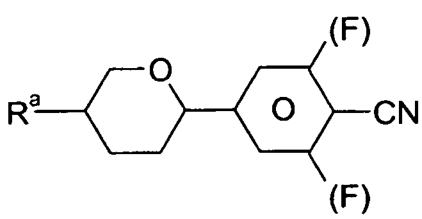 A-22

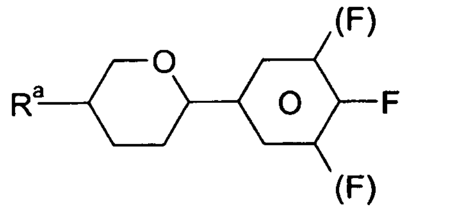

A-23

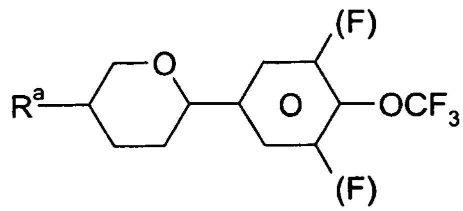

A-24

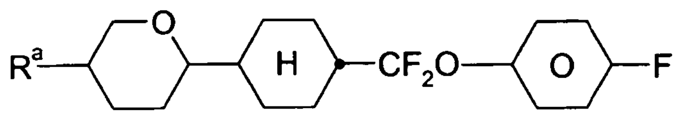

A-25

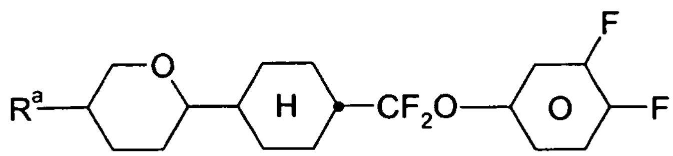

A-26

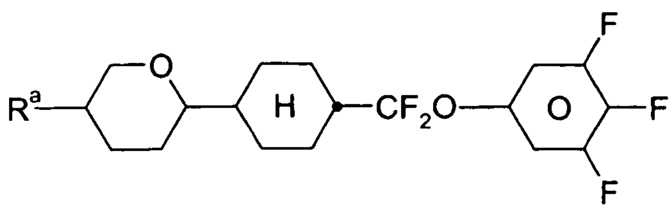

A-27

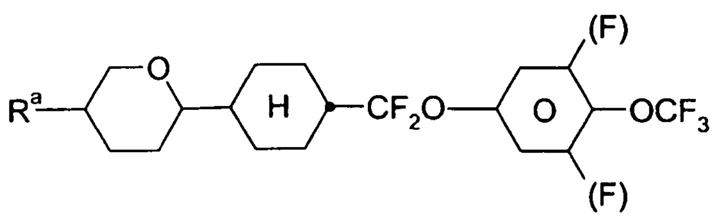

A-28

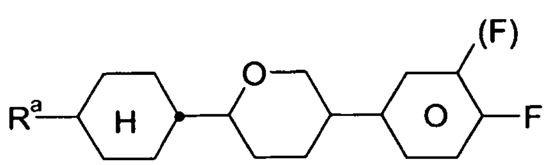

A-29

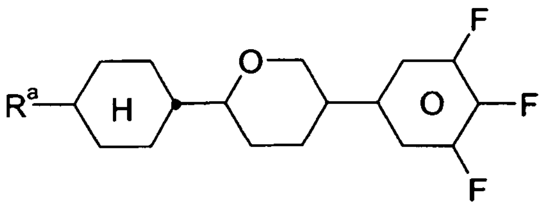

A-30

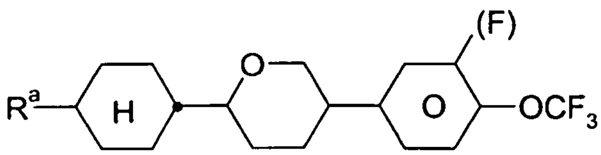

A-31

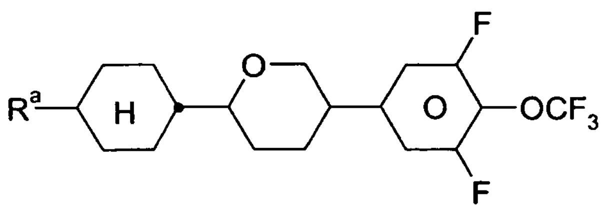

A-32

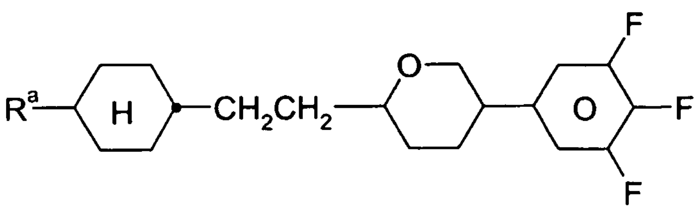

A-33

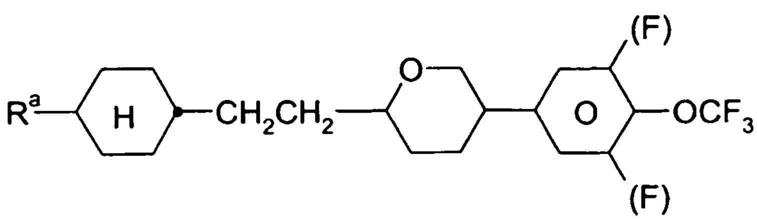

A-34

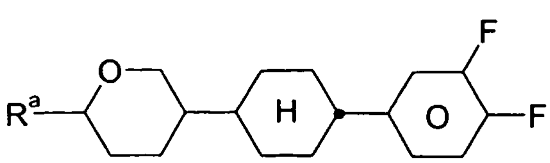

A-35

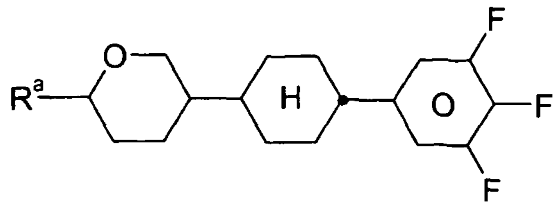

A-36

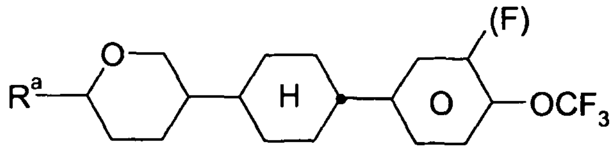

A-37

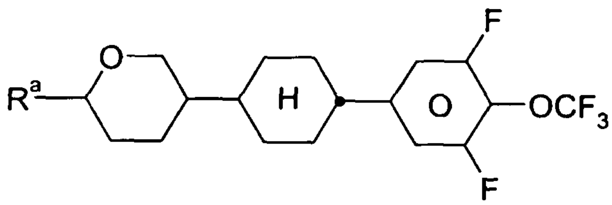

A-38

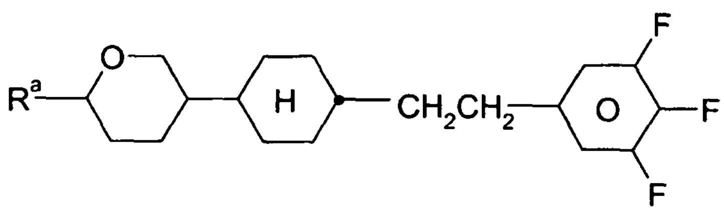

A-39

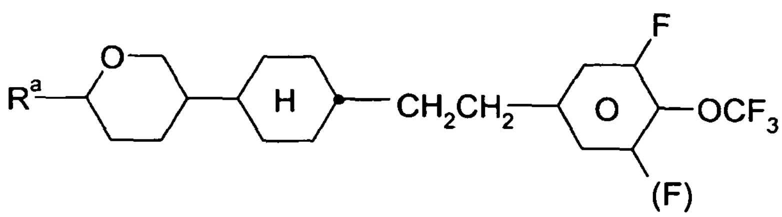

A-40

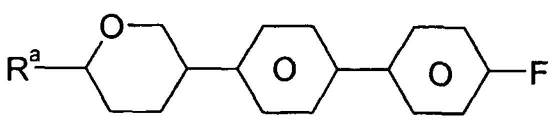

A-41

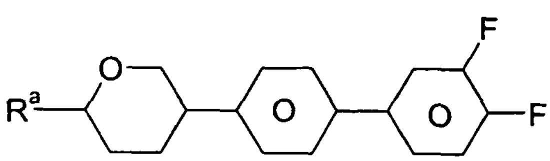

A-42

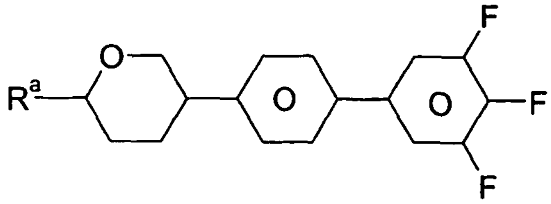

A-43

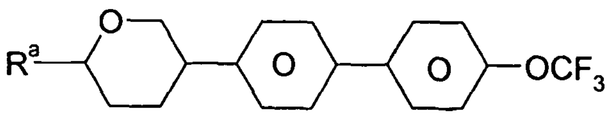

A-44

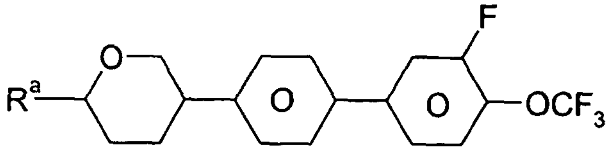

A-45

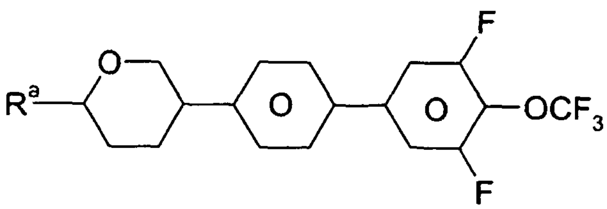

A-46

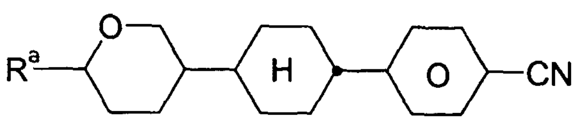

A-47

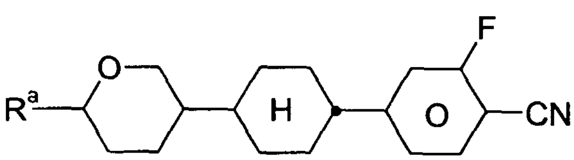

A-48

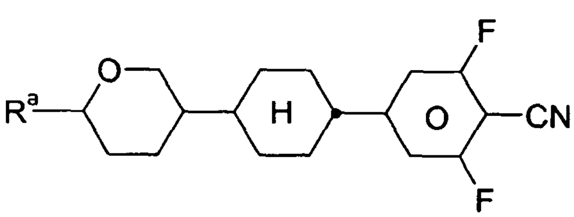

A-49

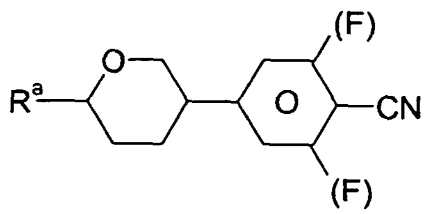
A-50

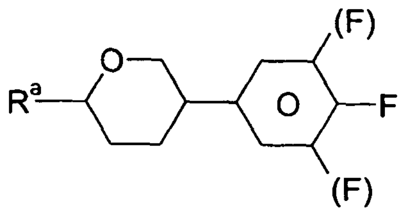
A-51

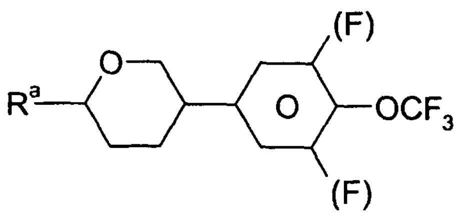
A-52

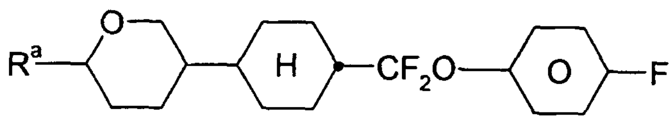
A-53

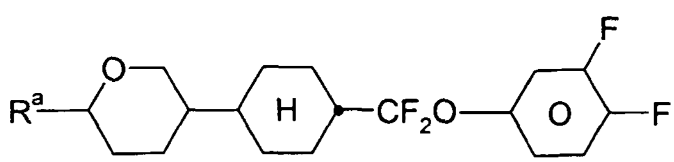
A-54

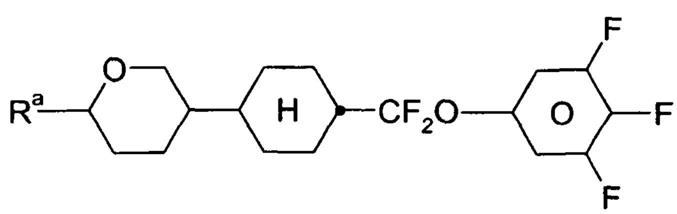
A-55

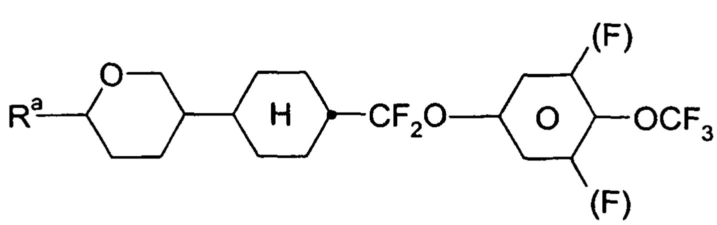
A-56

worin $R^a$ die in Anspruch 1 angegebenen Bedeutungen besitzt, enthält.

3. Flüssigkristallines Medium nach Anspruch 1 oder 2, **dadurch gekennzeichnet, dass** es mindestens eine Verbindung der Formeln B-1 bis B-6,

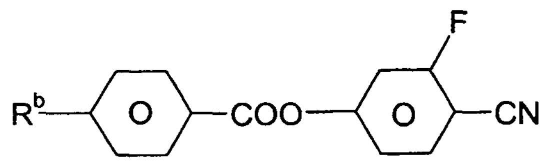

B-1

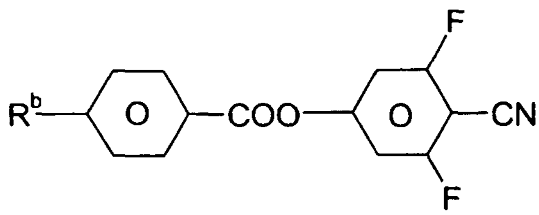

B-2

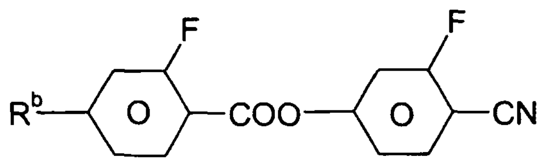

B-3

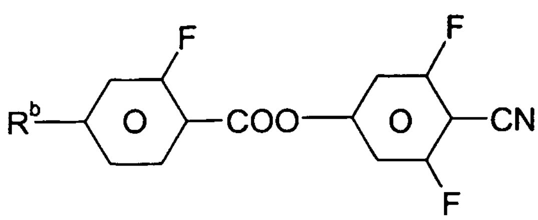

B-4

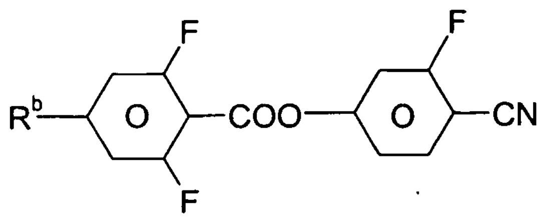

B-5

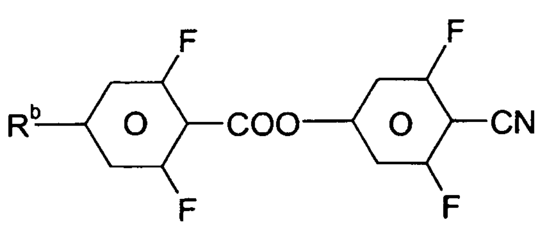

B-6

worin

$R^b$ die in Anspruch 1 angegebenen Bedeutungen besitzt, enthält.

4. Flüssigkristallines Medium nach einem der Ansprüche 1 bis 3, **dadurch gekennzeichnet, dass** es zusätzlich mindestens eine Verbindung der Formeln IIa bis IIk,

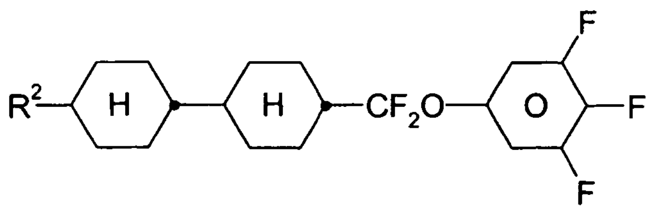

IIa

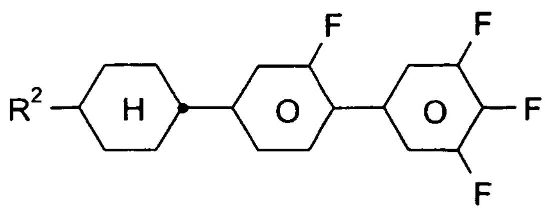

IIb

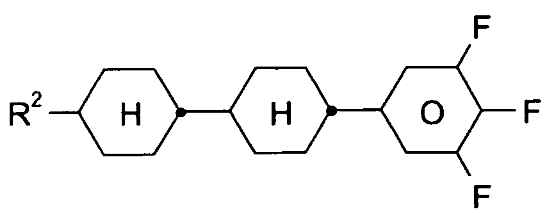

IIc

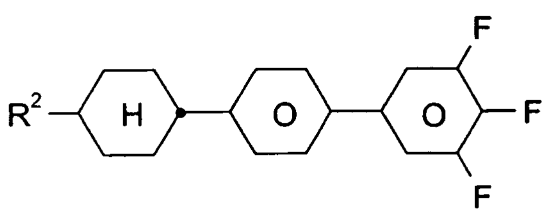

IId

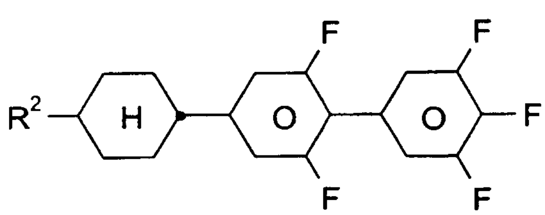

IIe

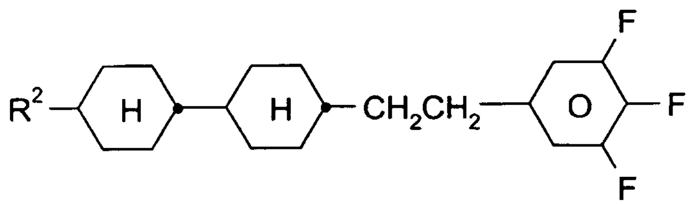

IIf

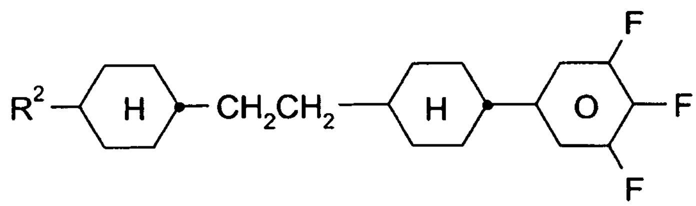

IIg

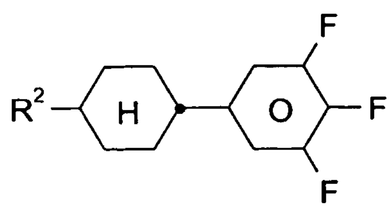

IIh

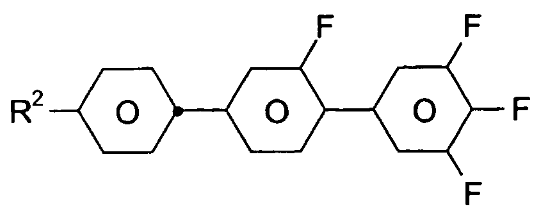

IIi

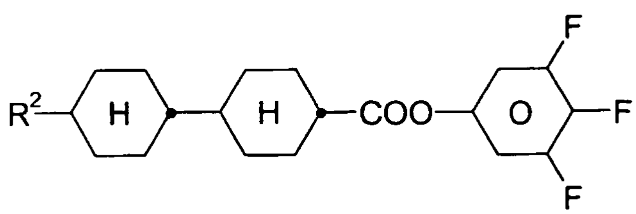

IIj

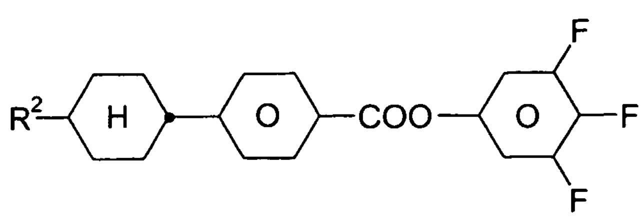

IIk

worin

$R^2$ einen unsubstituierten, einen einfach durch CN oder $CF_3$ oder einen mindestens einfach durch Halogen

substituierten Alkylrest mit 1 bis 12 C-Atomen, wobei in diesen Resten auch eine oder mehrere $CH_2$-Gruppen jeweils unabhängig voneinander durch -O-, -S-,

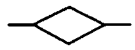

,

-CH=CH-, -C≡C-, -CO-, -CO-O-, -O-CO- oder -O-CO-O- so ersetzt sein können, dass O-Atome nicht direkt miteinander verknüpft sind,

bedeutet,
enthält.

**5.** Flüssigkristallines Medium nach einem der Ansprüche 1 bis 4, **dadurch gekennzeichnet, dass** es zusätzlich eine oder mehrere Cyanoverbindungen der Formeln IIIa bis IIIj,

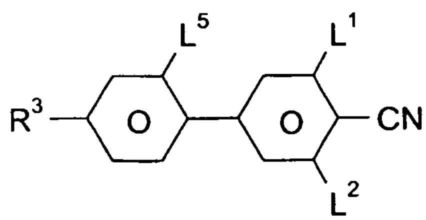

IIIa

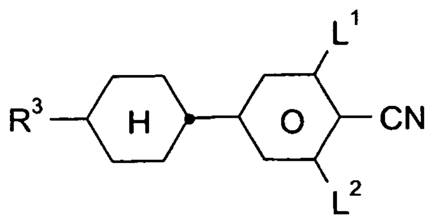

IIIb

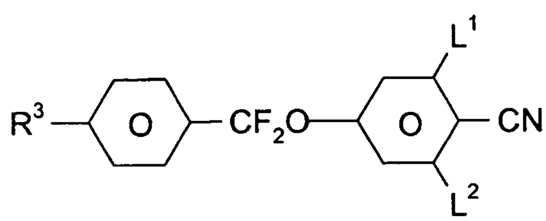

IIIc

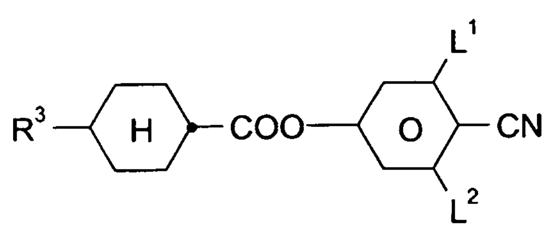

IIId

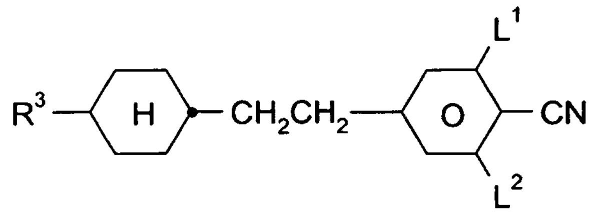

IIIe

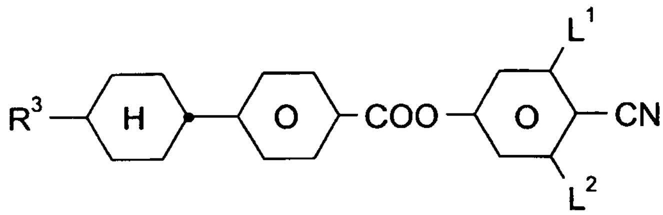

IIIf

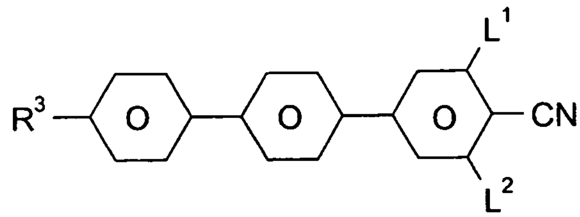

IIIg

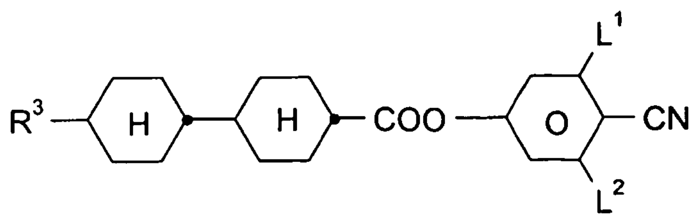

IIIh

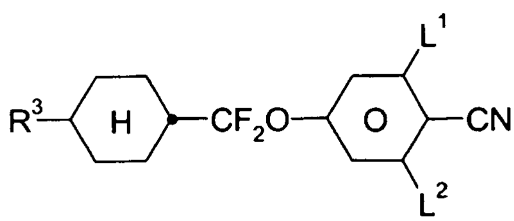

IIIi

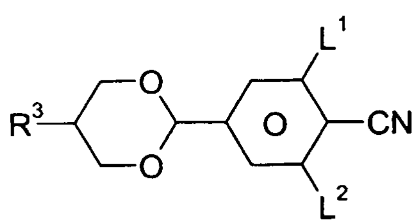

IIIj

worin

$R^3$ einen unsubstituierten, einen einfach durch CN oder $CF_3$ oder einen mindestens einfach durch Halogen substituierten Alkylrest mit 1 bis 12 C-Atomen, wobei in diesen Resten auch eine oder mehrere $CH_2$-Gruppen jeweils unabhängig voneinander durch -O-, -S-,

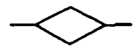
,

-CH=CH-, -C≡C-, -CO-, -CO-O-, -O-CO- oder -O-CO-O- so ersetzt sein können, dass O-Atome nicht direkt miteinander verknüpft sind, und
$L^1$, $L^2$ und $L^5$ jeweils unabhängig voneinander H oder F

bedeuten,
enthält.

**6.** Flüssigkristallines Medium nach einem der Ansprüche 1 bis 5, **dadurch gekennzeichnet, dass** es zusätzlich eine oder mehrere Verbindungen der Formel IV,

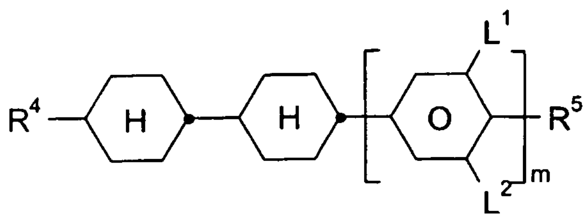
IV

worin

m 0 oder 1,
$R^4$ eine Alkenylgruppe mit 2 bis 7 C-Atomen,
$R^5$ eine der für $R^a$ angegebenen Bedeutungen, oder falls m = 1 ist, auch F, Cl, $CF_3$, $OCF_3$ bedeutet,
$L^1$ und $L^2$ jeweils unabhängig voneinander H oder F

bedeuten,
enthält.

**7.** Flüssigkristallines Medium nach einem der Ansprüche 1 bis 6, **dadurch gekennzeichnet, dass** das Medium zusätzlich eine oder mehrere Verbindungen der Formel VII,

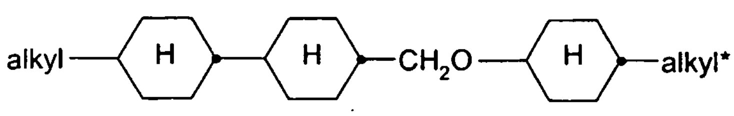
VII

worin
alkyl und alkyl* jeweils unabhängig voneinander eine Alkylgruppe mit 1 bis 7 C-Atomen
bedeuten,
enthält.

**8.** Flüssigkristallines Medium nach einem der Ansprüche 1 bis 7, **dadurch gekennzeichnet, dass** das Medium zusätzlich ein oder mehrere Tolanverbindungen der Formeln T2a, T2b und/oder T2c,

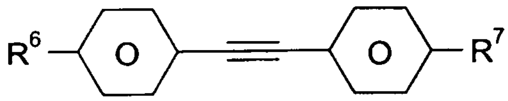

T2a

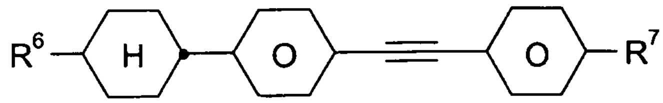

T2b

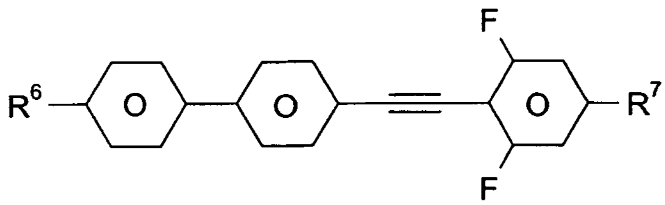

T2c

worin

$R^6$ und $R^7$ einen unsubstituierten, einen einfach durch CN oder $CF_3$ oder einen mindestens einfach durch Halogen substituierten Alkylrest mit 1 bis 12 C-Atomen, wobei in diesen Resten auch eine oder mehrere $CH_2$-Gruppen jeweils unabhängig voneinander durch -O-, -S-,

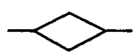 ,

-CH=CH-, -C≡C-, -CO-, -CO-O-, -O-CO- oder -O-CO-O- so ersetzt sein können, dass O-Atome nicht direkt miteinander verknüpft sind,

bedeuten,
enthält.

**9.** Flüssigkristallines Medium nach einem der Ansprüche 1 bis 8, **dadurch gekennzeichnet, dass** das Medium 5-30 Gew.% an Verbindungen der Formel A enthält.

**10.** Flüssigkristallines Medium nach einem der Ansprüche 1 bis 9, **dadurch gekennzeichnet, dass** das Medium 3-30 Gew.% an Verbindungen der Formel B enthält.

**11.** Flüssigkristallines Medium nach einem der Ansprüche 1 bis 10, **dadurch gekennzeichnet, dass** es mehr als 20 % an Verbindungen mit einer dielektrischen Anisotropie von $\Delta\varepsilon \geq +12$ enthält.

**12.** Verwendung des flüssigkristallinen Mediums nach Anspruch 1 für elektrooptische Zwecke.

**13.** Verwendung des flüssigkristallinen Mediums nach Anspruch 1 für TN-, STN- und IPS-Anwendungen.

**14.** Elektrooptische Flüssigkristallanzeige enthaltend ein flüssigkristallines Medium nach Anspruch 1.

**15.** Flüssigkristalline Verbindungen der Formel A

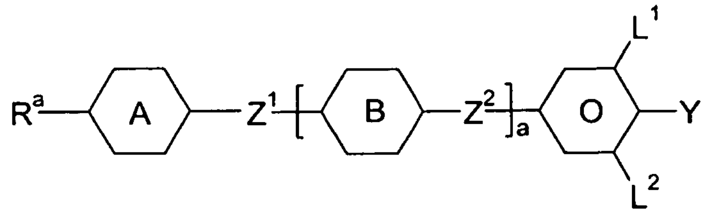

A

worin
$R^a$,

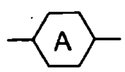 , 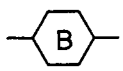 ,

a, $L^2$ und Y die in Anspruch 1 angegebenen Bedeutungen haben,

**$L^1$ F, und**
$Z^1$ und $Z^2$ jeweils unabhängig voneinander $-CH_2CH_2-$, $-(CH_2)_4-$, $-CH_2O-$ $-OCH_2-$, $-OCF_2-$, -C≡C-, -CF=CF-, -CH=CH-, -COO-, $-CH_2-$, $-(CH_2)_3-$ oder eine Einfachbindung,

bedeutet.

**16.** Flüssigkristalline Verbindungen **nach Anspruch 15** der **folgenden** Formeln

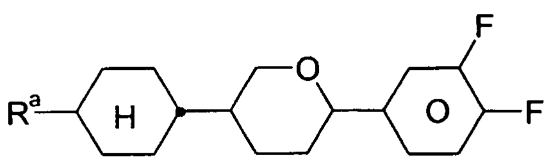

A-1*

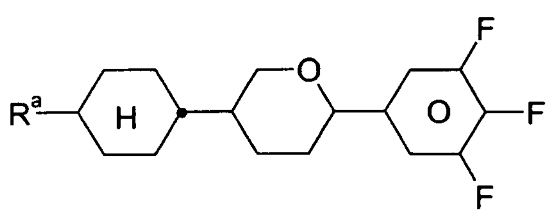

A-2

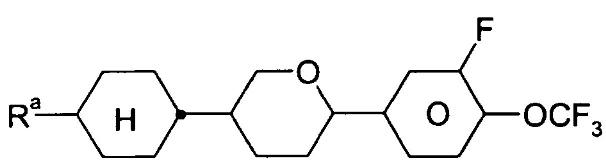

A-3*

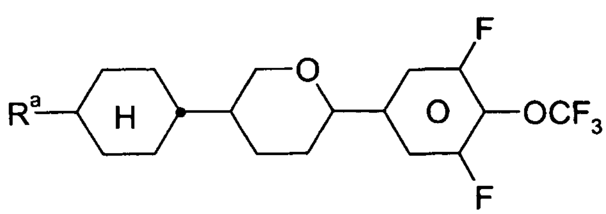

A-4

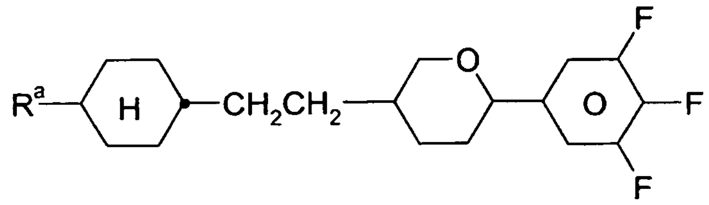

A-5

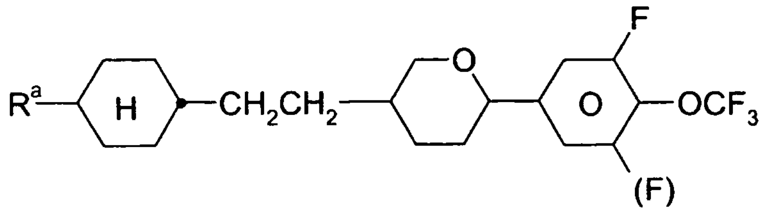

A-6*

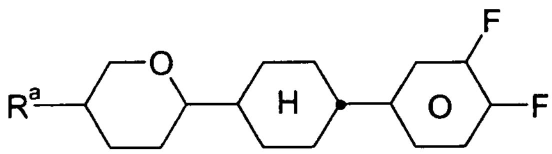

A-7

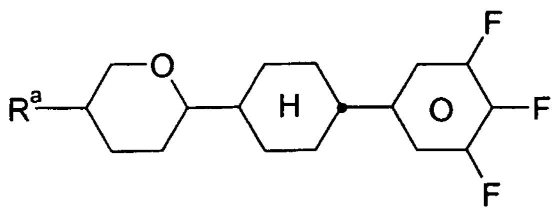

A-8

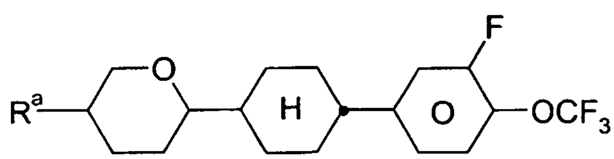

A-9*

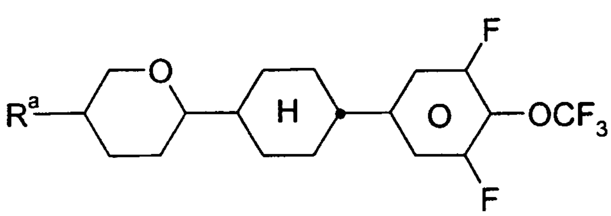

A-10

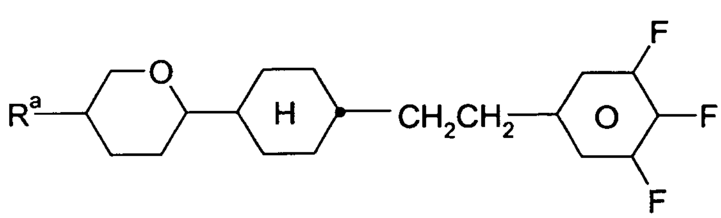

A-11

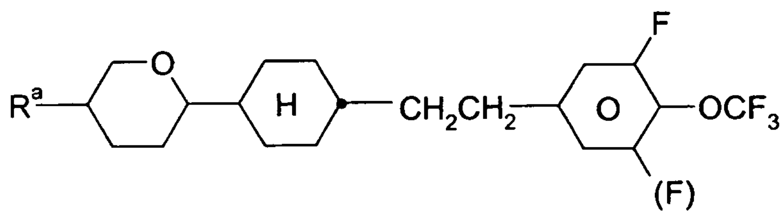

A-12

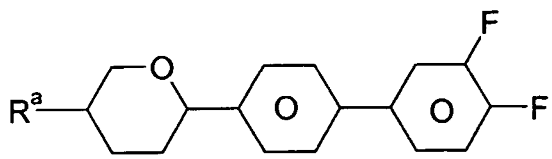

A-14

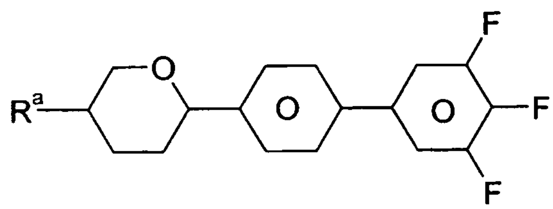

A-15

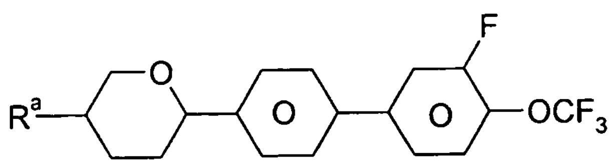

A-17

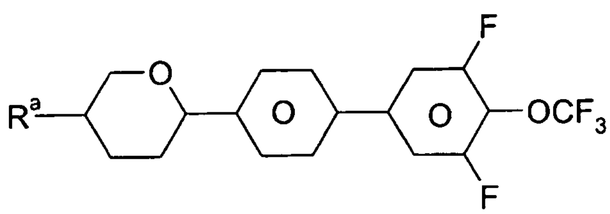

A-18

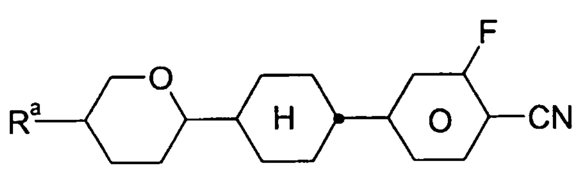

A-20

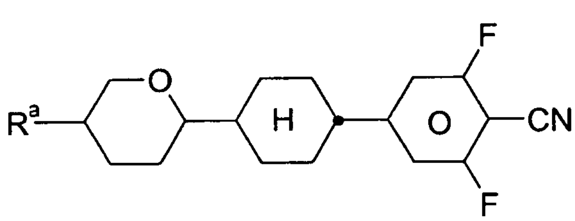

A-21

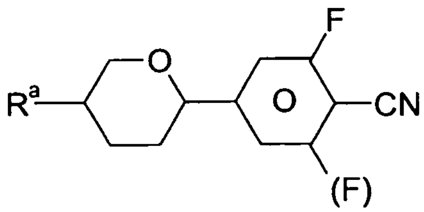

A-22*

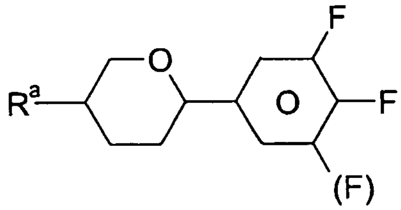

A-23*

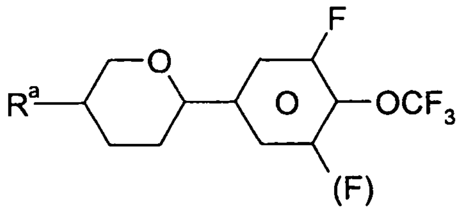

A-24*

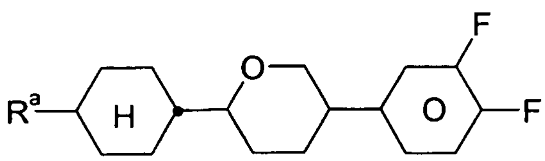

A-29*

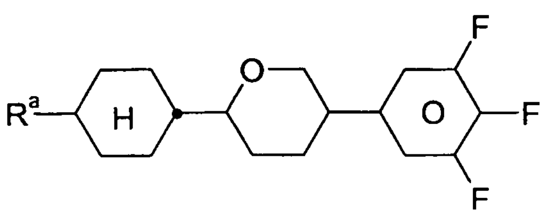

A-30

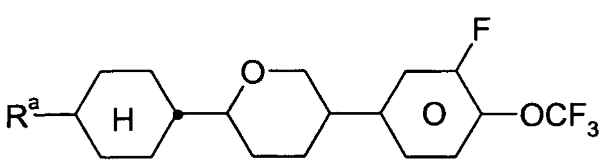

A-31*

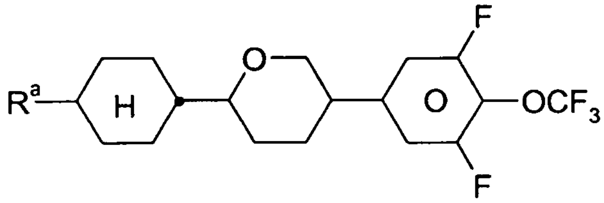

A-32

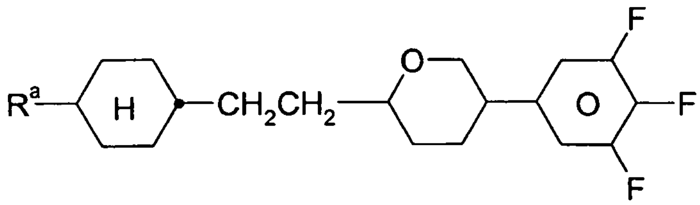

A-33

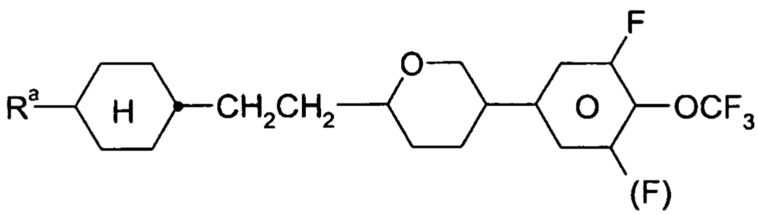

A-34*

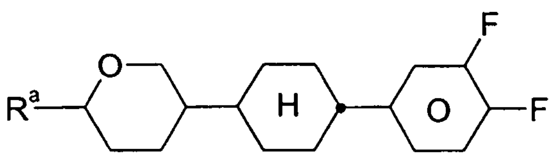

A-35

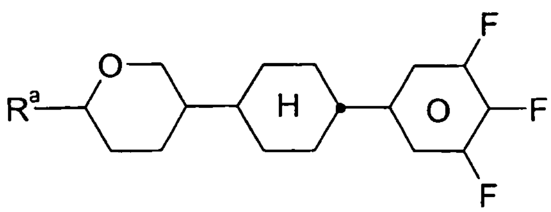

A-36

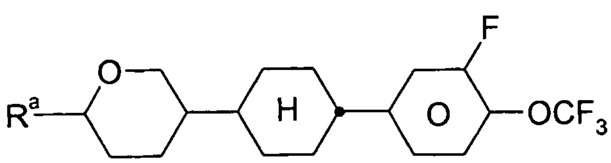

A-37*

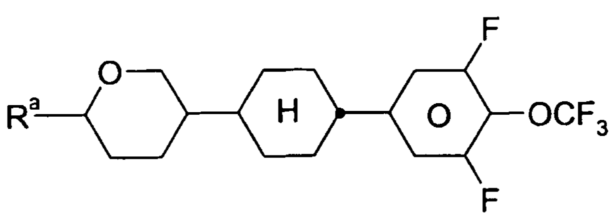

A-38

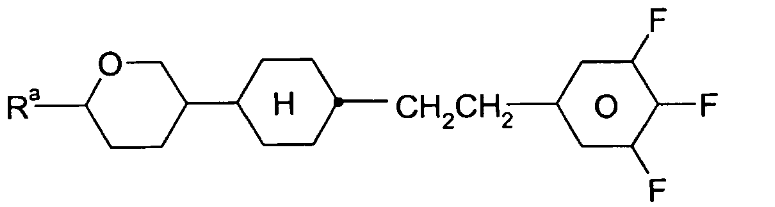

A-39

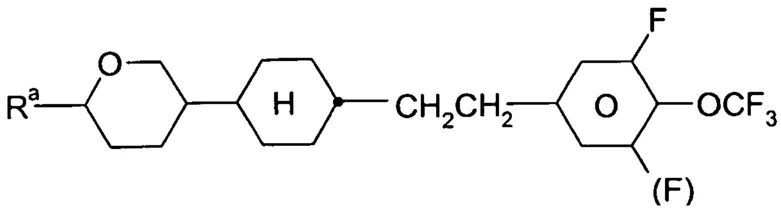

A-40

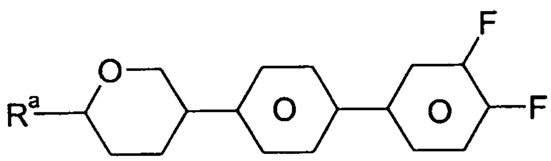

A-42

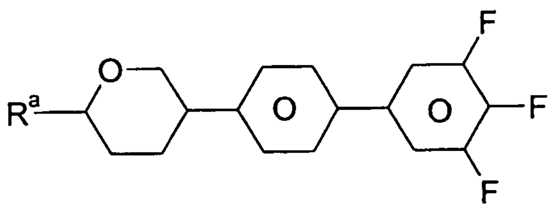

A-43

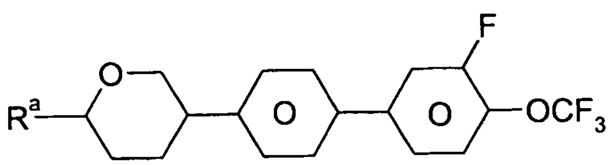

A-45

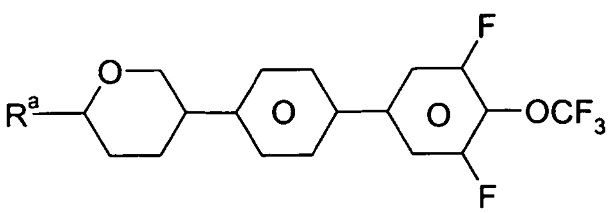

A-46

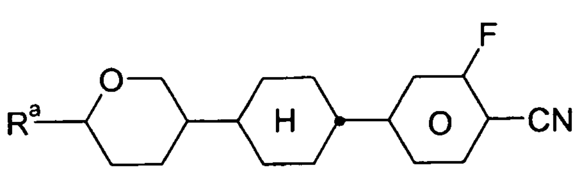

A-48

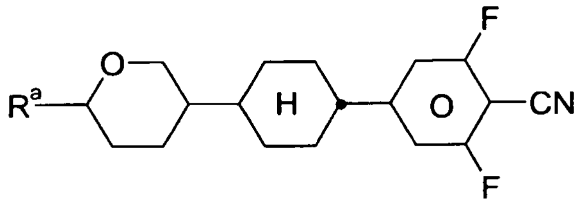

A-49

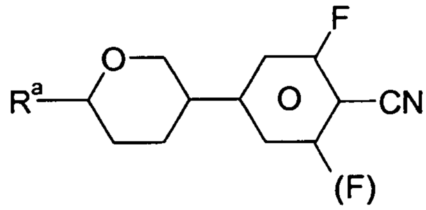

A-50*

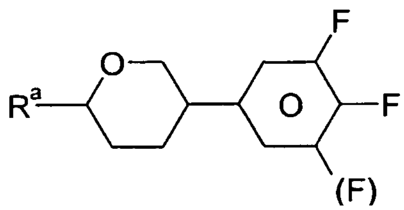

A-51*

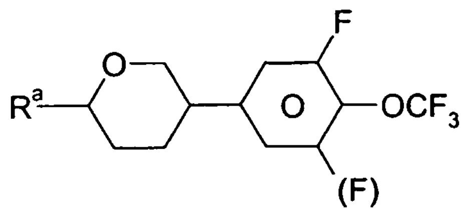

A-52*

worin $R^a$ die in Anspruch 1 angegebenen Bedeutungen hat.

**17.** TN- oder STN-Flüssigkristallanzeige mit

- zwei Trägerplatten, die mit einer Umrandung eine Zelle bilden,
- einer in der Zelle befindlichen nematischen Flüssigkristallmischung mit positiver dielektrischer Anisotropie,
- Elektrodenschichten mit Orientierungsschichten auf den Innenseiten der Trägerplatten,
- einem Anstellwinkel zwischen der Längsachse der Moleküle an der Oberfläche der Trägerplatten und den Trägerplatten von 0 Grad bis 30 Grad, und
- einem Verdrillungswinkel der Flüssigkristallmischung in der Zelle von Orientierungsschicht zu Orientierungsschicht dem Betrag nach zwischen 22,5° und 600°,
- einer nematischen Flüssigkristallmischung bestehend aus

a) 15 - 75 Gew.% einer flüssigkristallinen Komponente A, bestehend aus einer oder mehreren Verbindungen mit einer dielektrischen Anisotropie von über +1,5;
b) 25 - 85 Gew.% einer flüssigkristallinen Komponente B, bestehend aus einer oder mehreren Verbindungen mit einer dielektrischen Anisotropie zwischen -1,5 und +1,5;
c) 0 - 20 Gew.% einer flüssigkristallinen Komponente D, bestehend aus einer oder mehreren Verbindungen mit einer dielektrischen Anisotropie von unter -1,5 und
d) gegebenenfalls einer optisch aktiven Komponente C in einer Menge, dass das Verhältnis zwischen Schichtdicke (Abstand der Trägerplatten) und natürlicher Ganghöhe der chiralen nematischen Flüssigkristallmischung etwa 0,2 bis 1,3 beträgt,

**dadurch gekennzeichnet, dass** Komponente A mindestens eine Verbindung der Formel A

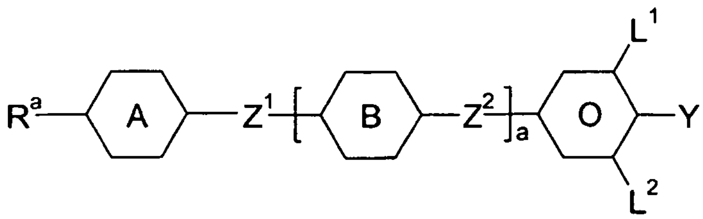

A

und mindestens eine Verbindung der Formel B,

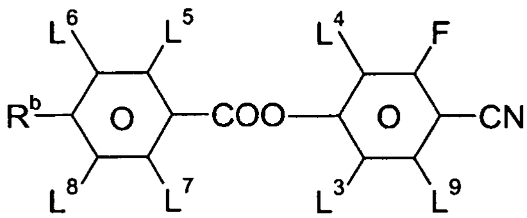

B

worin

$R^a$ und $R^b$ jeweils unabhängig voneinander H, einen unsubstituierten, einen einfach durch CN oder $CF_3$ oder einen mindestens einfach durch Halogen substituierten Alkylrest mit 1 bis 12 C-Atomen, wobei in diesen Resten auch eine oder mehrere $CH_2$Gruppen jeweils unabhängig voneinander durch -O-, -S-,

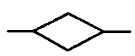 ,

-CH=CH-, -C≡C-, -CO-, -CO-O-, -O-CO- oder -O-CO-O- so ersetzt sein können, dass O-Atome nicht direkt miteinander verknüpft sind,

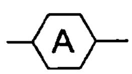 und 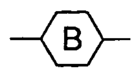

jeweils unabhängig voneinander

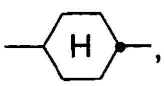, 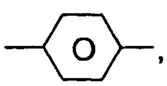, 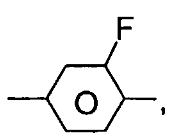, 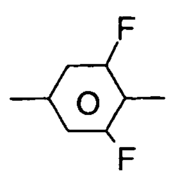

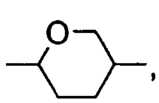, 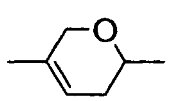 oder 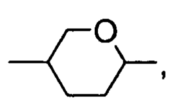,

mindestens ein Ring bedeutet

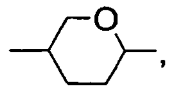,

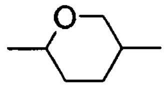 oder 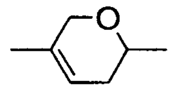,

$Z^1$ und $Z^2$ jeweils unabhängig voneinander $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ oder eine Einfachbindung,
a 0 oder 1,
$L^1$ bis $L^9$ jeweils unabhängig voneinander H oder F, und
Y F, Cl, CN, $SF_5$, NCS, OCN, SCN oder ein einfach oder mehrfach halogenierter Alkyl-, Alkoxy-, Alkenyl- oder Alkenyloxyrest mit jeweils 1 bis zu 5 C-Atomen,

bedeuten,
enthält.

## Claims

1. Liquid-crystalline medium, **characterised in that** it comprises one or more compounds of the formula A

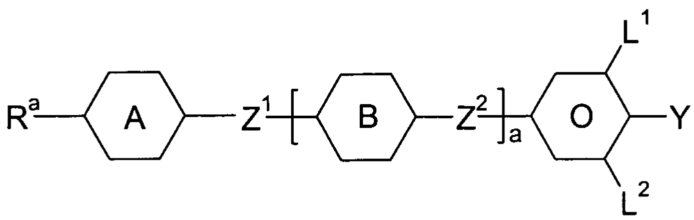 A

and at least one compound of the formula B

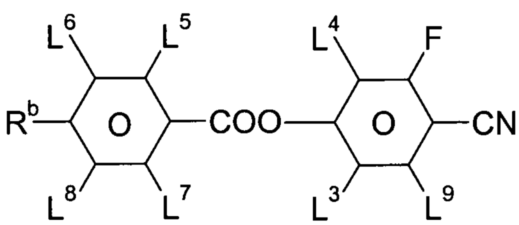 B

in which

$R^a$ and $R^b$ each, independently of one another, denote H, an alkyl radical having 1 to 12 C atoms which is unsubstituted, monosubstituted by CN or $CF_3$ or at least monosubstituted by halogen, where one or more $CH_2$ groups in these radicals may also each, independently of one another, be replaced by -O-, -S-,

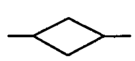,

-CH=CH-, -C≡C-, -CO-, -CO-O-, -O-CO- or -O-CO-O- in such a way that O atoms are not linked directly to one another,

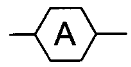 and 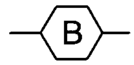

each, independently of one another, denote

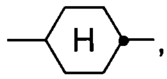, 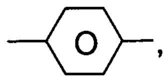, 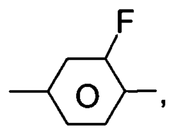, 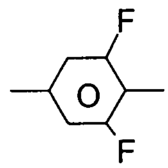

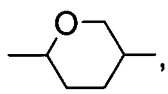, 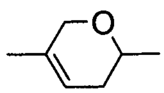 or 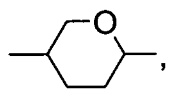,

at least one ring denotes

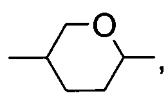,

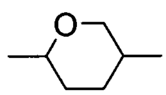 or 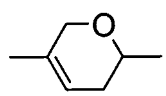,

$Z^1$ and $Z^2$ each, independently of one another, denote $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ or a single bond,
a denotes 0 or 1,
$L^1$ to $L^9$ each, independently of one another, denote H or F, and
Y denotes F, Cl, $SF_5$, CN, NCS, OCN, SCN or a mono- or polyhalogenated alkyl, alkoxy, alkenyl or alkenyloxy radical, in each case having 1 to 5 C atoms.

2. Liquid-crystalline medium according to Claim 1, **characterised in that** it comprises at least one compound of the formulae A-1 to A-56

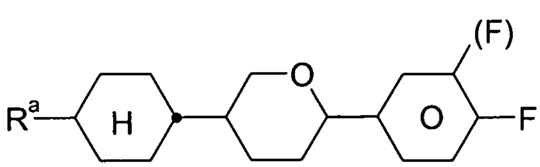

A-1

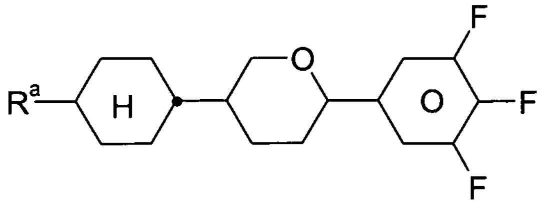

A-2

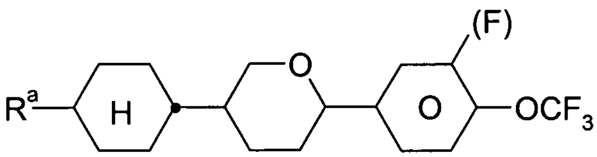

A-3

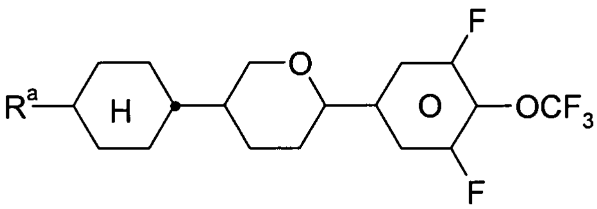

A-4

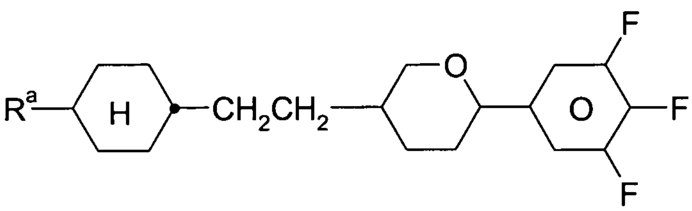

A-5

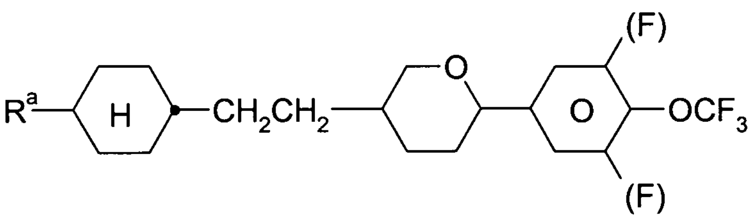

A-6

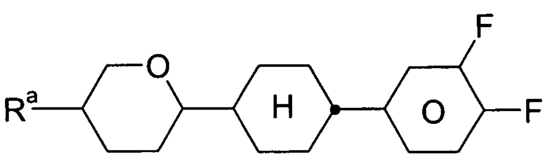

A-7

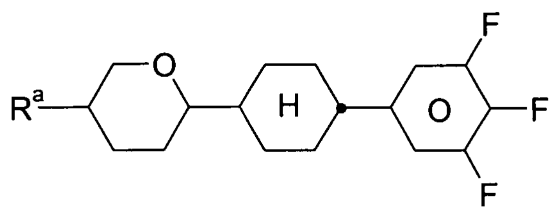

A-8

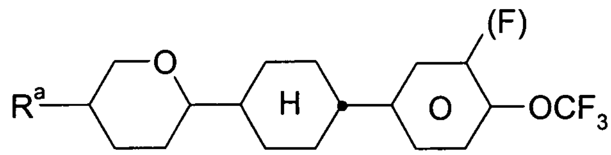

A-9

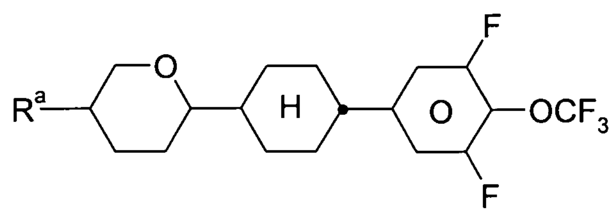

A-10

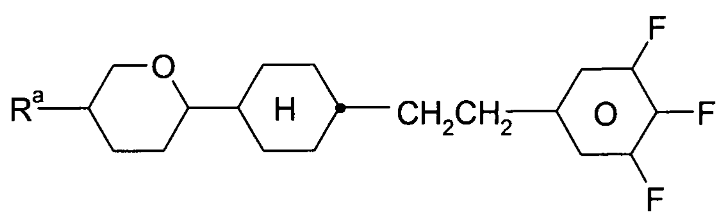

A-11

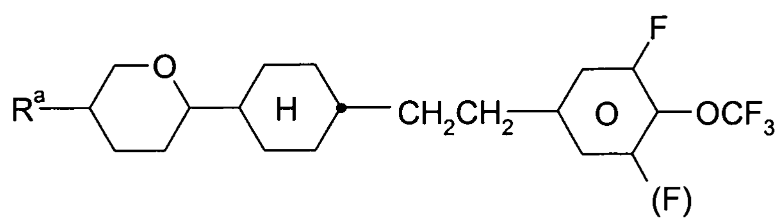

A-12

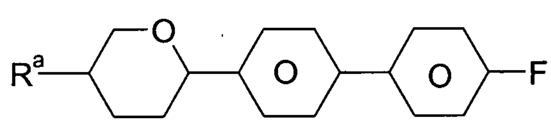

A-13

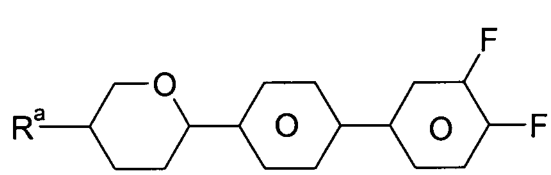

A-14

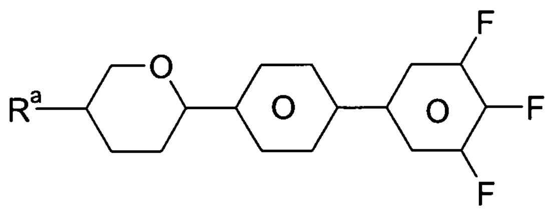

A-15

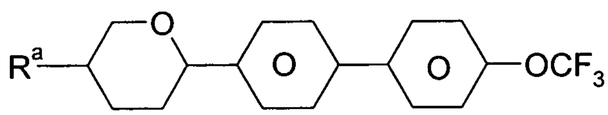

A-16

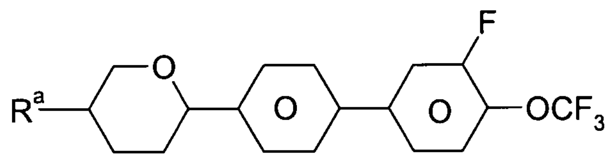

A-17

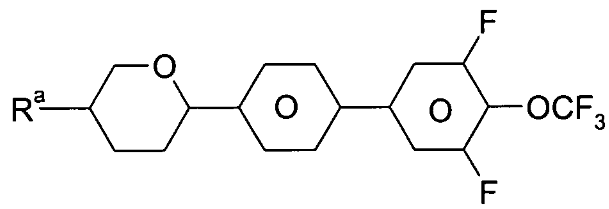

A-18

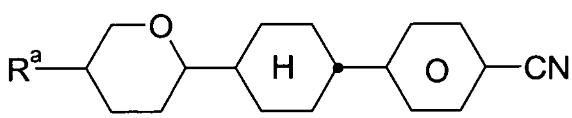

A-19

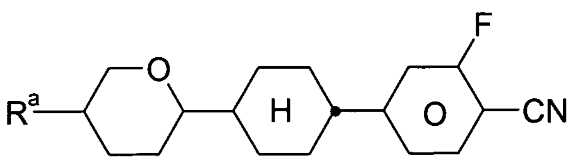

A-20

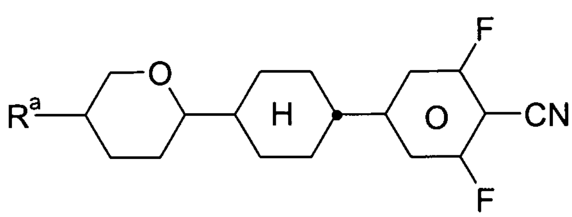

A-21

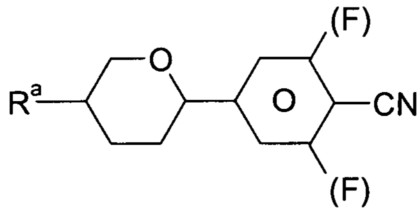

A-22

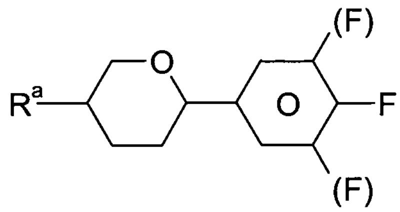

A-23

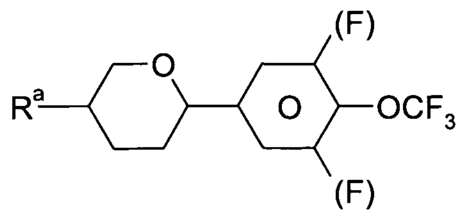

A-24

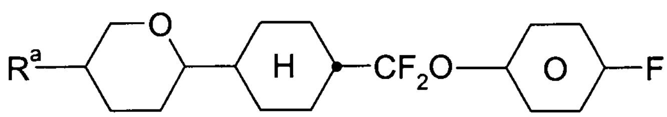

A-25

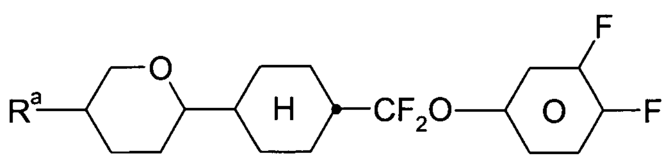

A-26

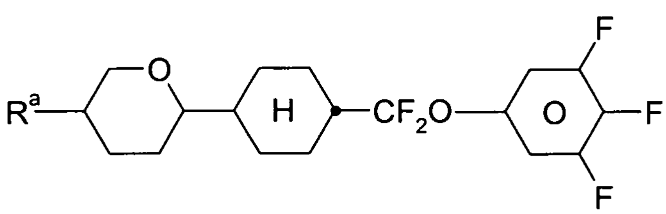

A-27

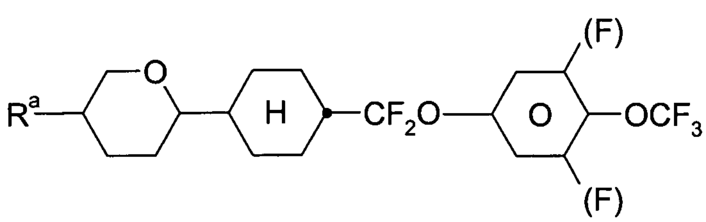

A-28

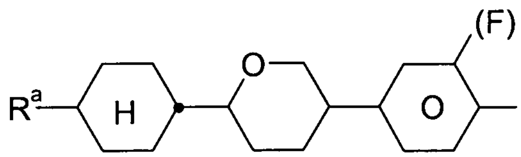

A-29

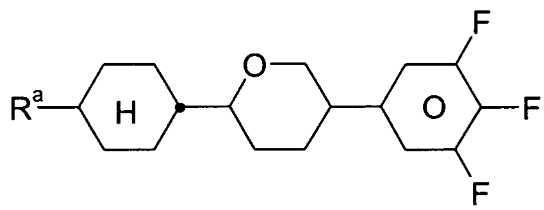

A-30

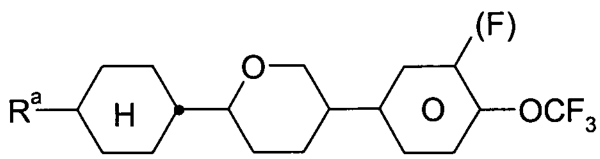

A-31

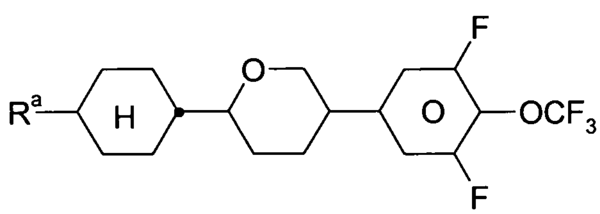

A-32

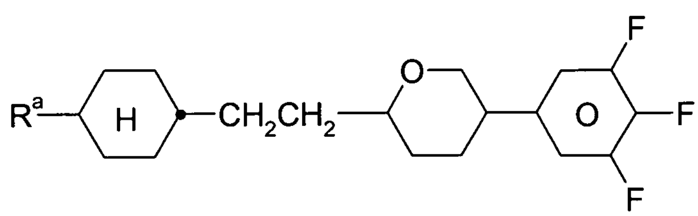

A-33

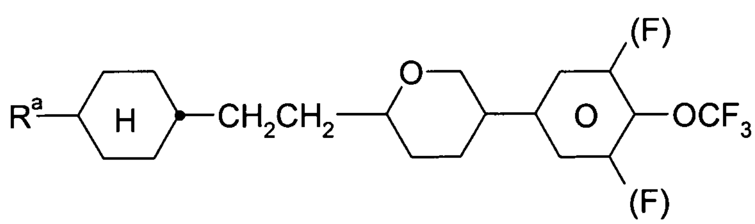

A-34

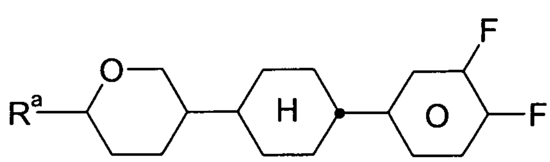

A-35

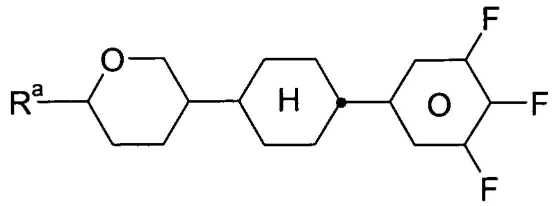

A-36

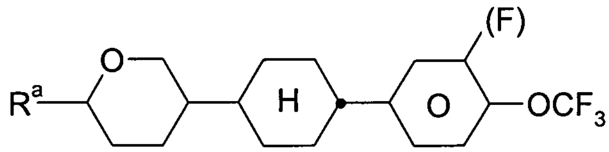

A-37

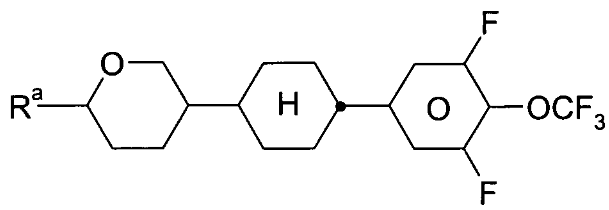

A-38

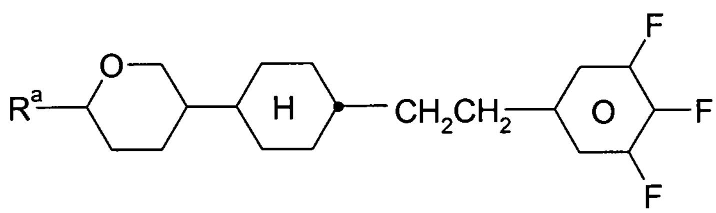

A-39

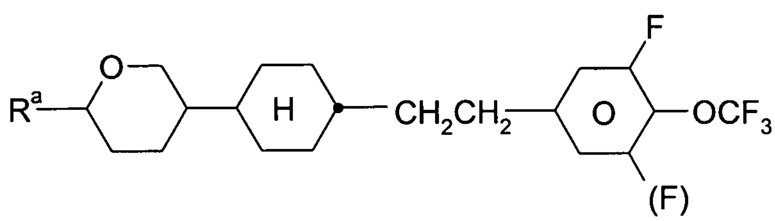

A-40

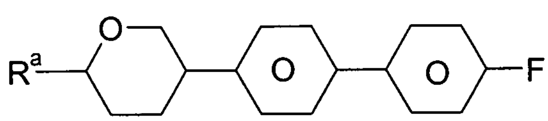

A-41

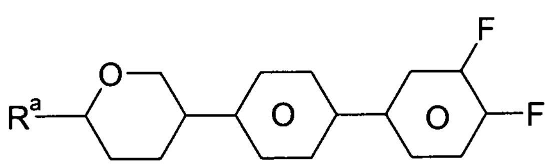

A-42

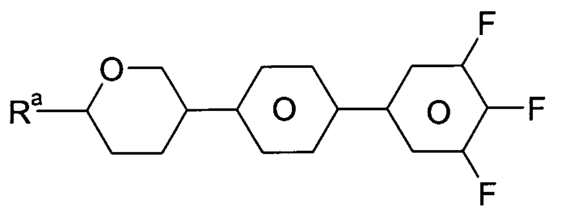

A-43

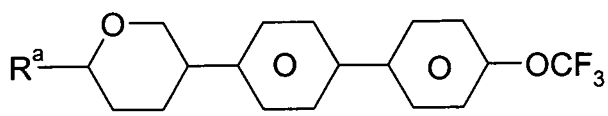

A-44

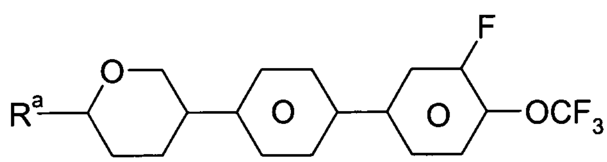

A-45

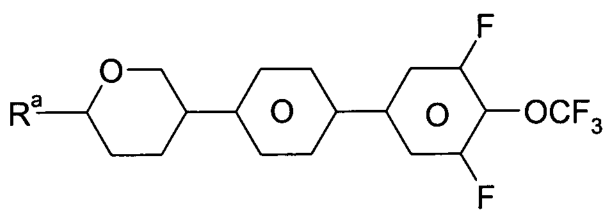

A-46

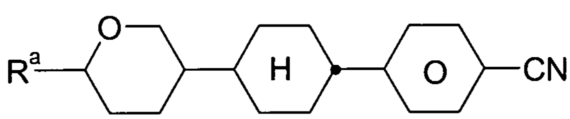

A-47

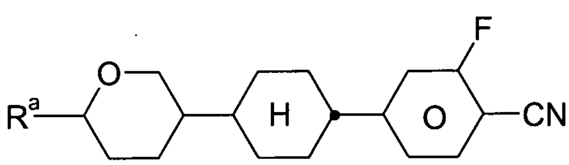

A-48

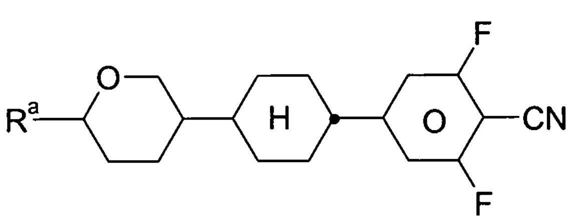

A-49

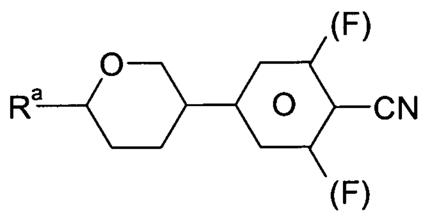

A-50

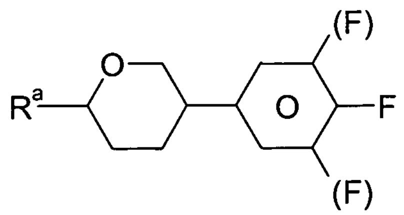

A-51

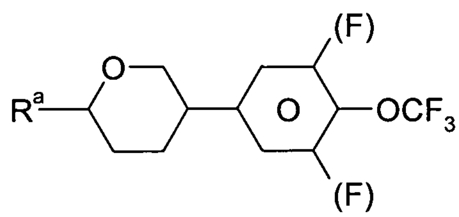

A-52

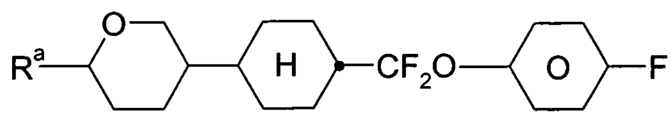

A-53

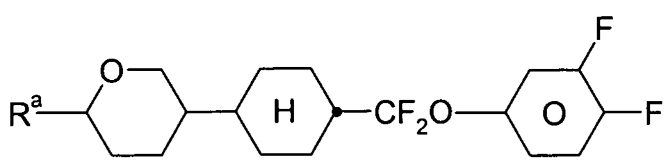

A-54

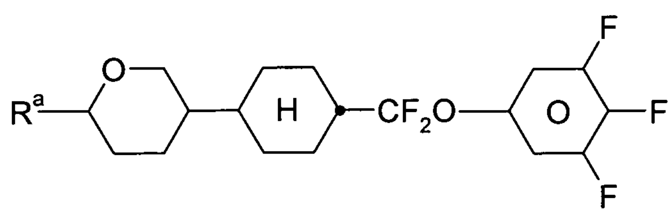

A-55

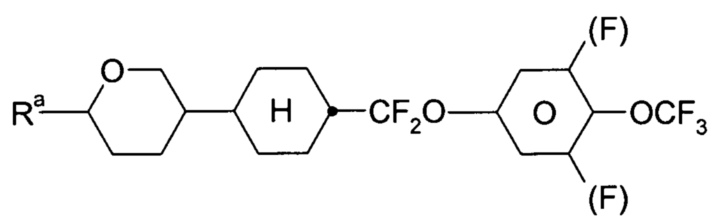

A-56

in which $R^a$ has the meanings indicated in Claim 1.

**3.** Liquid-crystalline medium according to Claim 1 or 2, **characterised in that** it comprises at least one compound of the formulae B-1 to B-6

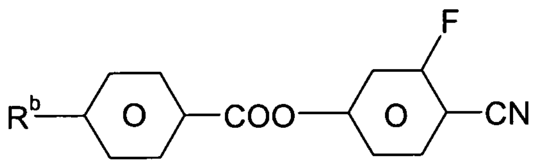

B-1

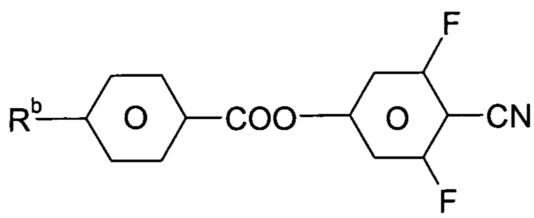

B-2

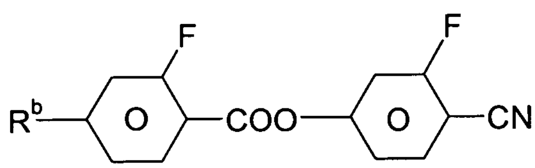

B-3

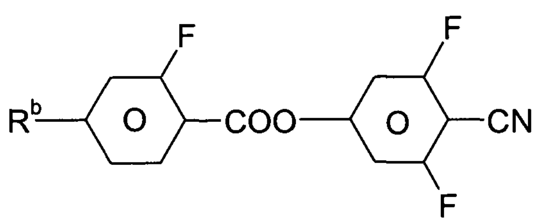

B-4

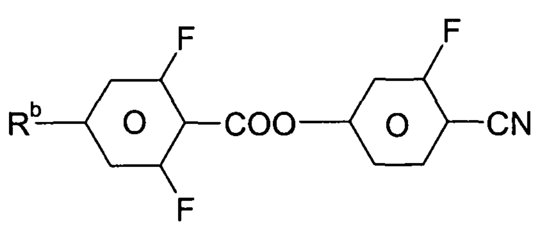

B-5

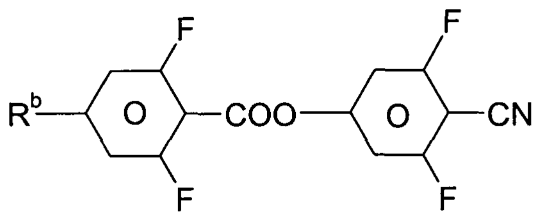

B-6

in which
$R^b$ has the meanings indicated in Claim 1.

**4.** Liquid-crystalline medium according to one of Claims 1 to 3, **characterised in that** it additionally comprises at least one compound of the formulae IIa to IIk

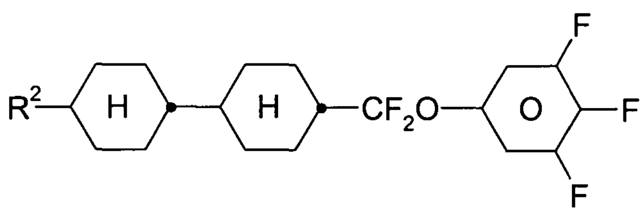

IIa

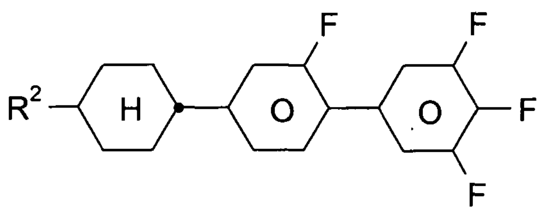

IIb

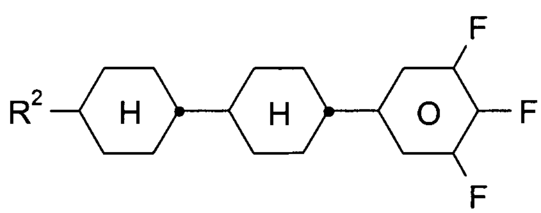

IIc

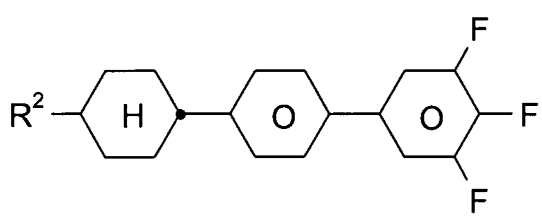

IId

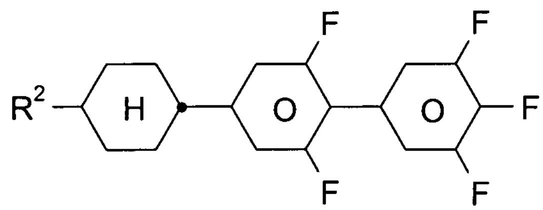

IIe

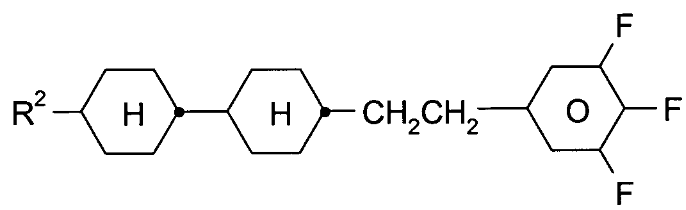

IIf

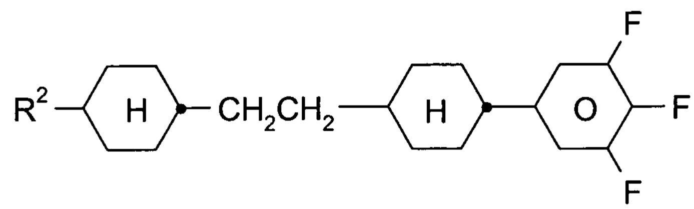

IIg

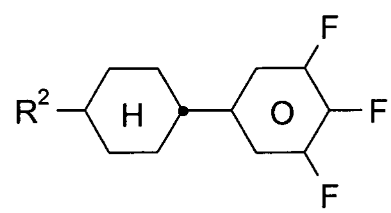

IIh

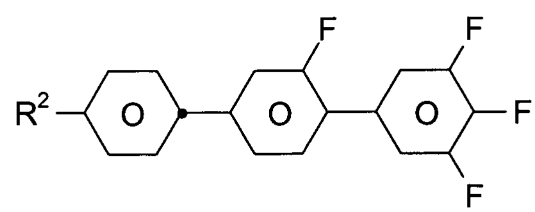

IIi

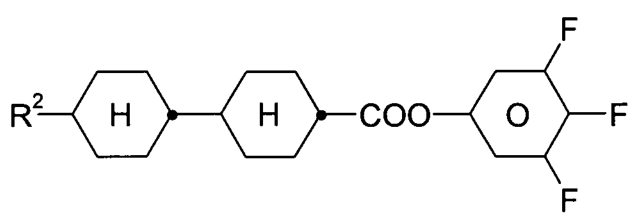

IIj

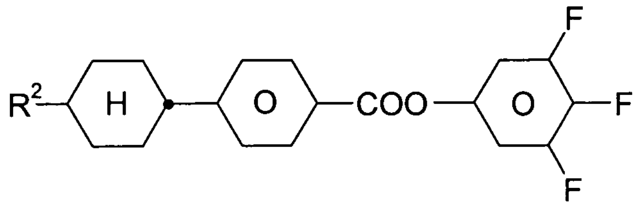

IIk

in which

$R^2$ denotes an alkyl radical having 1 to 12 C atoms which is unsubstituted, monosubstituted by CN or $CF_3$ or at least monosubstituted by halogen, where one or more $CH_2$ groups in these radicals may also each, independently of one another, be replaced by -O-, -S-,

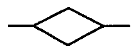

-CH=CH-, -C≡C-, -CO-, -CO-O-, -O-CO- or -O-CO-O- in such a way that O atoms are not linked directly to one another.

**5.** Liquid-crystalline medium according to one of Claims 1 to 4, **characterised in that** it additionally comprises one or more cyano compounds of the formulae IIIa to IIIj

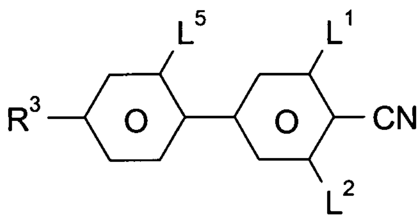

IIIa

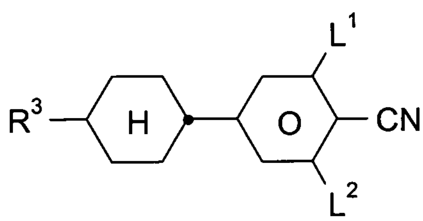

IIIb

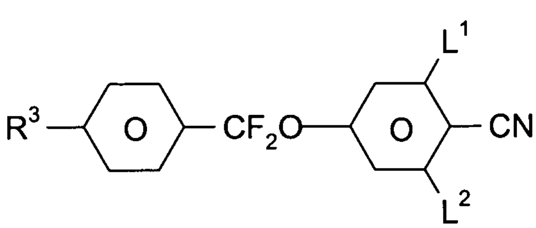

IIIc

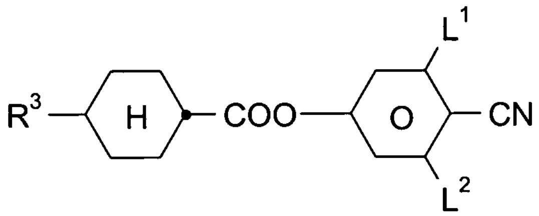

IIId

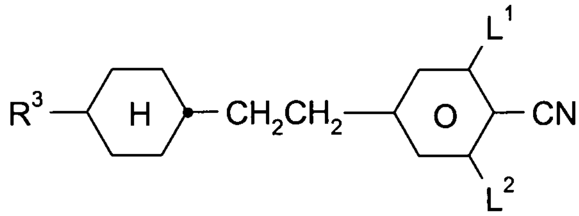

IIIe

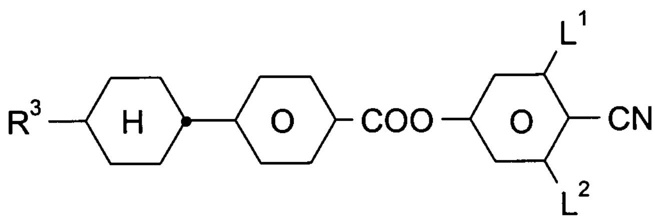

IIIf

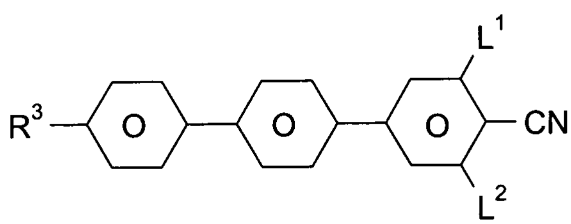

IIIg

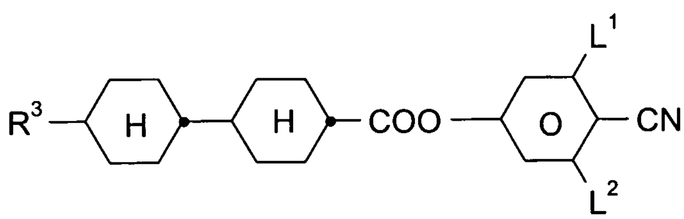

IIIh

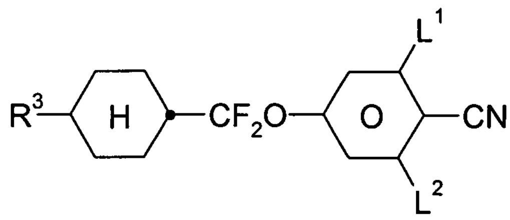

IIIi

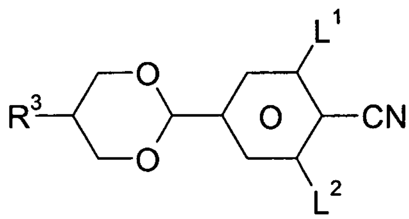

IIIj

in which

$R^3$ denotes an alkyl radical having 1 to 12 C atoms which is unsubstituted, monosubstituted by CN or $CF_3$ or at least monosubstituted by halogen, where one or more $CH_2$ groups in these radicals may also each, independently of one another, be replaced by -O-, -S-,

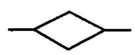 ,

-CH=CH-, -C≡C-, -CO-, -CO-O-, -O-CO- or -0-CO-0- in such a way that O atoms are not linked directly to one another, and
$L^1$, $L^2$ and $L^5$ each, independently of one another, denote H or F.

**6.** Liquid-crystalline medium according to one of Claims 1 to 5, **characterised in that** it additionally comprises one or more compounds of the formula IV

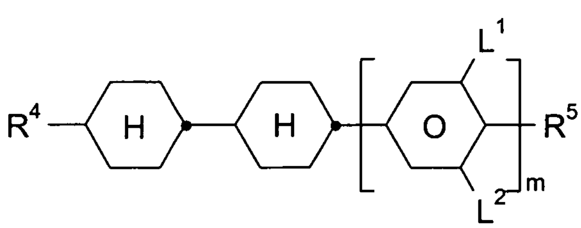

IV

in which

m denotes 0 or 1,
$R^4$ denotes an alkenyl group having 2 to 7 C atoms,
$R^5$ has one of the meanings indicated for $R^a$ or, if m = 1, alternatively denotes F, Cl, $CF_3$, $OCF_3$,
$L^1$ and $L^2$ each, independently of one another, denote H or F.

**7.** Liquid-crystalline medium according to one of Claims 1 to 6, **characterised in that** the medium additionally comprises one or more compounds of the formula VII

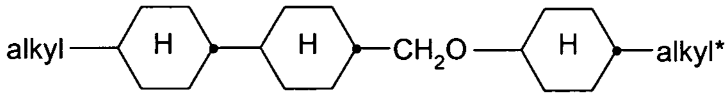 VII

in which
alkyl and alkyl* each, independently of one another, denote an alkyl group having 1 to 7 C atoms.

**8.** Liquid-crystalline medium according to one of Claims 1 to 7, **characterised in that** the medium additionally comprises one or more tolan compounds of the formulae T2a, T2b and/or T2c

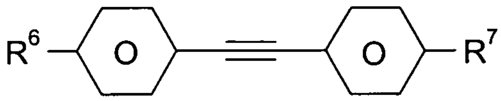 T2a

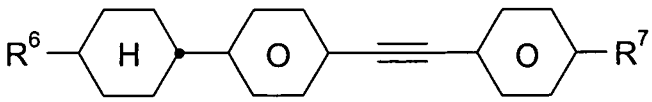 T2b

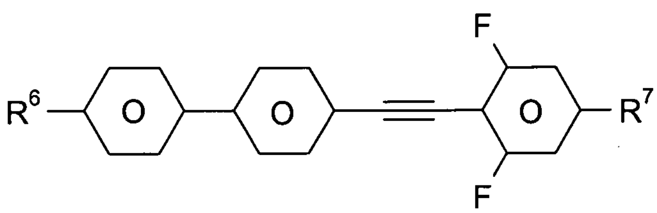 T2c

in which

$R^6$ and $R^7$ denote an alkyl radical having 1 to 12 C atoms which is unsubstituted, monosubstituted by CN or $CF_3$ or at least monosubstituted by halogen, where one or more $CH_2$ groups in these radicals may also each, independently of one another, be replaced by -O-, -S-,

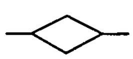 ,

-CH=CH-, -C≡C-, -CO-, -CO-O-, -O-CO- or -O-CO-O- in such a way that O atoms are not linked directly to one another.

**9.** Liquid-crystalline medium according to one of Claims 1 to 8, **characterised in that** the medium comprises 5-30% by weight of compounds of the formula A.

**10.** Liquid-crystalline medium according to one of Claims 1 to 9, **characterised in that** the medium comprises 3-30% by weight of compounds of the formula B.

**11.** Liquid-crystalline medium according to one of Claims 1 to 10, **characterised in that** it comprises more than 20%

of compounds having a dielectric anisotropy of $\Delta\varepsilon \geq +12$.

**12.** Use of the liquid-crystalline medium according to Claim 1 for electro-optical purposes.

**13.** Use of the liquid-crystalline medium according to Claim 1 for TN, STN and IPS applications.

**14.** Electro-optical liquid-crystal display containing a liquid-crystalline medium according to Claim 1.

**15.** Liquid-crystalline compounds of the formula A

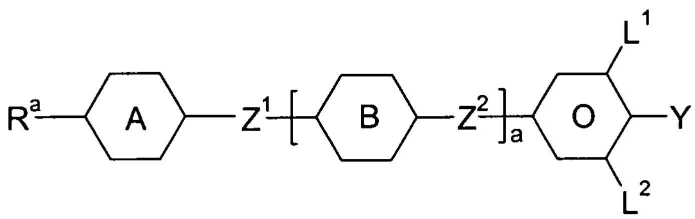

A

in which
$R^a$,

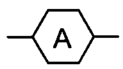 , 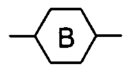 ,

a, $L^2$ and Y have the meanings indicated in Claim 1,

$L^1$ denotes F, and
$Z^1$ and $Z^2$ each, independently of one another, denote $-CH_2CH_2-$, $-(CH_2)_4-$, $-CH_2O-$, $-OCH_2-$, $-OCF_2-$, $-C{\equiv}C-$, $-CF{=}CF-$, $-CH{=}CH-$, $-COO-$, $-CH_2-$, $-(CH_2)_3-$ or a single bond.

**16.** Liquid-crystalline compounds according to Claim 15 of the following formulae

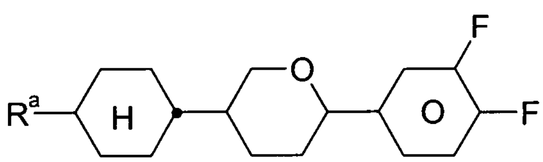

A-1*

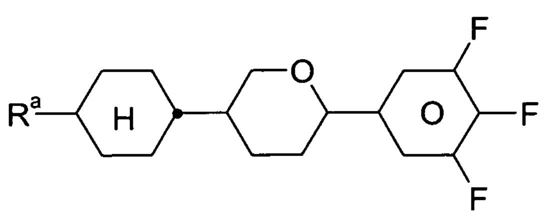

A-2

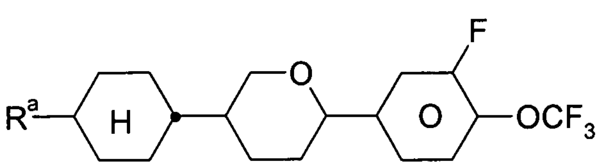

A-3*

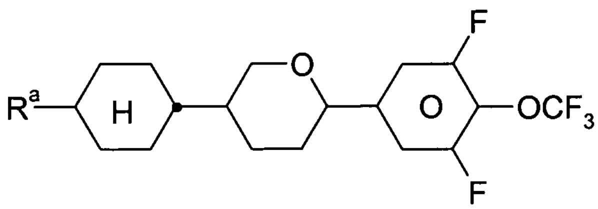

A-4

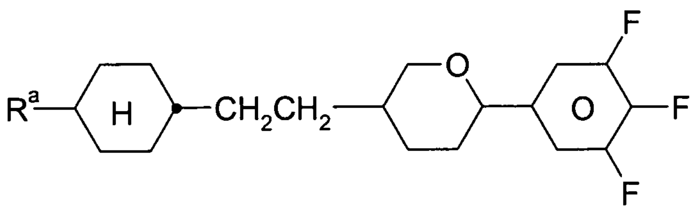

A-5

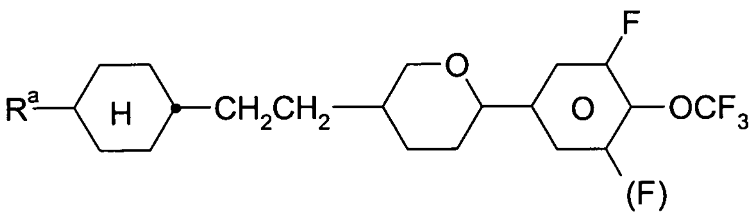

A-6*

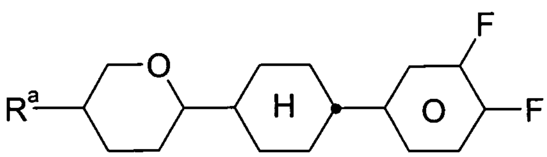

A-7

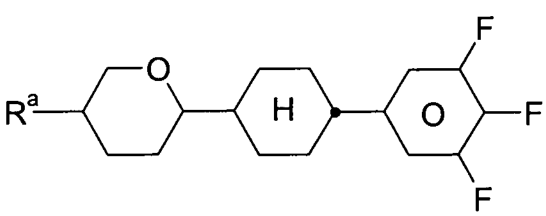

A-8

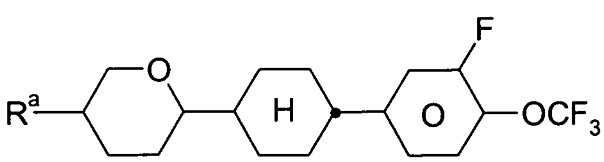

A-9*

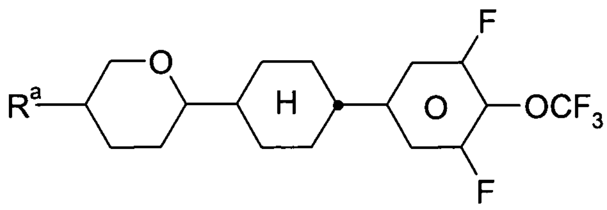

A-10

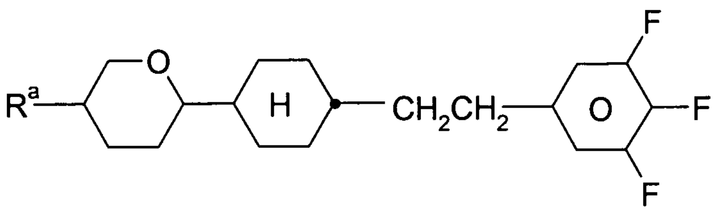

A-11

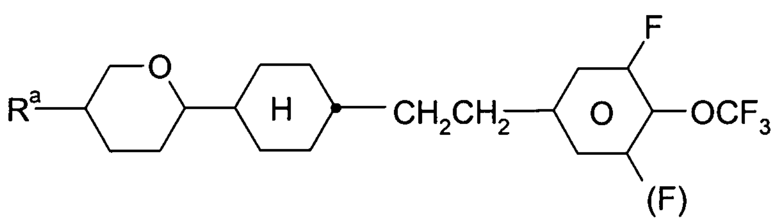

A-12

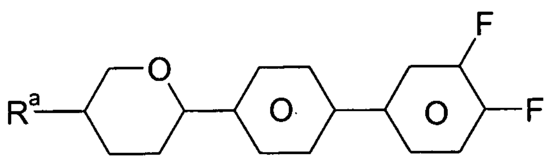

A-14

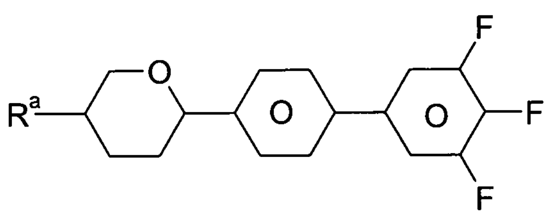

A-15

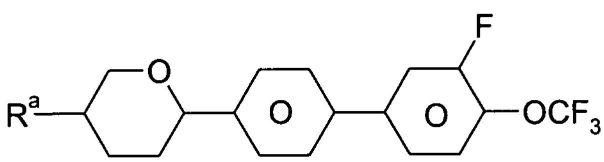

A-17

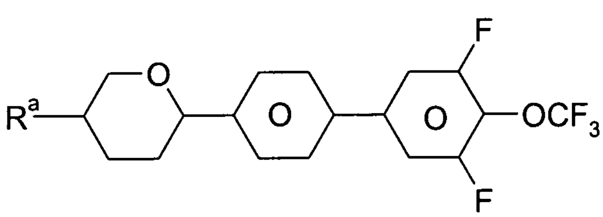

A-18

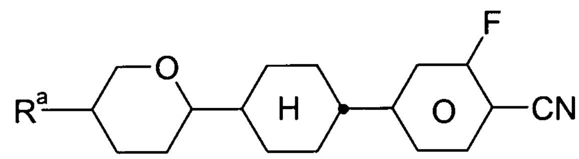

A-20

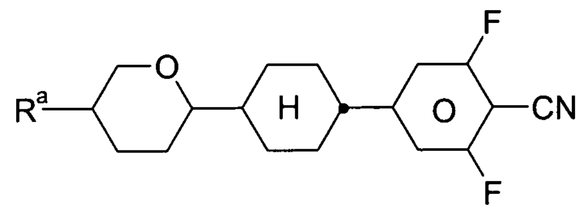

A-21

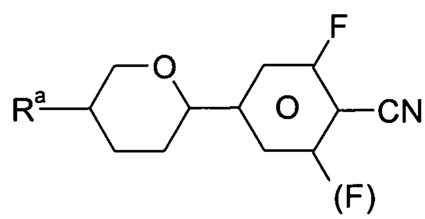

A-22*

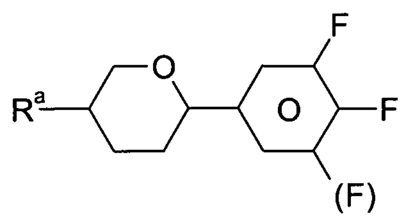

A-23*

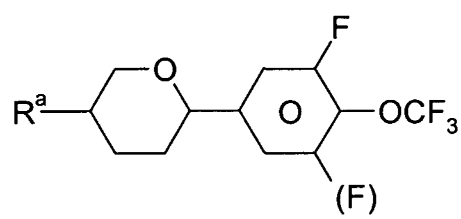

A-24*

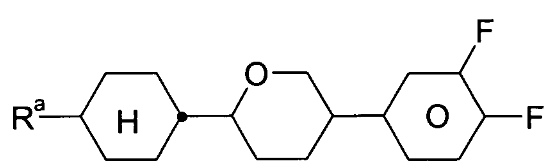

A-29*

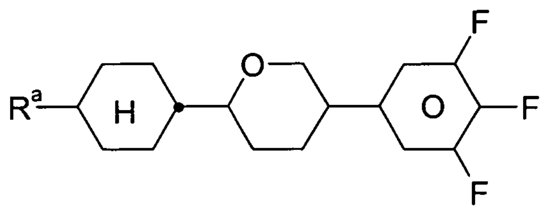

A-30

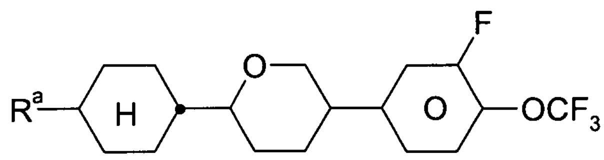

A-31*

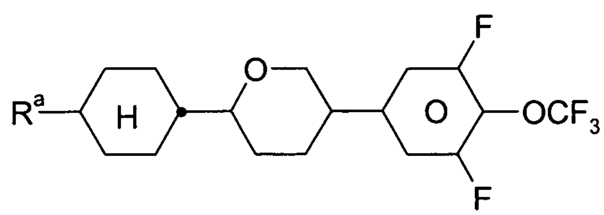

A-32

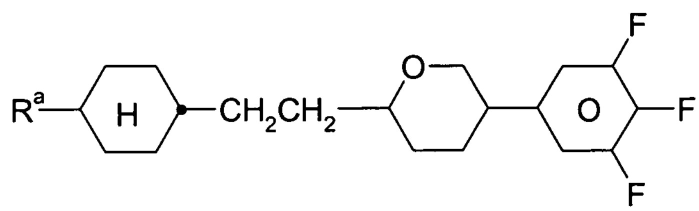

A-33

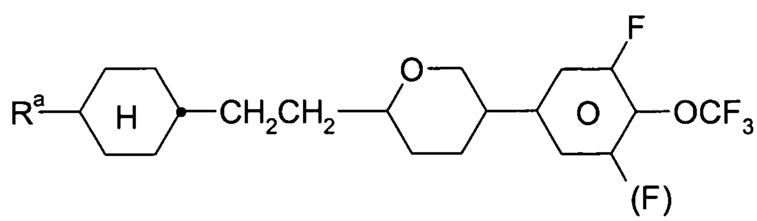

A-34*

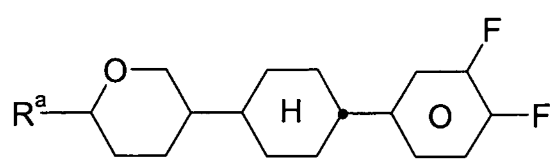

A-35

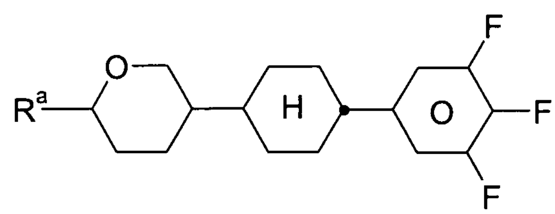

A-36

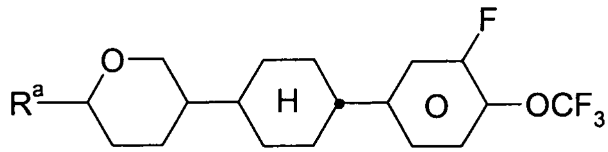

A-37*

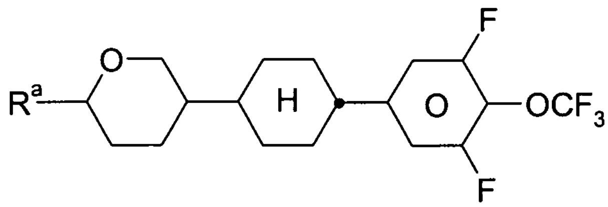

A-38

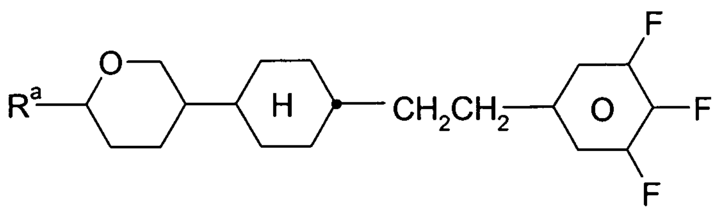

A-39

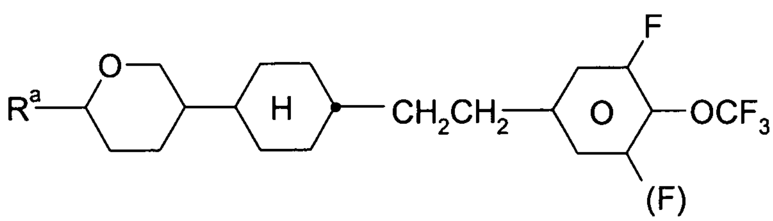

A-40

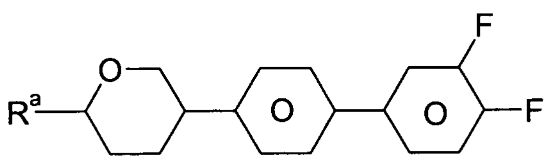

A-42

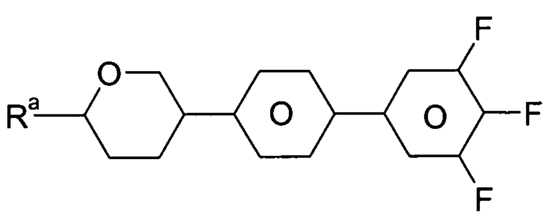

A-43

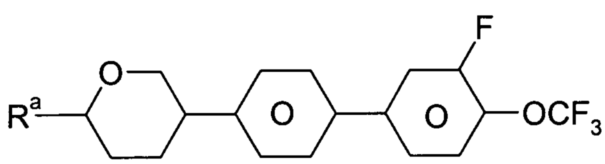

A-45

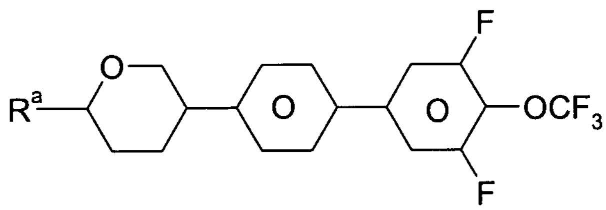

A-46

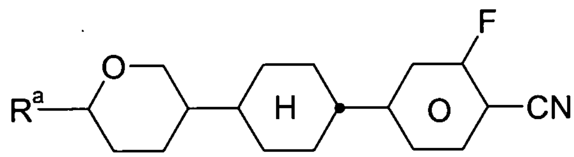

A-48

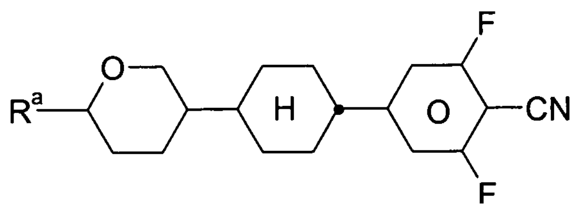

A-49

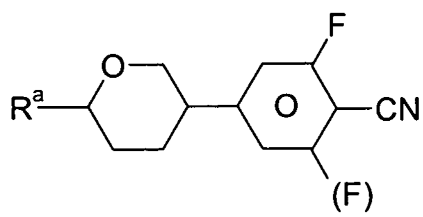

A-50*

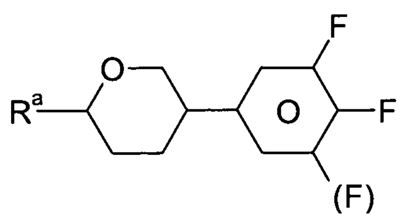

A-51*

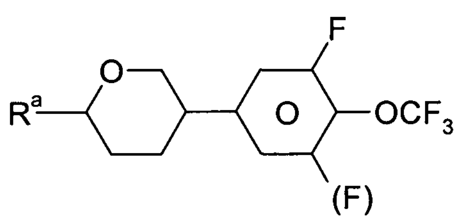

A-52*

in which $R^a$ has the meanings indicated in Claim 1.

**17.** TN or STN liquid-crystal display having

- two outer plates, which, with a frame, form a cell,
- a nematic liquid-crystal mixture of positive dielectric anisotropy located in the cell,

- electrode layers with alignment layers on the insides of the outer plates,
- a tilt angle between the longitudinal axis of the molecules at the surface of the outer plates and the outer plates of 0 degree to 30 degrees, and
- a twist angle of the liquid-crystal mixture in the cell from alignment layer to alignment layer with a value of between 22.5° and 600°,
- a nematic liquid-crystal mixture consisting of

a) 15 - 75% by weight of a liquid-crystalline component A consisting of one or more compounds having a dielectric anisotropy of greater than +1.5;
b) 25 - 85% by weight of a liquid-crystalline component B consisting of one or more compounds having a dielectric anisotropy of between -1.5 and +1.5;
c) 0 - 20% by weight of a liquid-crystalline component D consisting of one or more compounds having a dielectric anisotropy of below -1.5, and
d) if desired, an optically active component C in such an amount that the ratio between the layer thickness (separation of the outer plates) and the natural pitch of the chiral nematic liquid-crystal mixture is about 0.2 to 1.3,

**characterised in that** component A comprises at least one compound of the formula A

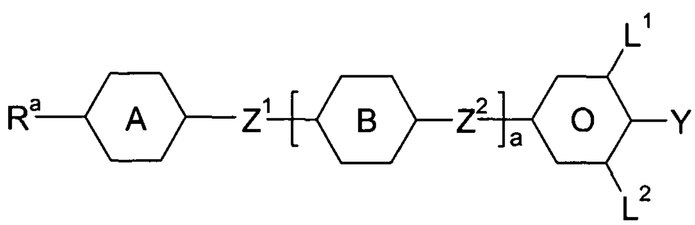

A

and at least one compound of the formula B

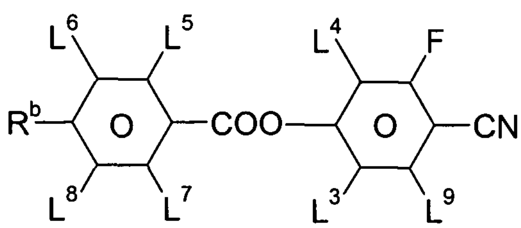

B

in which

$R^a$ and $R^b$ each, independently of one another, denote H, an alkyl radical having 1 to 12 C atoms which is unsubstituted, monosubstituted by CN or $CF_3$ or at least monosubstituted by halogen, where one or more $CH_2$ groups in these radicals may also each, independently of one another, be replaced by -O-, -S-,

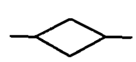 ,

-CH=CH-, -C≡C-, -CO-, -CO-O-, -O-CO- or -O-CO-O- in such a way that O atoms are not linked directly to one another,

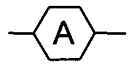 and 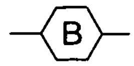

each, independently of one another, denote

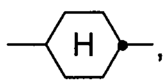, 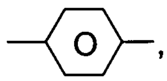, 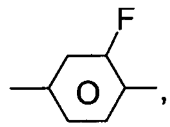, 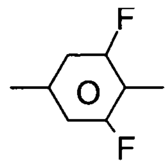

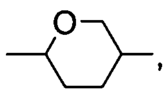, 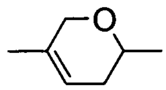 or 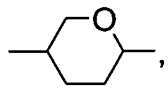,

at least one ring denotes

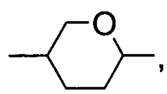,

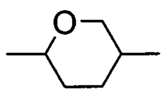 or 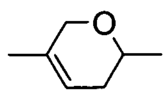,

$Z^1$ and $Z^2$ each, independently of one another, denote $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ or a single bond,
a denotes 0 or 1,
$L^1$ to $L^9$ each, independently of one another, denote H or F, and
Y denotes F, Cl, CN, $SF_5$, NCS, OCN, SCN or a mono- or polyhalogenated alkyl, alkoxy, alkenyl or alkenyloxy radical, in each case having 1 to 5 C atoms.

## Revendications

**1.** Milieu de cristal liquide, **caractérisé en ce qu'**il comprend un ou plusieurs composés de la formule A

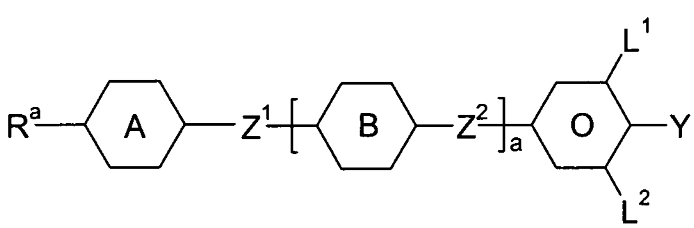

A

et au moins un composé de la formule B

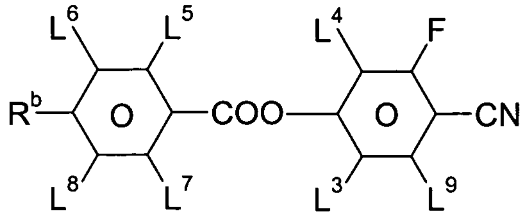

B

dans lesquelles

$R^a$ et $R^b$ chacun indépendamment l'un de l'autre, représentent H, un radical alkyle comportant de 1 à 12 atomes de C qui est non substitué, monosubstitué par CN ou $CF_3$ ou au moins monosubstitué par halogène où un ou plusieurs groupes $CH_2$ dans ces radicaux peuvent également, chacun indépendamment les uns des autres, être remplacés par -O-, -S-,

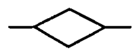 ,

-CH=CH-, -C≡C-, -CO-, -CO-O-, -O-CO- ou -O-CO-O- de telle sorte que des atomes de O ne soient pas liés directement les uns aux autres,

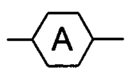 et 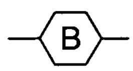

chacun indépendamment de l'autre, représentent

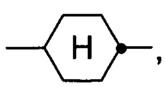, 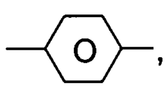, 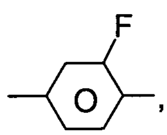, 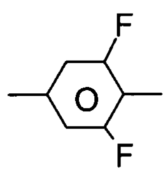

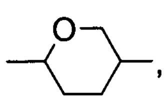, 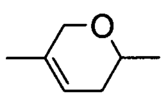 ou 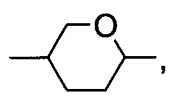,

au moins un anneau représente

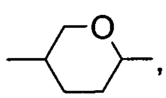,

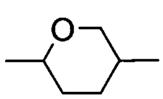 ou 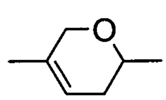,

$Z^1$ et $Z^2$ chacun indépendamment de l'autre, représentent $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ ou une liaison simple,
a représente 0 ou 1,

$L^1$ à $L^9$ chacun indépendamment les uns des autres, représentent H ou F, et
Y représente F, Cl, $SF_5$, CN, NCS, OCN, SCN ou un radical alkyle, alkoxy, alkényle ou alkényloxy mono- ou polyhalogéné, dans chaque cas comportant de 1 à 5 atomes de C.

**2.** Milieu de cristal liquide selon la revendication 1, **caractérisé en ce qu'**il comprend au moins un composé des formules A-1 à A-56

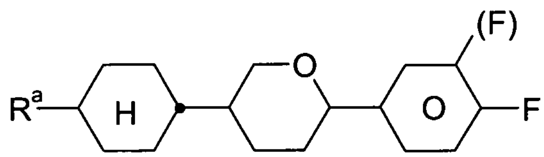

A-1

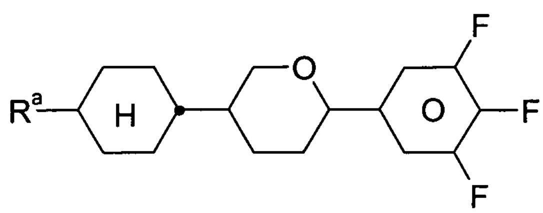

A-2

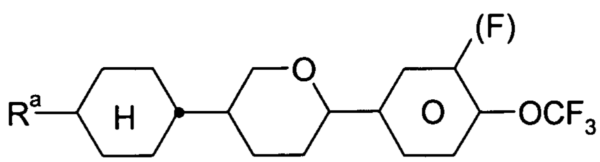

A-3

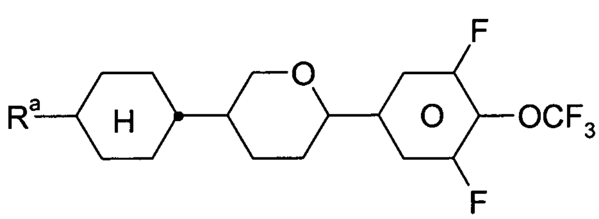

A-4

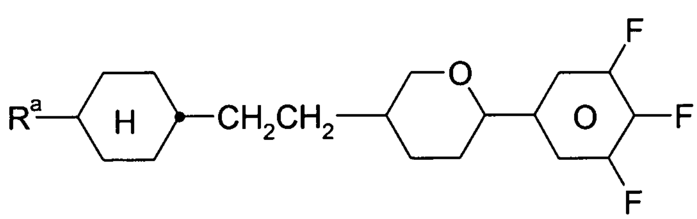

A-5

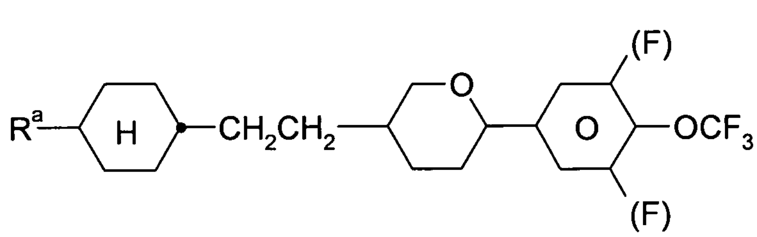

A-6

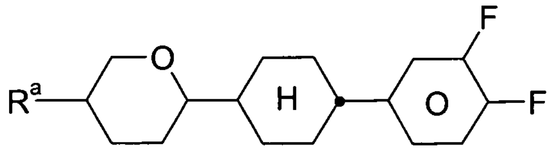
A-7

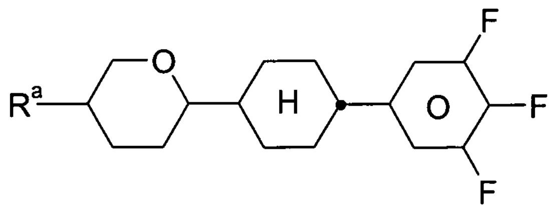
A-8

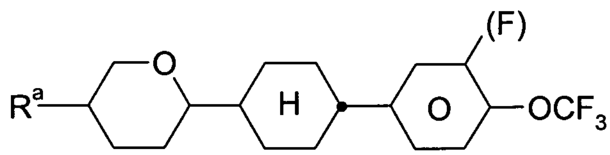
A-9

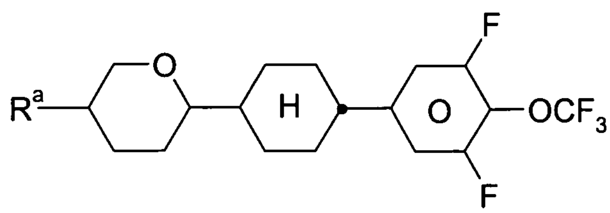
A-10

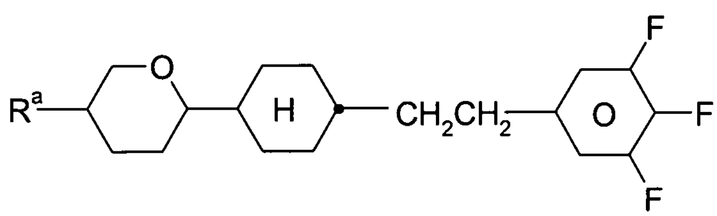
A-11

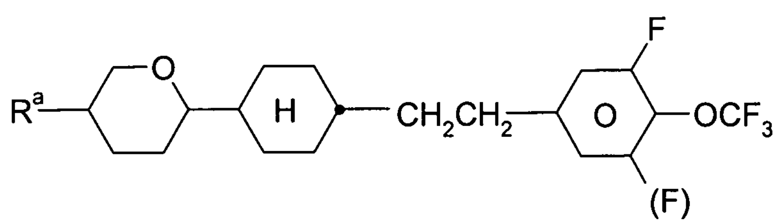
A-12

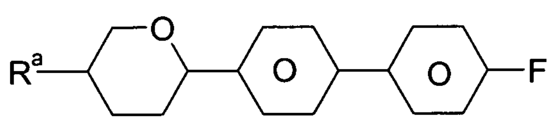
A-13

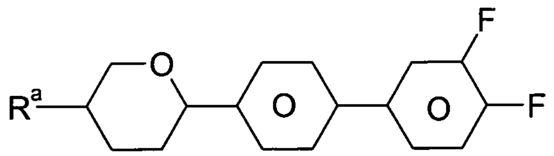

A-14

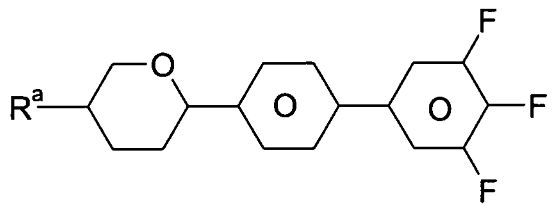

A-15

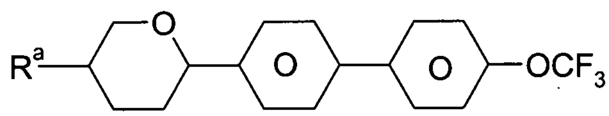

A-16

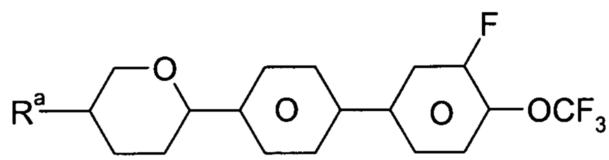

A-17

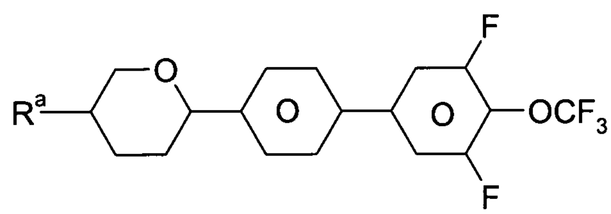

A-18

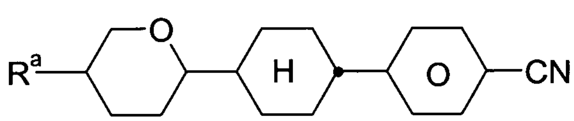

A-19

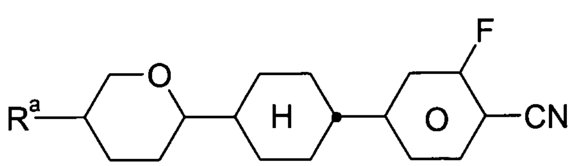

A-20

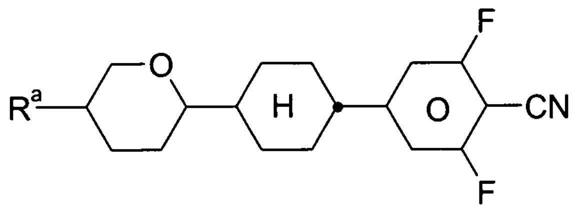

A-21

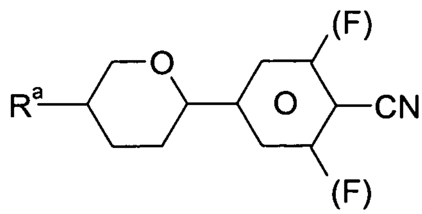

A-22

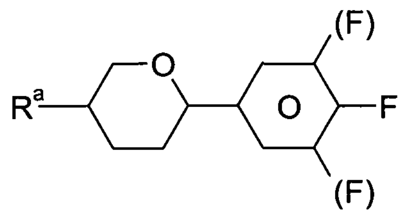

A-23

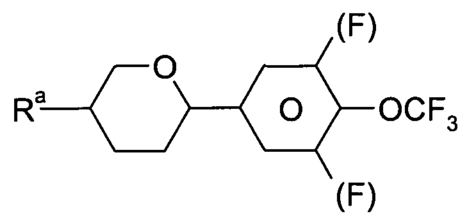

A-24

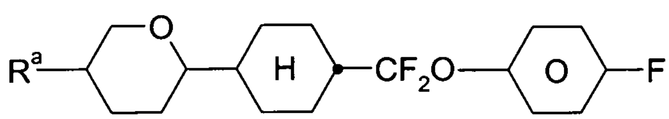

A-25

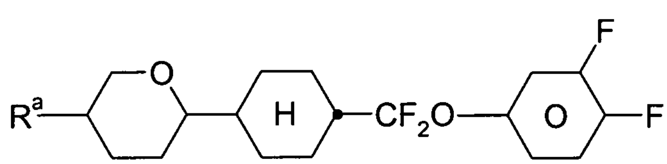

A-26

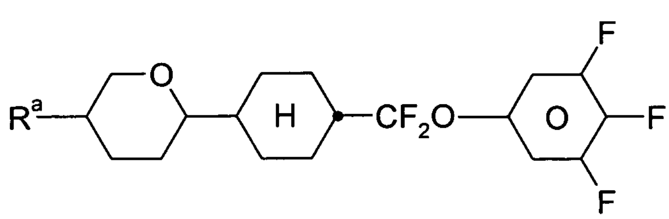

A-27

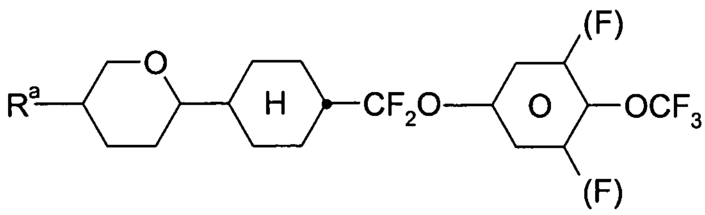

A-28

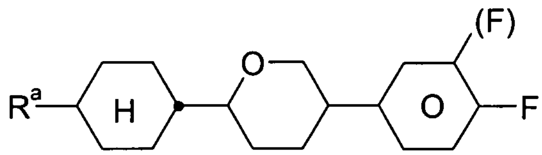

A-29

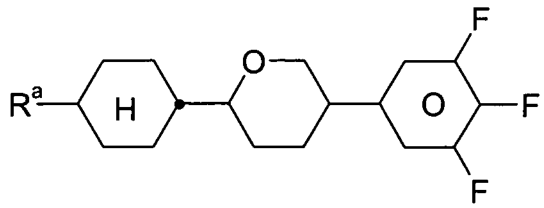

A-30

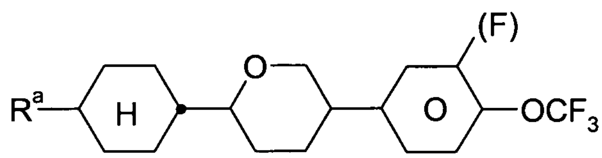

A-31

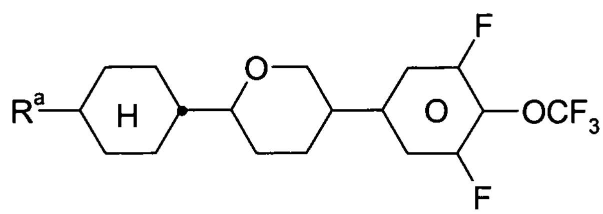

A-32

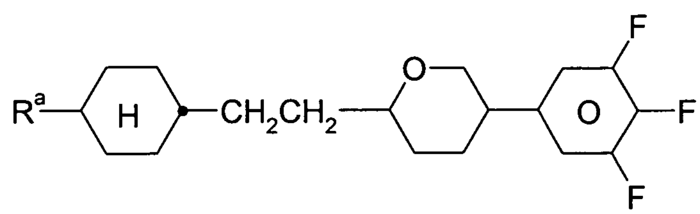

A-33

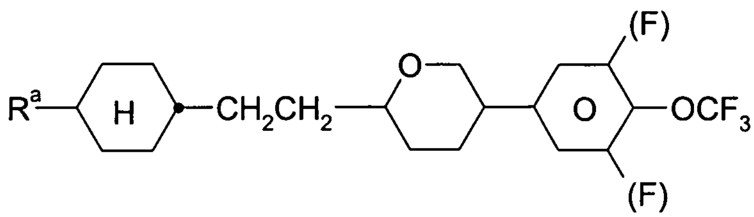

A-34

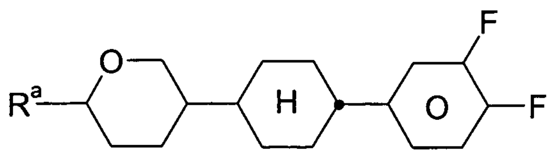

A-35

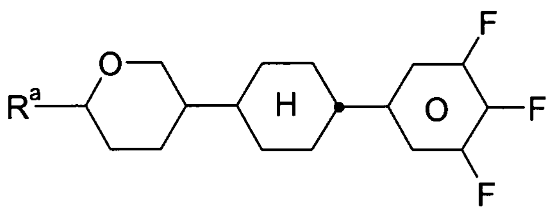

A-36

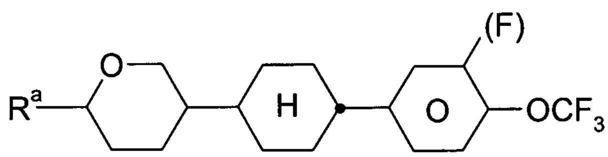

A-37

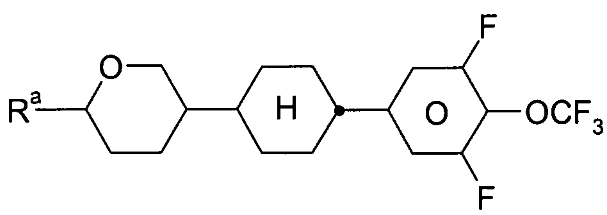

A-38

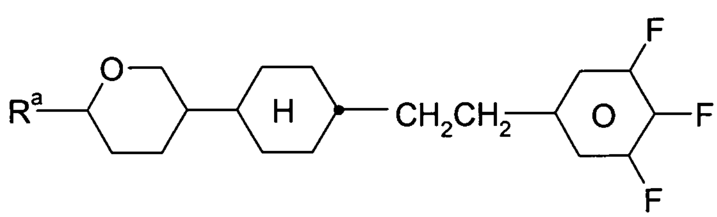

A-39

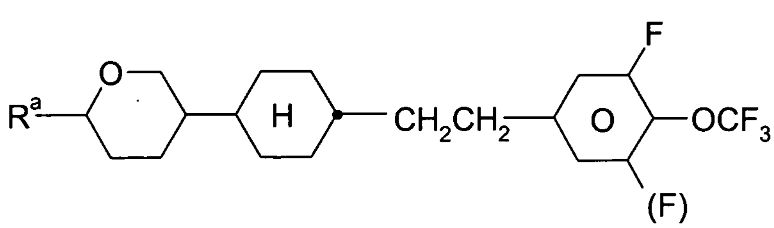

A-40

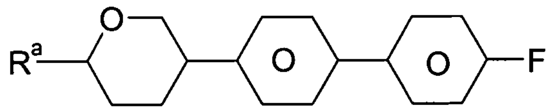

A-41

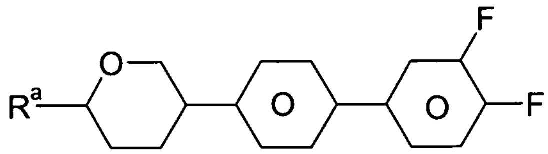

A-42

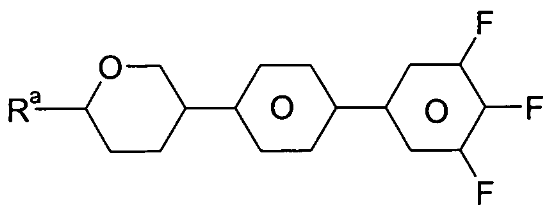

A-43

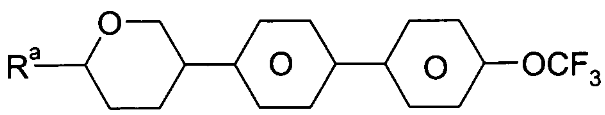

A-44

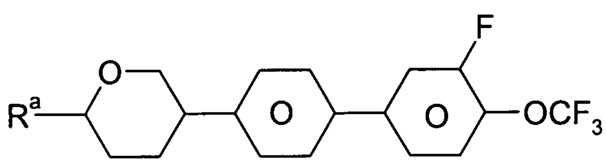

A-45

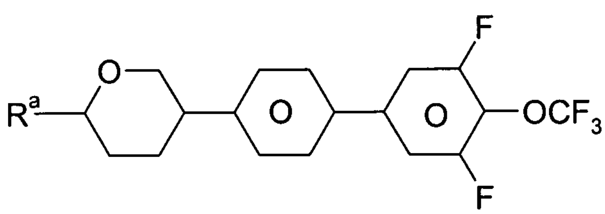

A-46

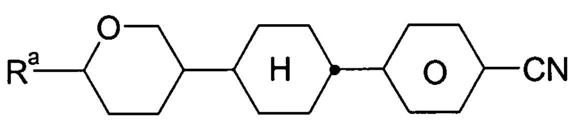

A-47

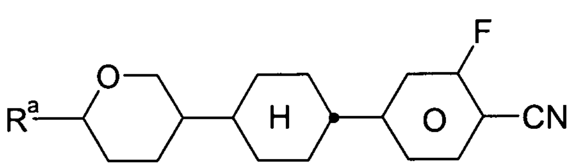

A-48

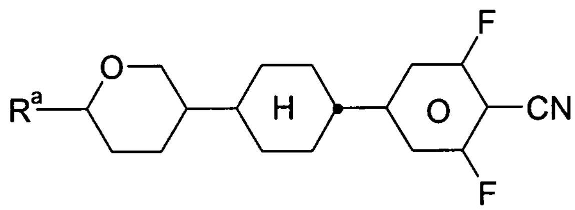

A-49

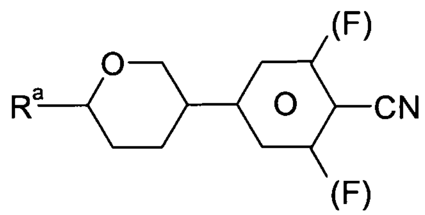

A-50

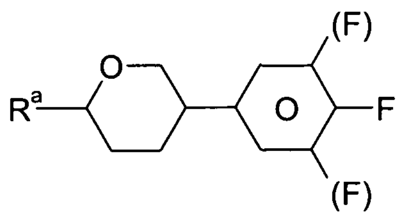

A-51

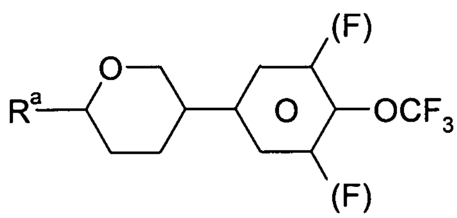

A-52

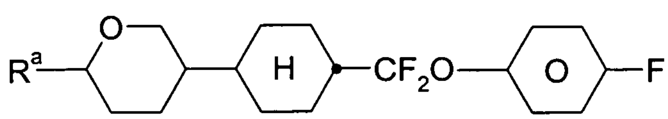

A-53

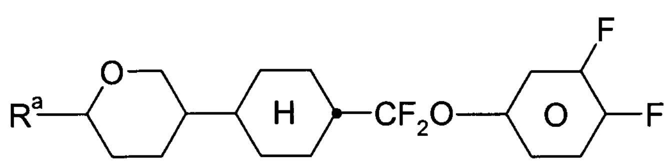

A-54

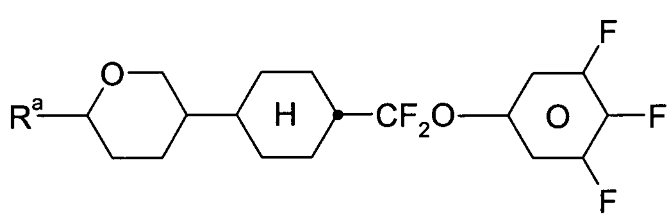

A-55

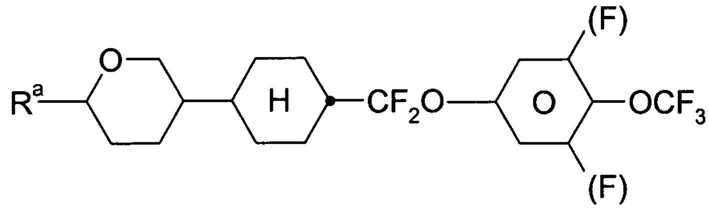

A-56

dans lesquelles Rª présente les significations indiquées selon la revendication 1.

**3.** Milieu de cristal liquide selon la revendication 1 ou 2, **caractérisé en ce qu'**il comprend au moins un composé des formules B-1 à B-6

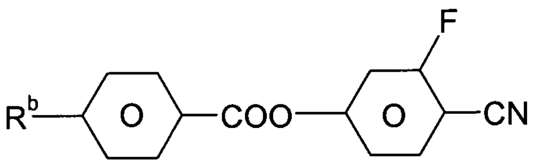

B-1

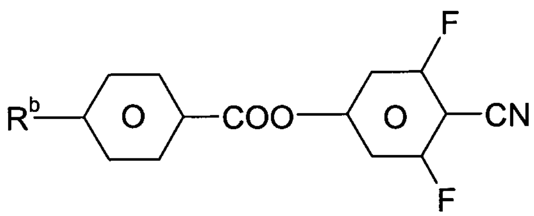

B-2

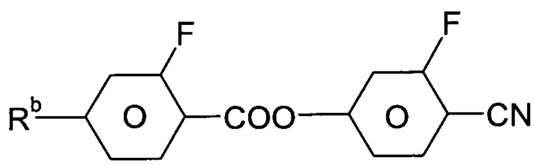

B-3

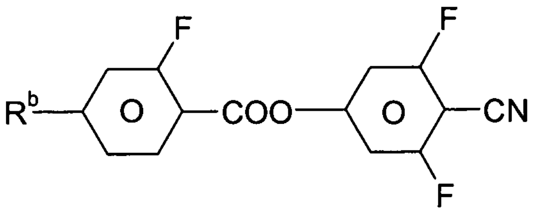

B-4

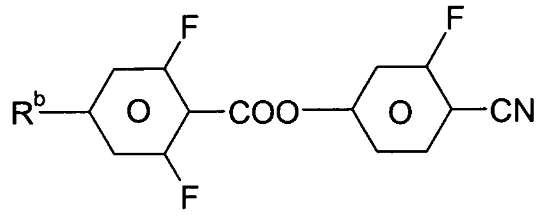

B-5

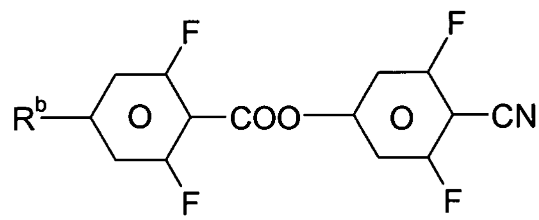

B-6

dans lesquelles

$R^b$ présente les significations indiquées selon la revendication 1.

**4.** Milieu de cristal liquide selon l'une des revendications 1 à 3, **caractérisé en ce qu'**il comprend de façon additionnelle au moins un composé des formules IIa à IIk

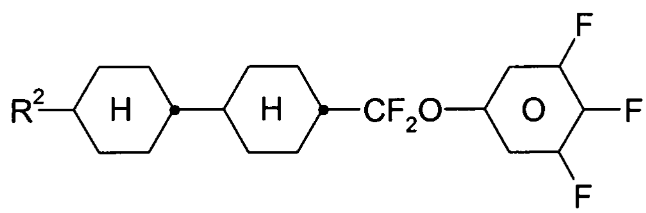

IIa

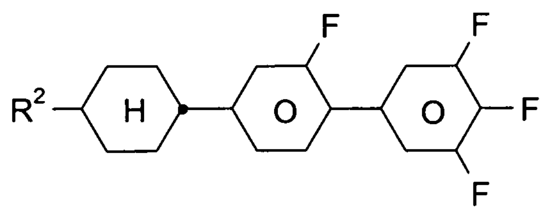

IIb

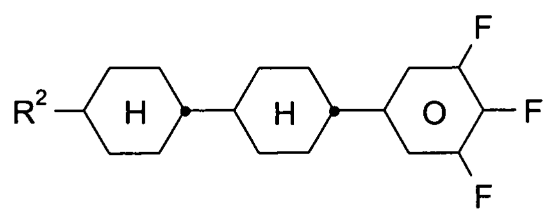

IIc

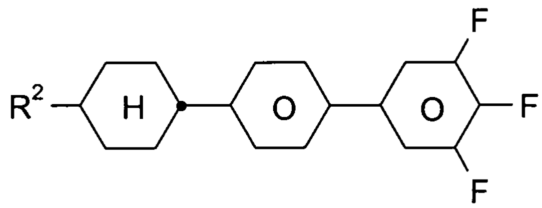

IId

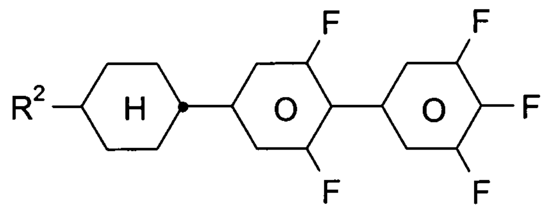

IIe

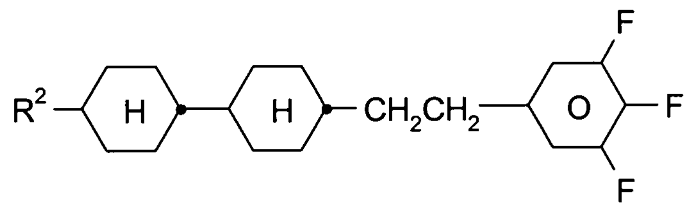

IIf

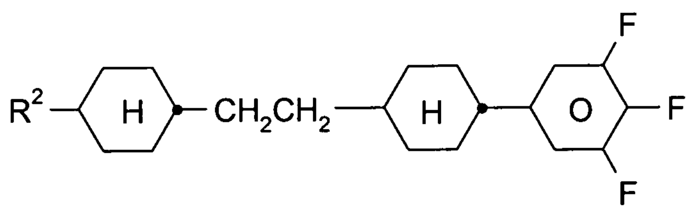

IIg

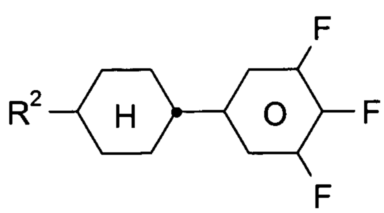

IIh

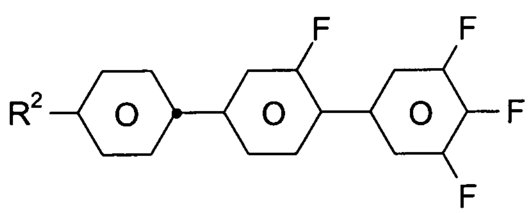

IIi

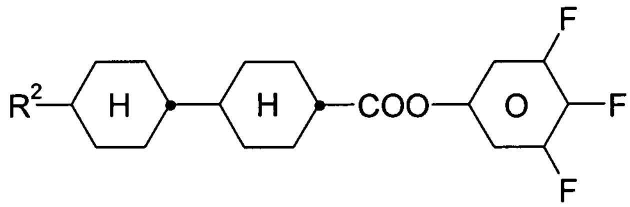

IIj

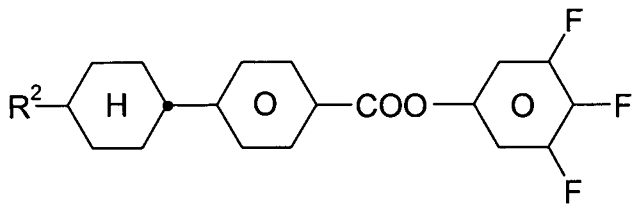

IIk

dans lesquelles

$R^2$ représente un radical alkyle comportant de 1 à 12 atomes de C qui est non substitué, monosubstitué par CN ou $CF_3$ ou au moins monosubstitué par halogène, où un ou plusieurs groupes $CH_2$ dans ces radicaux peuvent également, chacun indépendamment des autres, être remplacés par -O-, -S-,

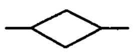 ,

-CH=CH-, -C≡C-, -CO-, -CO-O-, -O-CO- ou -O-CO-O- de telle sorte que des atomes de O ne soient pas liés directement les uns aux autres.

**5.** Milieu de cristal liquide selon l'une des revendications 1 à 4, **caractérisé en ce qu'**il comprend de façon additionnelle un ou plusieurs composés cyano des formules IIIa à IIIj

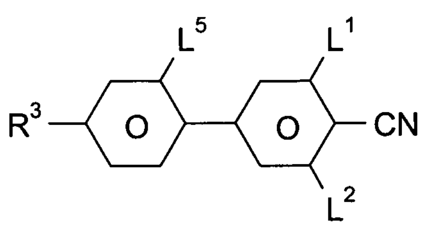

IIIa

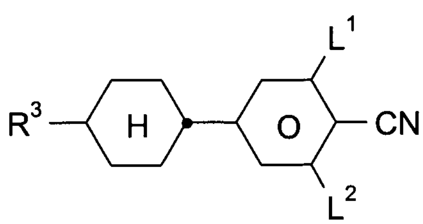

IIIb

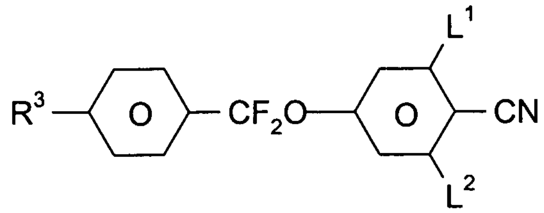
IIIc

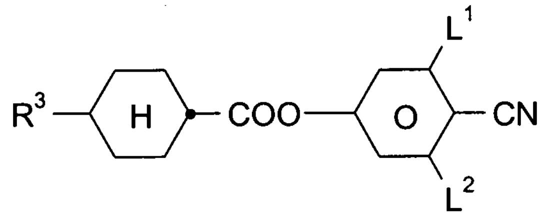
IIId

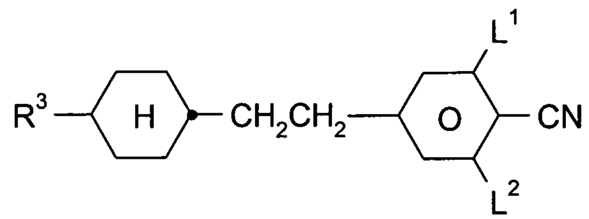
IIIe

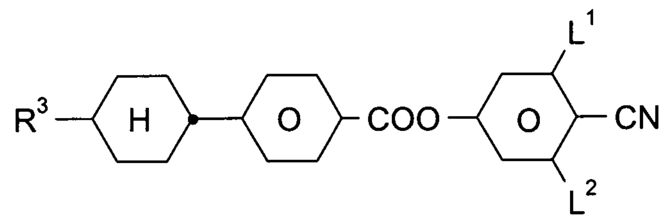
IIIf

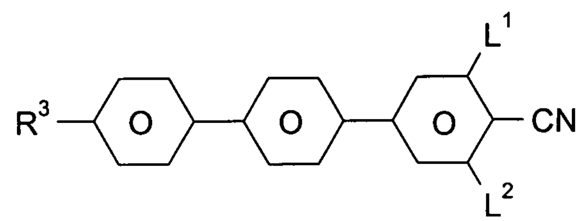
IIIg

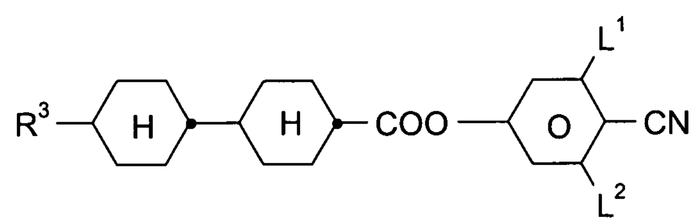
IIIh

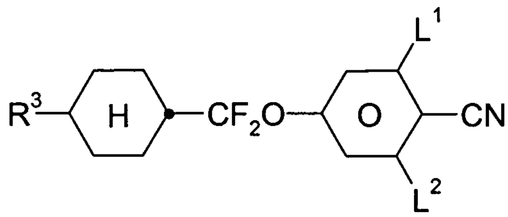

IIIi

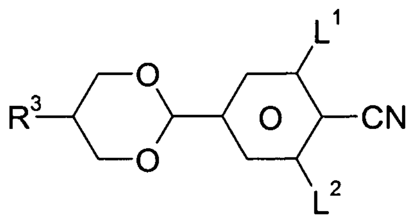

IIIj

dans lesquelles

$R^3$ représente un radical alkyle comportant de 1 à 12 atomes de C qui est non substitué, monosubstitué par CN ou $CF_3$ ou au moins monosubstitué par halogène, où un ou plusieurs groupes $CH_2$ dans ces radicaux peuvent également, chacun indépendamment des autres, être remplacés par -O-, -S-,

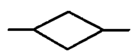 ,

-CH=CH-, -C≡C-, -CO-, -CO-O-, -O-CO- ou -O-CO-O- de telle sorte que des atomes de O ne soient pas liés directement les uns aux autres ; et
$L^1$, $L^2$ et $L^5$ chacun indépendamment des autres, représentent H ou F.

6. Milieu de cristal liquide selon l'une des revendications 1 à 5, **caractérisé en ce qu'**il comprend de façon additionnelle un ou plusieurs composés de la formule IV

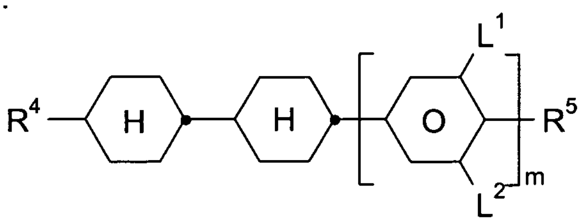

IV

dans laquelle

m représente 0 ou 1,
$R^4$ représente un groupe alkényle comportant de 2 à 7 atomes de C,
$R^5$ présente l'une des significations indiquées pour $R^a$ ou, si m = 1, représente à titre d'alternative F, Cl, $CF_3$, $OCF_3$,
$L^1$ et $L^2$ chacun indépendamment l'un de l'autre, représentent H ou F.

7. Milieu de cristal liquide selon l'une des revendications 1 à 6, **caractérisé en ce que** le milieu comprend de façon additionnelle un ou plusieurs composés de la formule VII

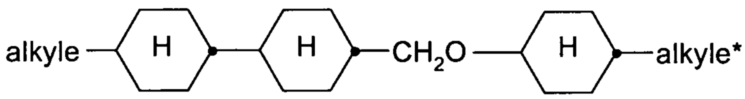 VII

dans laquelle
alkyle et alkyle*, chacun indépendamment l'un de l'autre, représentent un groupe alkyle comportant de 1 à 7 atomes de C.

8. Milieu de cristal liquide selon l'une des revendications 1 à 7, **caractérisé en ce que** le milieu comprend de façon additionnelle un ou plusieurs composés de tolane des formules T2a, T2b et/ou T2c

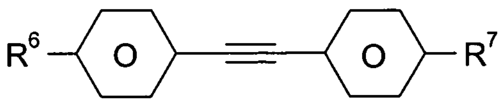 T2a

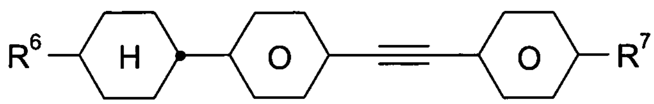 T2b

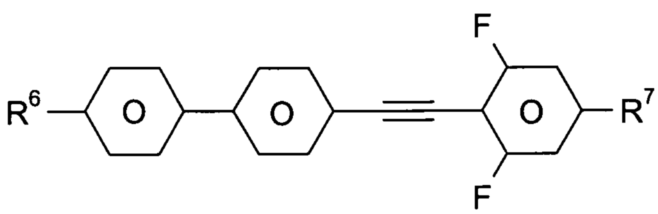 T2c

dans lesquelles

$R^6$ et $R^7$ représentent un radical alkyle comportant de 1 à 12 atomes de C qui est non substitué, monosubstitué par CN ou $CF_3$ ou au moins monosubstitué par halogène où un ou plusieurs groupes $CH_2$ dans ces radicaux peuvent également, chacun indépendamment des autres, être remplacés par -O-, -S-,

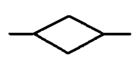 ,

-CH=CH-, -C≡C-, -CO-, -CO-O-, -O-CO- ou -O-CO-O- de telle sorte que des atomes de O ne soient pas liés directement les uns aux autres.

9. Milieu de cristal liquide selon l'une des revendications 1 à 8, **caractérisé en ce que** le milieu comprend 5-30% en poids de composés de la formule A.

10. Milieu de cristal liquide selon l'une des revendications 1 à 9, **caractérisé en ce que** le milieu comprend 3-30% en poids de composés de la formule B.

11. Milieu de cristal liquide selon l'une des revendications 1 à 10, **caractérisé en ce qu'**il comprend plus de 20% de composés présentant une anisotropie diélectrique de $\Delta\varepsilon \geq +12$

12. Utilisation du milieu de cristal liquide selon la revendication 1 pour des fins électro-optiques.

**13.** Utilisation du milieu de cristal liquide selon la revendication 1 pour des applications TN, STN et IPS.

**14.** Affichage à cristaux liquides électro-optique contenant un milieu de cristal liquide selon la revendication 1.

**15.** Composés de cristal liquide de la formule A

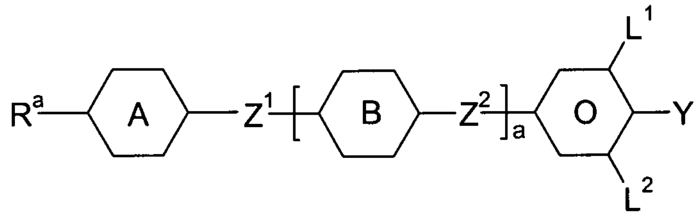

A

dans laquelle
$R^a$,

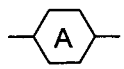 , 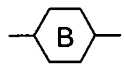 ,

a, $L^2$ et Y présentent les significations indiquées selon la revendication 1,

$L^1$ représente F, et
$Z^1$ et $Z^2$ chacun indépendamment de l'autre, représentent $-CH_2CH_2-$, $-(CH_2)_4-$, $-CH_2O-$, $-OCH_2-$, $-OCF_2-$, -C≡C-, -CF=CF-, -CH=CH-, -COO-, $-CH_2-$, $-(CH_2)_3-$ ou une liaison simple.

**16.** Composés de cristal liquide selon la revendication 15 des formules qui suivent

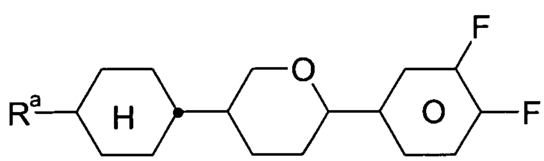

A-1*

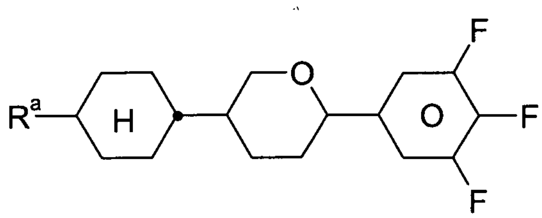

A-2

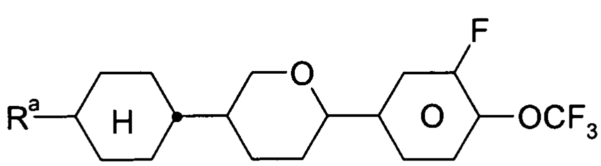

A-3*

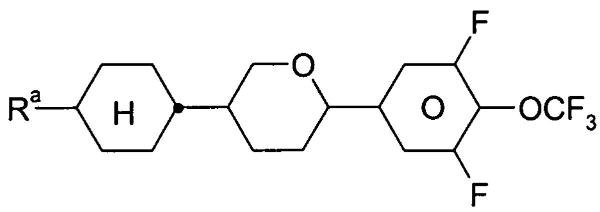

A-4

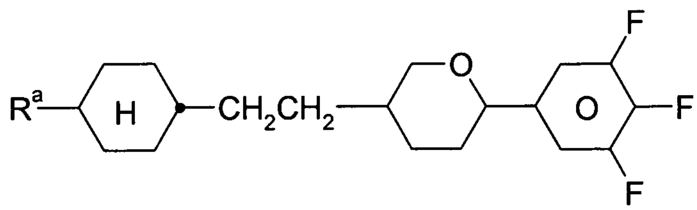

A-5

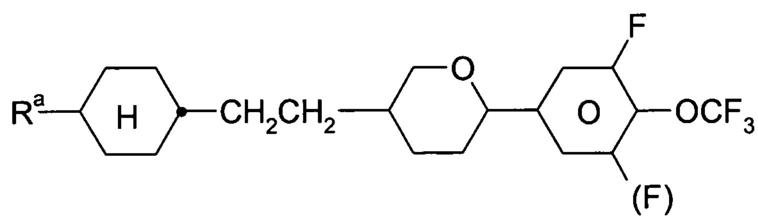

A-6*

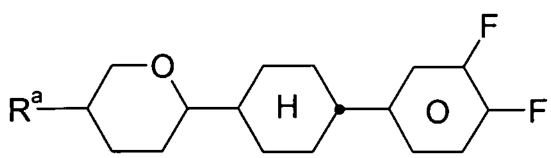

A-7

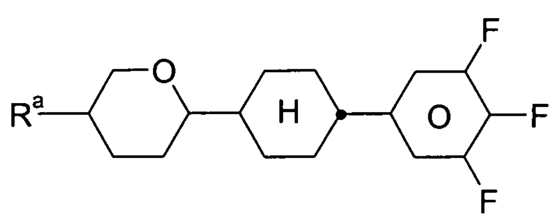

A-8

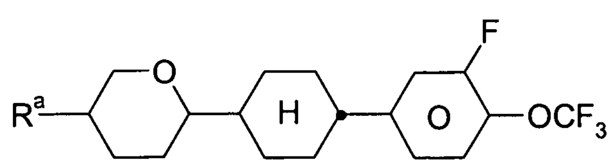

A-9*

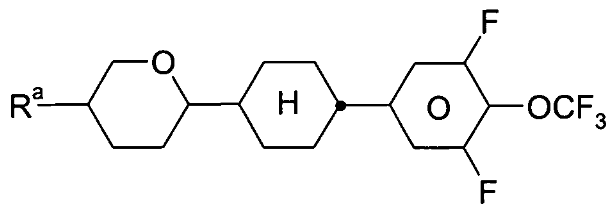

A-10

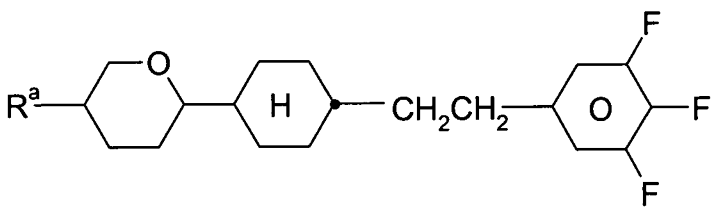

A-11

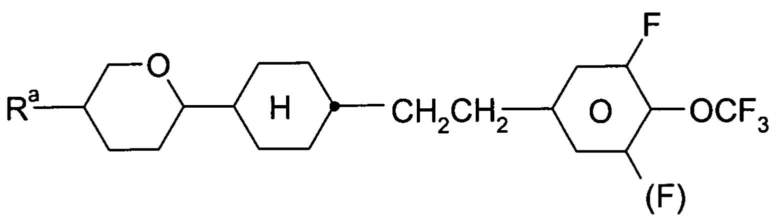

A-12

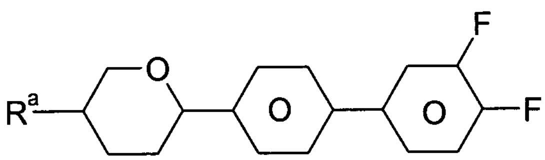

A-14

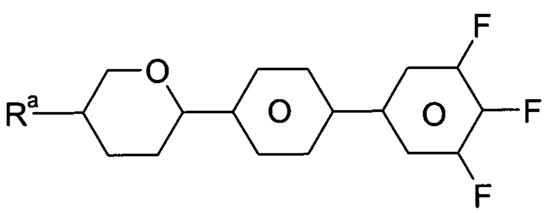

A-15

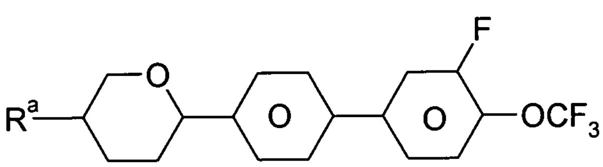

A-17

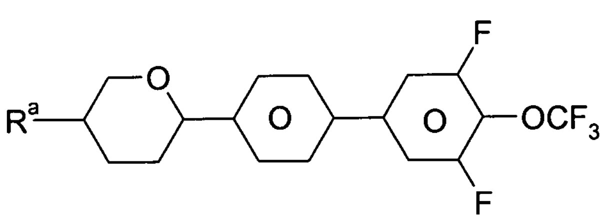

A-18

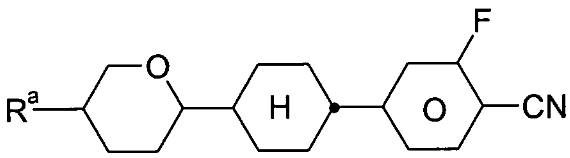

A-20

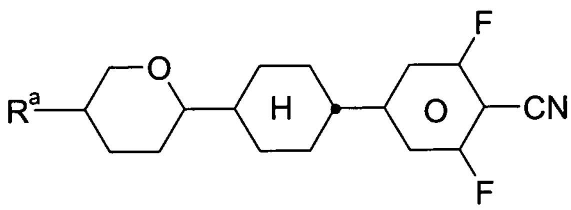

A-21

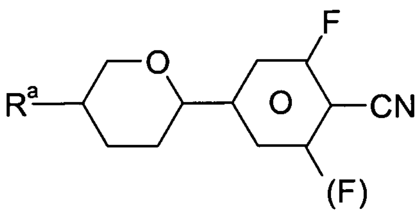

A-22*

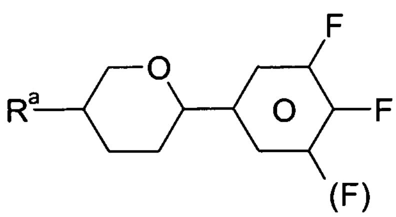

A-23*

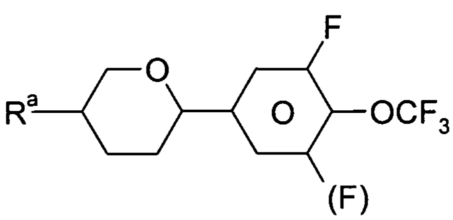

A-24*

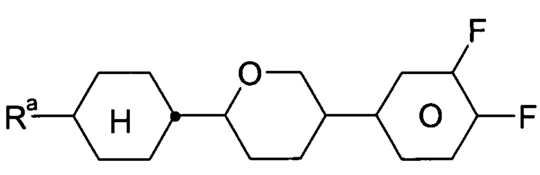

A-29*

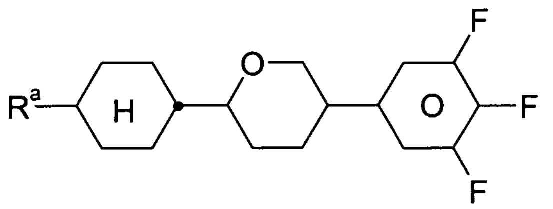

A-30

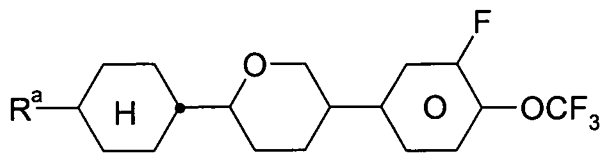

A-31*

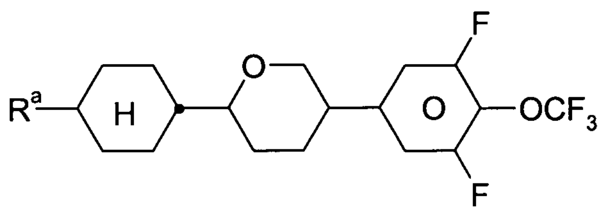

A-32

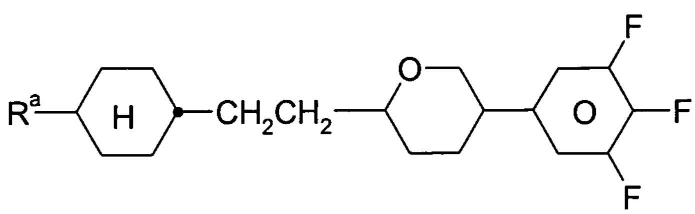

A-33

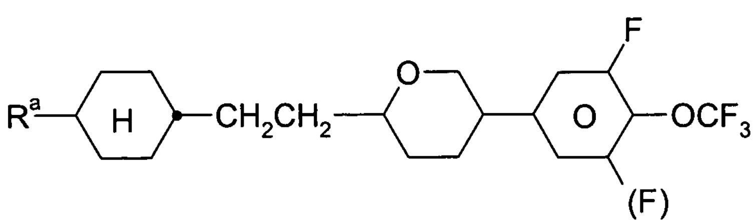

A-34*

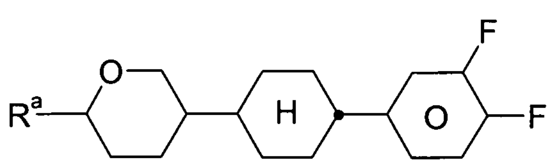

A-35

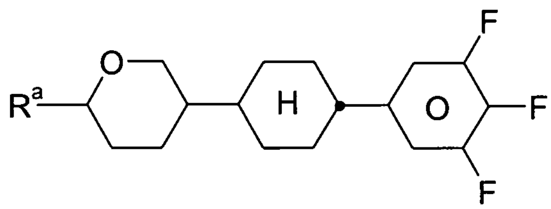

A-36

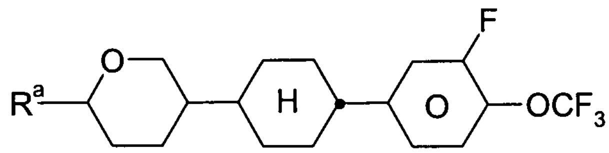

A-37*

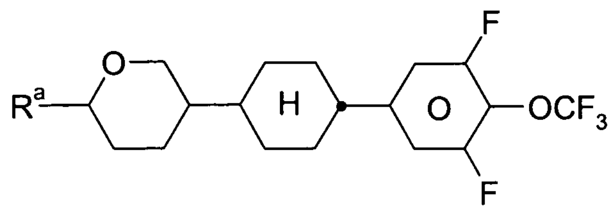

A-38

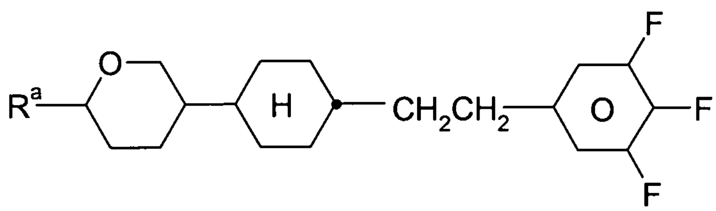

A-39

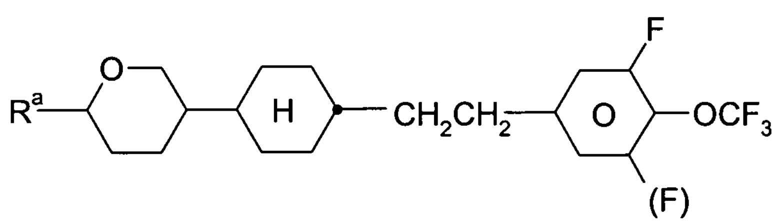

A-40

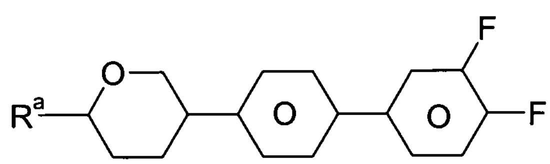

A-42

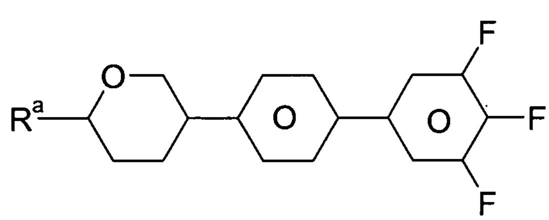

A-43

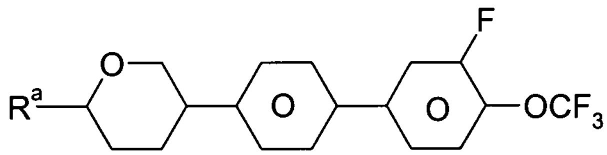

A-45

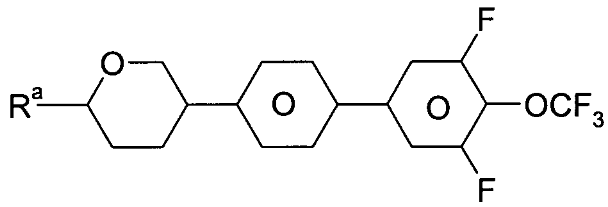

A-46

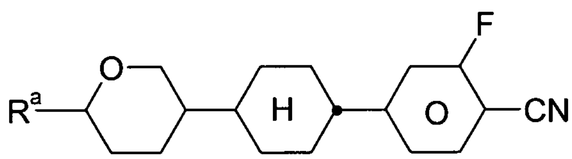

A-48

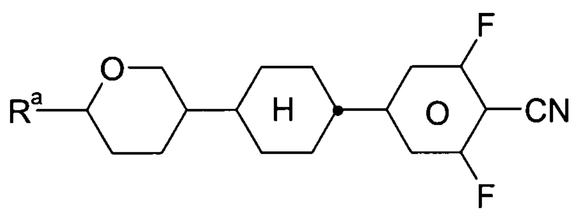

A-49

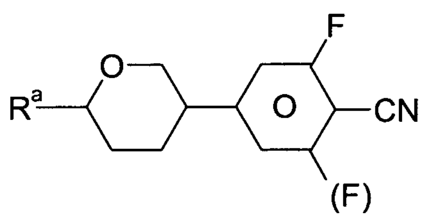

A-50*

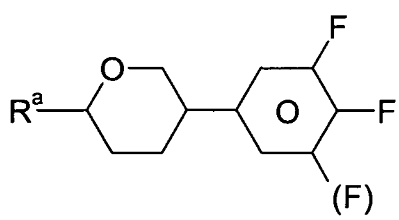

A-51*

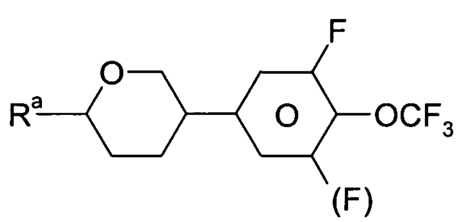

A-52*

dans lesquelles $R^a$ présente les significations indiquées selon la revendication 1.

**17.** Affichage à cristaux liquides TN ou STN comportant

- deux plaques externes qui, avec un cadre, forment une cellule,
- un mélange de cristaux liquides nématiques d'une anisotropie diélectrique positive localisé dans la cellule,
- des couches d'électrodes avec des couches d'alignement sur les intérieurs des plaques externes,
- un angle d'inclinaison entre l'axe longitudinal des molécules au niveau de la surface des plaques externes et les plaques externes de 0 degré à 30 degrés ; et
- un angle de vrillage du mélange de cristaux liquides dans la cellule depuis une couche d'alignement jusqu'à une autre couche d'alignement présentant une valeur entre 22,5° et 600°,
- un mélange de cristaux liquides nématiques constitué par

a) 15-75% en poids d'un <u>composant A</u> de cristal liquide comprenant un ou plusieurs composés présentant une anisotropie diélectrique supérieure à +1,5 ;
(b) 25-85% en poids d'un <u>composant B</u> de cristal liquide comprenant un ou plusieurs composés présentant une anisotropie diélectrique entre -1,5 et +1,5 ;
(c) 0-20% en poids d'un <u>composant D</u> de cristal liquide comprenant un ou plusieurs composés présentant une anisotropie diélectrique inférieure à -1,5 ; et
(d) si on le souhaite, un <u>composant C</u> actif optiquement selon une quantité qui est telle que le rapport entre l'épaisseur de couche (séparation des plaques externes) et le pas naturel du mélange de cristaux liquides nématiques chiraux est d'environ 0,2 à 1,3,

**caractérisé en ce que** le composant A comprend au moins un composé de la formule A

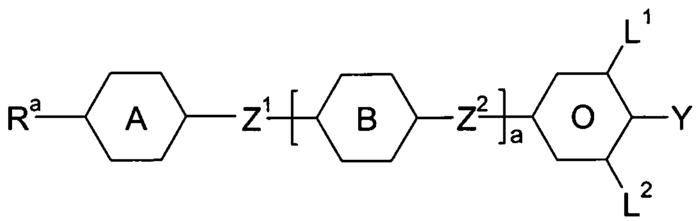

A

et au moins un composé de la formule B

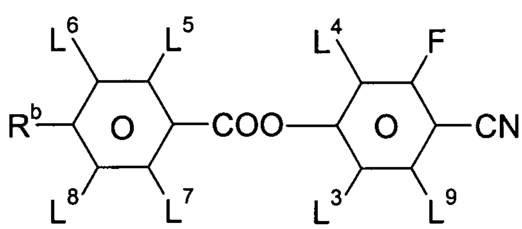

B

dans lesquelles

$R^a$ et $R^b$ chacun indépendamment l'un de l'autre, représentent H, un radical alkyle comportant de 1 à 12 atomes de C qui est non substitué, monosubstitué par CN ou $CF_3$ ou au moins monosubstitué par halogène où un ou plusieurs groupes $CH_2$ dans ces radicaux peuvent également, chacun indépendamment les uns des autres, être remplacés par -O-, -S-,

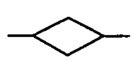 ,

-CH=CH-, -C≡C-, -CO-, -CO-O-, -O-CO- ou -O-CO-O- de telle sorte que des atomes de O ne soient pas liés directement les uns aux autres,

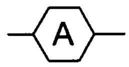 et 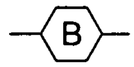

chacun indépendamment de l'autre, représentent

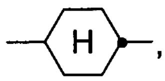, 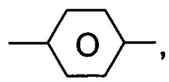, 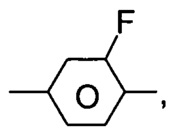, 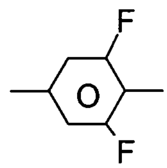

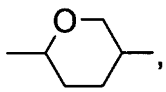, 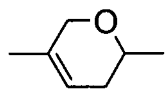 ou 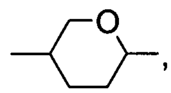,

au moins un anneau représente

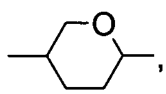,

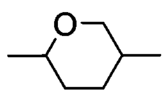 ou 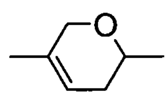,

$Z^1$ et $Z^2$ chacun indépendamment de l'autre, représentent $-CH_2CH_2-$, $-(CH_2)_4-$, $-CH_2O-$, $-OCH_2-$, $-CF_2O-$, $-OCF_2-$, -C≡C-, -CF=CF-, -CH=CH-, -COO-, $-CH_2-$, $-(CH_2)_3-$ ou une liaison simple,
a représente 0 ou 1,
$L^1$ à $L^9$ chacun indépendamment les uns des autres, représentent H ou F, et
Y représente F, Cl, $SF_5$, CN, NCS, OCN, SCN ou un radical alkyle, alkoxy, alkényle ou alkényloxy mono- ou polyhalogéné, dans chaque cas comportant de 1 à 5 atomes de C.